# EUROPEAN PATENT SPECIFICATION

(11) **EP 2 755 481 B1**
(45) Date of publication and mention of the grant of the patent: **18.03.2020**
(21) Application number: 12766466.2
(22) Date of filing: 14.09.2012
(51) Int. Cl.: A01N 43/16, A61K 31/734, A01N 43/90, A01N 43/653, A01P 3/00

(54) **USE OF ALGINATE OLIGOMERS TO ENHANCE THE EFFECTS OF ANTIFUNGAL AGENTS**
VERWENDUNG VON ALGINATOLIGOMEREN ZUR STEIGERUNG DER WIRKUNG VON ANTUGALEN WIRKSTOFFEN
UTILISATION D'OLIGOMERES D'ALGINATES POUR RENFORCER LES EFFETS D'AGENTS ANTIFONGIQUES

(30) Priority: 15.09.2011 GB 201116010
(43) Date of publication of application: 23.07.2014
(73) Proprietor: ALGIPHARMA AS, 1337 Sandvika (NO)
(72) Inventor: ONSØYEN, Edvar, N-3032 Drammen (NO); DESSEN, Arne, N-3440 Røyken (NO); THOMAS, David William, Cardiff CF14 4XY (GB); HILL, Katja Etel, Cardiff CF14 4XY (GB); SLETTA, Håvard, N-7024 Trondheim (NO); TØNDERVIK, Anne, N-7058 Jakobsli (NO); KLINKENBERG, Geir, N-7072 Heimdal (NO); MYRVOLD, Rolf, N-1389 Heggedal (NO)
(74) Representative: Dehns
(86) International application number: PCT/GB2012/052274
(87) International publication number: WO 2013/038197

(56) References cited:
- EP-A1- 0 807 631
- WO-A1-96/33164
- WO-A2-2009/068841

## Description

The present invention relates generally to the use of alginate oligomers to potentiate, or to enhance or improve, the efficacy of an antifungal agent, e.g. an antifungal drug or a fungicide, and in particular the effectiveness (or efficacy) of an antifungal agent to inhibit the growth and/or viability of fungi. In particular, it has been found that by combining the use of antifungal agents with alginate oligomers, the amount of antifungal agent used or necessary may be reduced. Accordingly, it is proposed that alginate oligomers may reduce the tolerance or enhance the susceptibility of fungi to antifungal agents. In some circumstances at least, it is believed that synergy may be occurring between alginate oligomers and antifungal agents. The invention accordingly generally provides alginate oligomers for use together with (i.e. in combination or conjunction with) an antifungal agent, e.g. an antifungal drug or a fungicide, for combating fungi, for example in the context of unwanted fungal colonisation (e.g. contamination) at any site or in the context of treating or preventing a fungal infection or disease (e.g. a mycosis), whether in an animal subject or in a plant. Thus, both medical and non-medical uses and methods are provided.

Fungi are members of a kingdom of eukaryotic organisms that are considered distinct from plants and animals. Unlike plant and animal cells, they are characterised by the presence of a cell wall containing chitin. Fungi are ubiquitous in nature and although many fungi are benign, many plant and animal diseases are attributed to their activities, either through infection of a host or through their production of toxic metabolites. Thus, the means to control fungal populations provides treatments for certain animal and plant diseases and conditions, and is important for the health and well-being of humans and the plants and animals they raise. The ability to control fungal populations on plants is of particular importance in the field of agriculture where economically valuable plants may be lost to fungal disease. The human cost of losing a food crop to fungal disease can also be high if access to alternative food sources is restricted. Fungi are also responsible for the spoilage of animal foodstuffs and other materials which leads to wastage and the necessity for repair or replacement of compromised materials. Control of fungi in these areas would minimise the significant economic costs associated with spoilage.

Thus, there is an ongoing need to find alternative or improved strategies to combat fungi, both in plants and animals but also in the wider environment. WO9633164 and EP 0807631 disclose novel chemical entities having antifungal properties.

Alternative or improved strategies to treat fugal diseases and infections in animal subjects are especially sought because certain inherent similarities between fungal and animal cells has made the identification of chemotherapeutic molecules specific to fungi difficult. As a result, very few effective antifungal drugs are currently available and those that are cause side effects at high dose because of their lack of specificity for fungal cells, which limits their systemic use. Thus, a strategy that can improve the effectiveness (or efficacy) of an antifungal agent to inhibit the growth and/or viability of fungi will be useful because doses of the antifungal agent can be reduced and side effects minimised.

Alginates are linear polymers of (1-4) linked β-D-mannuronic acid (M) and/or its C-5 epimer α-L-guluronic acid (G). The primary structure of alginates can vary greatly. The M and G residues can be organised as homopolymeric blocks of contiguous M or G residues, as blocks of alternating M and G residues and single M or G residues can be found interspacing these block structures. An alginate molecule can comprise some or all of these structures and such structures might not be uniformly distributed throughout the polymer. In the extreme, there exists a homopolymer of guluronic acid (polyguluronate) or a homopolymer of mannuronic acid (polymannuronate).

Alginates have been isolated from marine brown algae (e.g. certain species of *Durvillea, Lessonia* and *Laminaria*) and bacteria such as *Pseudomonas aeruginosa* and *Azotobacter vinelandii.* Other pseudomonads (e.g. *Pseudomonas fluorescens, Pseudomonas putida,* and *Pseudomonas mendocina*) retain the genetic capacity to produce alginates but in the wild they do not produce detectable levels of alginate. By mutation these non-producing pseudomonads can be induced stably to produce large quantities of alginate.

Alginate is synthesised as polymannuronate and G residues are formed by the action of epimerases (specifically C-5 epimerases) on the M residues in the polymer. In the case of alginates extracted from algae, the G residues are predominantly organised as G blocks because the enzymes involved in alginate biosynthesis in algae preferentially introduce the G neighbouring another G, thus converting stretches of M residues into G-blocks. Elucidation of these biosynthetic systems has allowed the production of alginates with specific primary structures (WO 94/09124, Gimmestad, M et al, Journal of Bacteriology, 2003, Vol 185(12) 3515-3523 and WO 2004/011628).

Alginates are typically isolated from natural sources as large high molecular weight polymers (e.g. an average molecular weight in the range 300,000 to 500,000 Daltons). It is known, however, that such large alginate polymers may be degraded, or broken down, e.g. by chemical or enzymatic hydrolysis to produce alginate structures of lower molecular weight. Alginates that are used industrially typically have an average molecular weight in the range of 100,000 to 300,000 Daltons (such alginates are still considered to be large polymers) although alginates of an average molecular weight of approximately 35,000 Daltons have been used in pharmaceuticals.

Alginate oligomers have been proposed to be of use in the combat of biofilms (WO 2009/068841).

It has now been found that alginate oligomers have the ability to potentiate. or to enhance or improve, the efficacy of an antifungal agent, e.g. an antifungal drug or a fungicide, and in particular the effectiveness (or efficacy) of an antifungal agent to inhibit the growth and/or viability of fungi. Thus the use of alginate oligomers together with (or in combination or conjunction with) an antifungal agent, e.g. an antifungal drug or a fungicide, constitutes an especially effective approach to the combat of fungal colonisation (e.g. contamination), infection and disease.

In one aspect the invention provides an *in vitro* method for combating colonisation of a site with a fungus, which is not in a biofilm, said method comprising contacting the site and/or the fungus, which is not in a biofilm, with an alginate oligomer of 2 to 50 monomer residues at least 80% of which are G residues together with at least one antifungal agent, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not in a biofilm, is enhanced.

In another aspect the invention provides an alginate oligomer of 2 to 50 monomer residues at least 80% of which are G residues for use together with at least one antifungal agent in treating a subject infected, suspected to be infected, or at risk of infection, with a fungus, wherein the fungus is not in a biofilm, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not in a biofilm, is enhanced.

In another aspect the invention provides a product containing an alginate oligomer of 2 to 50 monomer residues at least 80% of which are G residues and an antifungal agent as a combined preparation for separate, simultaneous or sequential use in treating a subject infected, suspected to be infected, or at risk of infection, with a fungus , wherein the fungus is not in a biofilm, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not it a biofilm, is enhanced.

Also described herein is a method to improve the efficacy of an antifungal agent against a fungus, and in particular the effectiveness (or efficacy) of an antifungal agent to inhibit the growth and/or viability of a fungus, (which includes inhibition of the growth and/or viability of a population of fungi), said method comprising using said antifungal agent together with (or in combination or conjunction with) an alginate oligomer.

Such a method may involve contacting said fungus, or a site at which said fungus may or does occur, with an alginate oligomer together with (or in conjunction or combination with) the antifungal agent.

It will thus be appreciated that in addition to improving anti-fungal agent action when a fungus is actually present (i.e. in the presence of fungal infection or fungal colonisation of any site), the method may also be used prophylactically to inhibit (e.g. prevent, reduce or delay) fungal infection or colonisation, for example at sites or in subjects susceptible to or at risk from fungal colonisation or infection.

The contacting step may comprise contacting the fungus (more particularly the fungi) or the site with the alginate oligomer at the same, or substantially the same, time as or prior to contacting the fungus or site with the antifungal agent in an amount effective to improve the efficacy of the antifungal agent against the fungus, and in particular the effectiveness (or efficacy) of the antifungal agent to inhibit the growth and/or viability of the fungus (or population of fungi).

The term "contacting" encompasses any means of delivering the alginate oligomer to the fungus or site, whether directly or indirectly, and thus any means of applying the alginate oligomer to the fungus or site, or exposing the fungus or site to the alginate oligomer, e.g. applying the alginate oligomer directly to the fungus or site. As explained in more detail below, expressly included within the scope of the invention are methods which are not carried out in or on the human or non-human animal body, or in relation to, or in or on a device or material whollyor partly contained in or on the human or non-human animal body.

By "colonisation" is meant the presence of a fungus at a particular site or location. In particular, unwanted colonisation, e.g. contamination, is encompassed by this term.
Colonisation may thus be viewed as the establishment of a fungus at a location and the expansion of the numbers of that organism by replication or the recruitment of additional fungi, which may be of the same or of a different type. This colony may be considered to be a population of fungi.

Also described herein is a method of combating a fungus (which includes a population of fungi as well as an individual or single fungus or fungal cell), said method comprising contacting said fungus, or a site at which said fungus is or may be located, with (particularly with an effective amount of) an alginate oligomer together with (particularly together with an effective amount of) at least one antifungal agent. Such a method may particularly be an *in vitro* or an *ex vivo* method.

A population of fungi may be homogenous (i.e. contain a single type of fungus) or may be heterogeneous (i.e. contain a plurality of types of fungus and/or other microorganisms). Some or all of the fungi in the population may be pathogenic. The population may be an established population or be a partially established population. In other words, the location to be treated has previously been colonised by at least one fungus that has multiplied or recruited other fungi to establish the population.

By "use together" or "together with" (or in combination or conjunction with) it is particularly meant that an effective amount of the alginate oligomer and an effective amount of the antifungal agent are administered/applied in a manner that results in the fungus (more particularly the fungi) or site being contacted with the alginate oligomer at the same, or substantially the same, time or prior to being contacted with the antifungal agent.

In the context of pharmaceutical treatments, the effective amounts will be pharmaceutically effective amounts. Any clinically acceptable dosing regime may be employed to achieve this "use together". The skilled man would be able to take into account any relevant variable factors (e.g. the routes of administration, the bioavailability, and the pharmacokinetics of the oligomer and the antifungal agent being used, the subject's physical state, the location of the fungus, etc.) in order to design an appropriate dosing regime for a particular subject. In one embodiment, a pharmaceutically effective amount of the alginate oligomer is administered at the same or substantially the same time as or prior to administering a pharmaceutically effective amount of the antifungal agent. In other embodiments the oligomer is administered separately to and after the antifungal agent. The skilled man would readily be able to design his dosing regime to maximise the improvement in the effectiveness of the antifungal agent against fungi and fungal infections. He would also be able to select optimal combinations of the two active agents depending on the particular clinical situation he is faced with.

In a non-pharmaceutical context any environmentally (e.g. agriculturally) acceptable application regime may be employed to achieve this "use together". Such regimes may however also be pharmaceutically acceptable and/or physiologically acceptable. The skilled man would be able to take into account any relevant variable factors (e.g. the routes of application, the bioavailability, and the environmental longevity of the oligomer and the antifungal agent being used, the location of the fungus, the wider environmental context of the location to be treated, etc.) in order to design an appropriate application regime for a particular location to be treated. In one embodiment, an environmentally effective amount of the alginate oligomer is administered at the same or substantially the same time as or prior to administering an environmentally effective amount of the antifungal agent. In other embodiments the oligomer is administered separately to and after the antifungal agent. The skilled man would readily be able to design his application regime to maximise the improvement in the effectiveness of the antifungal agent against fungi. He would also be able to select optimal combinations of the two active agents depending on the particular environmental situation he is faced with.

"Use together/together with" does not imply that the respective agents are necessarily present in the same formulation or composition, and accordingly even if used, or administered, at the same or substantially the same time, the alginate oligomer and antifungal agent need not, indeed most likely will not, be present in the same composition or formulation, but may be administered separately. Thus "separate" use/administration includes use/administration at the same or substantially the same time, or at different times, e.g. sequentially, or at different time intervals according to the desired dosage or usage regime.

The term "infected with" (or "infected by" or "a fungal infection of a subject") is used broadly herein to indicate that the subject may comprise, or contain, or carry, the fungus in question, i.e. that the fungus may simply be present in or on the subject, and this may include any site or location in or on the body of the subject. It is not necessary that the infection of the subject be manifest as a clinical disease (i.e. that the infection result in clinical symptoms in the subject), although this is of course encompassed. A subject who is suspected to be infected or who is at risk of infection may be a subject who has been exposed to the fungus or to an infected subject, or a subject presenting with clinical signs or symptoms of infection (in the case of a suspected infection), or a subject who is susceptible to infection, whether generally (e.g. due to the clinical status of the subject) or particularly to the fungus in question. The term "fungal infection of a plant" should be construed in line with this.

Also disclosed herein is the use of an alginate oligomer for the manufacture of a medicament for use together with at least one antifungal agent in treating a subject infected, suspected to be infected, or at risk of infection, with a fungus, or for increasing the efficacy of said antifungal agent against a fungus or a fungal infection of a subject.

The medicament may further comprise the antifungal agent (or antifungal agents). The medicament may be in the form of a single composition or formulation comprising the alginate oligomer and antifungal agent(s) or separate compositions or formulations may be prepared and used, each containing the alginate oligomer or the antifungal agent(s), respectively.

Thus more particularly disclosed herein is the use of an alginate oligomer and at least one antifungal agent for the manufacture of a medicament for use in treating a subject infected, suspected to be infected, or at risk of infection, with a fungus, or for increasing the efficacy of said antifungal agent against a fungus or a fungal infection of a subject.

As noted above, the antifungal agent may be applied or administered separately from the alginate oligomer.

In accordance with the various aspects of the invention, the antifungal agent may be applied or administered simultaneously with the alginate oligomer or sequentially. As noted above, in one embodiment the antifungal agent is administered at the same or substantially the same time as the alginate oligomer, and in another embodiment it is administered after the alginate oligomer. In other embodiments the oligomer is administered separately to and after the antifungal agent. Included within the scope of "substantially the same time" is application or administration of the antifungal agent immediately or almost immediately before or after the alginate oligomer. The term "almost immediately" may be read as including application or administration within one hour of the previous application or administration, preferably within 30 minutes. However the antifungal agent may be applied or administered at least 1 hour, at least 3 hours, or at least 6 hours or more after the alginate oligomer. In these embodiments the antifungal agent can be applied or administered with or without a further application of an alginate oligomer. The alginate oligomer can be applied or administered in a plurality of applications prior to or with the antifungal agent, including as noted above, an application or administration immediately or almost immediately after the antifungal agent. In other embodiments the antifungal agent(s) may conveniently be applied or administered before the alginate oligomer, e.g. at least 1 hour, at least 3 hours, at least 6 hours before the alginate oligomer. In these embodiments the alginate oligomer can be applied or administered with or without a further application of the antifungal agent. The antifungal agent can be applied or administered in a plurality of applications prior to, or with, the alginate oligomer.

Also in accordance with certain aspects of the invention there may be a preceding step of identifying a subject as being infected, suspected of being infected, or at risk of infection, with a fungus, or a step of diagnosing a subject as being infected, or at risk of infection, with a fungus. In other aspects there may be a preceding step of identifying a site as being colonised, suspected of being colonised, or at risk of colonisation, with a fungus.

As noted above, alginates typically occur as polymers of an average molecular weight of at least 35,000 Daltons, i.e. approximately 175 to approximately 190 monomer residues, although typically much higher and an alginate oligomer may be defined as a material obtained by fractionation (i.e. size reduction) of an alginate polymer, commonly a naturally occurring alginate. An alginate oligomer can be considered to be an alginate of an average molecular weight of less than 35,000 Daltons (i.e. less than approximately 190 or less than approximately 175 monomer residues), in particular an alginate of an average molecular weight of less than 30,000 Daltons (i.e. less than approximately 175 or less than approximately 150 monomer residues) more particularly an average molecular weight of less than 25,000 or 20,000 Daltons (i.e. less than approximately 135 or 125 monomer residues or less than approximately 110 or 100 monomer residues).

Viewed alternatively, an oligomer generally comprises 2 or more units or residues and an alginate oligomer for use according to the invention will typically contain 2 to 50, more preferably 2 to 40, 2 to 35 or 2 to 30 residues. Thus, an alginate oligomer may have an average molecular weight of 350 to 20,000 Daltons, preferably 350 to 15,000 Daltons, preferably 350 to 10,000 Daltons and more preferably 350 to 8000 Daltons, 350 to 7000 Daltons, or 350 to 6,000 Daltons.

Alternatively put, the alginate oligomer may have a degree of polymerisation (DP), or a number average degree of polymerisation (DPn) of 2 to 50, more preferably 2 to 40, 2 to 35, 2 to 30, 2 to 28, 2 to 25, 2 to 22, 2 to 20, 2 to 18, 2 to 17, 2 to 15 or 2 to 12.

Other representative ranges (whether for the number of residues, DP or DPn) include any one of 3, 4, 5, 6, 7, 8, 9, 10 or 11 to any one of 50, 45, 40, 39, 38, 37, 36, 35, 34, 33, 32, 31, 30, 29, 28, 27, 26, 25, 24, 23, 22, 21, 20, 19, 18, 17, 16, 15, 14, 13 or 12.

Other representative ranges (whether for the number of residues, DP or DPn) include any one of 8, 9, 10, 11, 12, 13, 14 or 15 to any one of 50, 45, 40, 39, 38, 37, 36, 35, 34, 33, 32, 31, 30, 29, 28, 27, 26, 25, 24, 23, 22, 21, 20, 19, 18, 17 or 16.

Other representative ranges (whether for the number of residues, DP or DPn) include any one of 11, 12, 13, 14, 15, 16, 17 or 18 to any one of 50, 45, 40, 39, 38, 37, 36, 35, 34, 33, 32, 31, 30, 29, 28, 27, 26, 25, 24, 23, 22, 21, 20 or 19.

An alginate oligomer will, as noted above, contain (or comprise) guluronate or guluronic acid (G) and/or mannuronate or mannuronic acid (M) residues or units. An alginate oligomer according to the invention will preferably be composed solely, or substantially solely (i.e. consist essentially of) uronate/uronic acid residues, more particularly solely or substantially solely of G and/or M residues. Alternatively expressed, in the alginate oligomer of use in the present invention, at least 80%, more particularly at least 85, 90, 95 or 99% of the monomer residues may be uronate/uronic acid residues, or, more particularly G residues. In other words, preferably the alginate oligomer will not comprise other residues or units (e.g. other saccharide residues, or more particularly other uronic acid/uronate residues).

The alginate oligomer is preferably a linear oligomer.

At least 80, 85, 90, 91, 92, 93, 94, 95, 96, 97, 98 or 99% of the monomer residues are guluronate. In one embodiment the alginate oligomer may be an oligoguluronate (i.e. a homooligomer of G, or 100% G)

In a further preferred embodiment, the above described alginates of the invention have a primary structure wherein the majority of the G residues are in so called G-blocks. Preferably at least 50%, more preferably at least 70 or 75%, and most preferably at least 80, 85, 90, 92 or 95% of the G residues are in G-blocks. A G block is a contiguous sequence of at least two G residues, preferably at least 3 contiguous G residues, more preferably at least 4 or 5 contiguous G residues, most preferably at least 7 contiguous G residues.

In particular at least 90% of the G residues are linked 1-4 to another G residue. More particularly at least 95%, more preferably at least 98%, and most preferably at least 99% of the G residues of the alginate are linked 1-4 to another G residue.

The alginate oligomer of use in the invention is preferably a 3- to 35-mer, more preferably a 3- to 28-mer, in particular a 4- to 25-mer, e.g. a 5- to 20-mer, especially a 6- to 22-mer, in particular an 8- to 20-mer, especially a 10- to 15-mer, e.g. having a molecular weight in the range 350 to 6400 Daltons or 350 to 6000 Daltons, preferably 550 to 5500 Daltons, preferably 750 to 5000 Daltons, and especially 750 to 4500 Daltons or 2000 to 3000 Daltons or 900 to 3500 Daltons. Other representative alginate oligomers include, as mentioned above, oligomers with 5, 6, 7, 8, 9, 10, 11 or 12 to 50, 45, 40, 35, 28, 25, 22 or 20 residues.

It may be a single compound or it may be a mixture of compounds, e.g. of a range of degrees of polymerization. As noted above, the monomeric residues in the alginate oligomer, may be the same or different and not all need carry electrically charged groups although it is preferred that the majority (e.g. at least 60%, preferably at least 80% more preferably at least 90%) do. It is preferred that a substantial majority, e.g. at least 80%, more preferably at least 90% of the charged groups have the same polarity. In the alginate oligomer, the ratio of hydroxyl groups to charged groups is preferably at least 2:1, more especially at least 3:1.

The alginate oligomer of the invention may have a degree of polymerisation (DP), or a number average degree of polymerisation (DPₙ), of 3-28, 4-25, 6-22, 8-20 or 10-15, or 5-18 or 7-15 or 8-12, especially 10.

The alginate oligomer of the invention may have a degree of polymerisation (DP), or a number average degree of polymerisation (DPₙ), of 5-50, 5-40, 5-35, 5-30, 5-28, 5-25, 5-22, 5-20, 5-18, 5-16 or 5-14.

The alginate oligomer of the invention may have a degree of polymerisation (DP), or a number average degree of polymerisation (DPₙ), of 8-50, 8-40, 8-35, 8-30, 8-28, 8-25, 8-22, 8-20, 8-18, 8-16 or 8-14.

The alginate oligomer of the invention may have a degree of polymerisation (DP), or a number average degree of polymerisation (DPₙ), of 9-50, 9-40, 9-35, 9-30, 9-28, 9-25, 9-22, 9-20, 9-18, 9-16 or 9-14.

The alginate oligomer of the invention may have a degree of polymerisation (DP), or a number average degree of polymerisation (DPₙ), of 10-50, 10-40, 10-35, 10-30, 10-28, 10-25, 10-22, 10-20, 10-18, 10-16 or 10-14.

The alginate oligomer of the invention may have a degree of polymerisation (DP), or a number average degree of polymerisation (DPₙ), of 12-50, 12-40, 12-35, 12-30, 12-28, 12-25, 12-22, 12-20, 12-18, 12-16 or 12-14.

The alginate oligomer of the invention may have a degree of polymerisation (DP), or a number average degree of polymerisation (DPₙ), of 15-50, 15-40, 15-35, 15-30, 15-28, 15-25, 15-22, 15-20, 15-18 or 15-16.

The alginate oligomer of the invention may have a degree of polymerisation (DP), or a number average degree of polymerisation (DPₙ), of 18-50, 18-40, 18-35, 18-30, 18-28, 18-25, 18-22 or 18-20.

Preferably the alginate oligomer of the invention is substantially free, preferably essentially free, of alginate oligomers having a degree of polymerisation outside of the ranges disclosed herein. This may be expressed in terms of the molecular weight distribution of the alginate oligomer of the invention, e.g. the percentage of each mole of the alginate oligomer being used in accordance with the invention which has a DP outside the relevant range. The molecular weight distribution is preferably such that no more than 10%, preferably no more than 9, 8, 7, 6, 5, 4, 3, 2, or 1% mole has a DP of three, two or one higher than the relevant upper limit for DPₙ. Likewise it is preferred that no more than 10%, preferably no more than 9, 8, 7, 6, 5, 4, 3, 2, or 1% mole has a DP below a number three, two or one smaller than the relevant lower limit for DPₙ.

Suitable alginate oligomers are described in WO2007/039754, WO2007/039760, WO 2008/125828, and WO2009/068841.

Representative suitable alginate oligomers have a DPₙ in the range 5 to 30, a guluronate/galacturonate fraction (F_{G}) of at least 0.80, a mannuronate fraction (F_{M}) of no more than 0.20, and at least 95 mole% of DP no more than 25.

Further suitable alginate oligomers have a number average degree of polymerization in the range 7 to 15 (preferably 8 to 12), a guluronate/galacturonate fraction (F_{G}) of at least 0.85 (preferably at least 0.90), a mannuronate fraction (F_{M}) of no more than 0.15 (preferably no more than 0.10), and having at least 95% mole with a degree of polymerization less than 17 (preferably less than 14).

Further suitable alginate oligomers have a number average degree of polymerization in the range 5 to 18 (especially 7 to 15), a guluronate/galacturonate fraction (F_{G}) of at least 0.80 (preferably at least 0.85, especially at least 0.92), a mannuronate fraction (F_{M}) of no more than 0.20 (preferably no more than 0.15, especially no more than 0.08), and having at least 95% mole with a degree of polymerization less than 20 (preferably less than 17).

Further suitable alginate oligomers have a number average degree of polymerization in the range 5 to 18, a guluronate/galacturonate fraction (F_{G}) of at least 0.92, a mannuronate fraction (F_{M}) of no more than 0.08, and having at least 95% mole with a degree of polymerization less than 20.

Further suitable alginate oligomers have a number average degree of polymerization in the range 5 to 18 (preferably 7 to 15, more preferably 8 to 12, especially about 10), a guluronate/galacturonate fraction (F_{G}) of at least 0.80 (preferably at least 0.85, more preferably at least 0.90, especially at least 0.92, most especially at least 0.95), a mannuronate fraction (F_{M}) of no more than 0.20 (preferably no more than 0.15, more preferably no more than 0.10, especially no more than 0.08, most especially no more than 0.05), and having at least 95% mole with a degree of polymerization less than 20 (preferably less than 17, more preferably less than 14).

Further suitable alginate oligomers have a number average degree of polymerization in the range 7 to 15 (preferably 8 to 12), a guluronate/galacturonate fraction (F_{G}) of at least 0.92 (preferably at least 0.95), a mannuronate fraction (F_{M}) of no more than 0.08 (preferably no more than 0.05), and having at least 95% mole with a degree of polymerization less than 17 (preferably less than 14).

Further suitable alginate oligomers have a number average degree of polymerization in the range 5 to 18, a guluronate/galacturonate fraction (F_{G}) of at least 0.80, a mannuronate fraction (F_{M}) of no more than 0.20, and having at least 95% mole with a degree of polymerization less than 20.

Further suitable alginate oligomers have a number average degree of polymerization in the range 7 to 15, a guluronate/galacturonate fraction (F_{G}) of at least 0.85, a mannuronate fraction (F_{M}) of no more than 0.15, and having at least 95% mole with a degree of polymerization less than 17.

Further suitable alginate oligomers have a number average degree of polymerization in the range 7 to 15, a guluronate/galacturonate fraction (F_{G}) of at least 0.92, a mannuronate fraction (F_{M}) of no more than 0.08, and having at least 95% mole with a degree of polymerization less than 17.

Further suitable alginate oligomers have a number average degree of polymerization in the range 5 to 20, a guluronate fraction (F_{G}) of at least 0.85 and a mannuronate fraction (F_{M}) of no more than 0.15.

It will thus be seen that a particular class of alginate oligomers favoured according to the present invention is alginate oligomers defined as so-called "high G" or "G-block" oligomers i.e. having a high content of G residues or G-blocks (e.g. wherein at least 80% of the monomer residues are G, preferably arranged in G-blocks). However, other types of alginate oligomer exist, including in particular "high M" or "M-block" oligomers or MG-block oligomers, as described further below. Oligomers wherein at least 80% of the monomer residues in the oligomer are G residues linked 1-4 to another G-residue, or more preferably at least 85, 90, 92, 93, 94, 95, 96, 97, 98, 99% of the monomers residues of the oligomer are G residues linked 1-4 to another G residue may be used in accordance with the invention. This 1-4 linkage of two G residues can be alternatively expressed as a guluronic unit bound to an adjacent guluronic unit.

In other alginate oligomers at least, or more particularly more than, 50% of the monomer residues of the alginate oligomer may be M residues (i.e. mannuronate or mannuronic acid). In other words such alginate oligomers may contain at least or alternatively more than 50% mannuronate (or mannuronic acid) residues. Specific examples thus include alginate oligomers with (e.g. containing) 50 to 70% M (mannuronate) residues or e.g. 70 to 100% M (mannuronate) residues. Further specific examples also include oligomers containing 71 to 85% M residues or 85 to 100% M residues. Thus, an alginate oligomer may contain more than 70% M residues (i.e. more than 70% of the monomer residues of the alginate oligomer will be M residues).

In other examples at least 50% or 60%, more particularly at least 70% or 75%, even more particularly at least 80, 85, 90, 95 or 99% of the monomer residues are mannuronate. In one example the alginate oligomer may be an oligomannuronate (i.e. a homooligomer of M, or 100% M).

In a further example the above described alginates of the invention have a primary structure wherein the majority of the M residues are in so called M-blocks. In such examples preferably at least 50%, more preferably at least 70 or 75%, and most preferably at least 80, 85, 90 or 95% of the M residues are in M-blocks. An M block is a contiguous sequence of at least two M residues, preferably at least 3 contiguous M residues, more preferably at least 4 or 5 contiguous M residues, most preferably at least 7 contiguous M residues.

In particular, at least 90% of the M residues are linked 1-4 to another M residue. More particularly at least 95%, more preferably at least 98%, and most preferably at least 99% of the M residues of the alginate may be linked 1-4 to another M residue.

Other oligomers are alginate oligomers wherein at least 70% of the monomer residues in the oligomer are M residues linked 1-4 to another M-residue, or more preferably at least 75%, and most preferably at least 80, 85, 90, 92, 93, 94, 95, 96, 97, 98, 99% of the monomers residues of the oligomer are M residues linked 1-4 to another M residue. This 1-4 linkage of two M residues can be alternatively expressed as a mannuronic unit bound to an adjacent mannuronic unit.

In a still further embodiment, the alginate oligomers of the invention comprise a sequence of alternating M and G residues. A sequence of at least three, preferably at least four, alternating M and G residues represents an MG block. Preferably the alginate oligomers of the invention comprise an MG block. Expressed more specifically, an MG block is a sequence of at least three contiguous residues consisting of G and M residues and wherein each non-terminal (internal) G residue in the contiguous sequence is linked 1-4 and 4-1 to an M residue and each non-terminal (internal) M residue in the contiguous sequence is linked 1-4 and 4-1 to a G residue. Preferably the MG block is at least 5 or 6 contiguous residues, more preferably at least 7 or 8 contiguous residues.

In a further embodiment the minority uronate in the alginate oligomer (i.e. mannuronate or guluronate) is found predominantly in MG blocks. In this embodiment preferably at least 50%, more preferably at least 70 or 75% and most preferably at least 80, 85, 90 or 95% of the minority uronate monomers in the MG block alginate oligomer are present in MG blocks. In another embodiment the alginate oligomer is arranged such that at least 50%, at least 60%, at least 70%, at least 80%, at least 85%, at least 90%, at least 95%, at least 99%, e.g. 100% of the G and M residues in the oligomer are arranged in MG blocks.

MG block containing alginate oligomers may contain at least 1%, but less than 100%, guluronate (or guluronic acid) residues, but generally MG block containing alginate oligomers will contain at least 30% (or at least 35, 40 or 45% or 50% G) but less than 100% G. Specific examples thus include MG block containing alginate oligomers with (e.g. containing) 1 to 30% G (guluronate) residues, 30 to 70% G (guluronate) residues or 70 to 99% G (guluronate) residues. Thus, MG block containing alginate oligomers may contain more than 30%, but less than 70%, G residues (i.e. more than 30%, but less than 70%, of the monomer residues of the MG block alginate oligomer will be G residues).

Preferably more than 30%, more particularly more than 35% or 40%, even more particularly more than 45, 50, 55, 60 or 65%, but in each case less than 70%, of the monomer residues of an MG block containing alginate oligomer may be guluronate. Alternatively, less than 70%, more preferably less than 65% or 60%, even more preferably less than 55, 50, 45, 40 or 35%, but in each case more than 30% of the monomer residues of an MG block containing alginate oligomer may be guluronate. Any range formed by any combination of these values may be chosen. Therefore for instance an MG block containing alginate oligomer can have e.g. between 35% and 65%, 40% and 60% or 45% and 55% G residues.

In another example an MG block containing alginate oligomer may have approximately equal amounts of G and M residues (e.g. ratios between 65% G/35% M and 35% G/65% M, for instance 60% G/40% M and 40% G/60% M; 55% G/45% M and 45% G/55% M; 53% G/47% M and 47% G/53% M; 51% G/49% M and 49% G/51% M; e.g. about 50% G and about 50% M) and these residues are arranged predominantly, preferably entirely or as completely as possible, in an alternating MG pattern (e.g. at least 50% or at least 60, 70, 80, 85, 90 or 95% or 100% of the M and G residues are in an alternating MG sequence).

In certain embodiments the terminal uronic acid residues of the oligomers of use in the invention do not have a double bond, especially a double bond situated between the C₄ and C₅ atom. Such oligomers may be described as having saturated terminal uronic acid residues. The skilled man would be able to prepare oligomers with saturated terminal uronic acid residues without undue burden. This may be through the use of production techniques which yield such oligomers, or by converting (saturating) oligomers produced by processes that yield oligomers with unsaturated terminal uronic acid residues.

The alginate oligomer will typically carry a charge and so counter ions for the alginate oligomer may be any physiologically tolerable ion, especially those commonly used for charged drug substances, e.g. sodium, potassium, ammonium, chloride, mesylate, meglumine, etc. Ions which promote alginate gelation e.g. group 2 metal ions may also be used.

While the alginate oligomer may be a synthetic material generated from the polymerisation of appropriate numbers of guluronate and mannuronate residues, the alginate oligomers of use in the invention may conveniently be obtained, produced or derived from natural sources such as those mentioned above, namely natural alginate source materials.

Polysaccharide to oligosaccharide cleavage to produce the alginate oligomer useable according to the present invention may be performed using conventional polysaccharide lysis techniques such as enzymatic digestion and acid hydrolysis. In one favoured embodiment acid hydrolysis is used to prepare the alginate oligomers of use in the invention. In other embodiments enzymic digestion is used with an additional processing step(s) to saturate the terminal uronic acids in the oligomers.

Oligomers may then be separated from the polysaccharide breakdown products chromatographically using an ion exchange resin or by fractionated precipitation or solubilisation or filtration. US 6,121,441 and WO 2008/125828 describe a process suitable for preparing the alginate oligomers of use in the invention. Further information and discussion can be found in for example in "Handbooks of Hydrocolloids", Ed. Phillips and Williams, CRC, Boca Raton, Florida, USA, 2000.

The alginate oligomers may also be chemically modified, including but not limited to modification to add charged groups (such as carboxylated or carboxymethylated glycans) and alginate oligomers modified to alter flexibility (e.g. by periodate oxidation).

Alginate oligomers (for example oligoguluronic acids) suitable for use according to the invention may conveniently be produced by acid hydrolysis of alginic acid from, but not limited to, *Laminaria hyperbora* and *Lessonia nigrescens,* dissolution at neutral pH, addition of mineral acid reduce the pH to 3.4 to precipitate the alginate oligomer (oligoguluronic acid), washing with weak acid, resuspension at neutral pH and freeze drying.

The alginates for production of alginate oligomers of use in the invention can also be obtained directly from suitable bacterial sources e.g. *Pseudomonas aeruginosa* or *Azotobacter vinelandii.*

In embodiments where alginate oligomers which have primary structures in which the majority of the G residues are arranged in G-blocks rather than as single residues are required, algal sources are expected to be most suitable on account of the fact that the alginates produced in these organisms tend to have these structures. The bacterial sources may be more suitable for obtaining alginate oligomers of different structures.

The molecular apparatus involved in alginate biosynthesis in *Pseudomonas fluorescens* and *Azotobacter vinelandii* has been cloned and characterised (WO 94/09124; Ertesvag, H., et al, Metabolic Engineering, 1999, Vol 1, 262-269; WO 2004/011628; Gimmestad, M., *et al (supra)*; Remminghorst and Rehm, Biotechnology Letters, 2006, Vol 28, 1701-1712; Gimmestad, M. et al, Journal of Bacteriology, 2006, Vol 188(15), 5551-5560) and alginates of tailored primary structures can be readily obtained by manipulating these systems.

The G content of alginates (for example an algal source material) can be increased by epimerisation, for example with mannuronan C-5 epimerases from A. *vinelandii* or other epimerase enzymes. Thus, for example *in vitro* epimerisation may be carried out with isolated epimerases from *Pseudomonas* or *Azotobacter,* e.g. AlgG from *Pseudomonas fluorescens* or *Azotobacter vinelandii* or the AlgE enzymes (AlgE1 to AlgE7) from *Azotobacter vinelandii.* The use of epimerases from other organisms that have the capability of producing alginate, particularly algae, is also specifically contemplated. The *in vitro* epimerisation of low G alginates with *Azotobacter vinelandii* AlgE epimerases is described in detail in Ertesvåg *et al* (*supra*) and Strugala et al (Gums and Stabilisers for the Food Industry, 2004, 12, The Royal Society of Chemistry, 84 - 94).

To obtain G-block containing alginates or alginate oligomers, epimerisation with one or more *Azotobacter vinelandii* AlgE epimerases other than AlgE4 is preferred as these enzymes are capable of producing G block structures. On the other hand AlgE4 epimerase can be used to create alginates or alginate oligomers with alternating stretches of M/G sequence or primary structures containing single G residue as it has been found that this enzyme seems preferentially to epimerise individual M residues so as to produce single G residues linked to M residues rather than producing G blocks. Particular primary structures can be obtained by using different combinations of these enzymes.

Mutated versions of these enzymes or homologues from other organisms are also specifically contemplated as of use. WO 94/09124 describes recombinant or modified mannuronan C-5 epimerase enzymes (AlgE enzymes) for example encoded by epimerase sequences in which the DNA sequences encoding the different domains or modules of the epimerases have been shuffled or deleted and recombined. Alternatively, mutants of naturally occurring epimerase enzymes, (AlgG or AlgE) may be used, obtained for example by site directed or random mutagenesis of the AlgG or AlgE genes.

A different approach is to create *Pseudomonas* and *Azotobacter* organisms that are mutated in some or all of their epimerase genes in such a way that those mutants produce alginates of the required structure for subsequent alginate oligomer production, or even alginate oligomers of the required structure and size (or molecular weight). The generation of a number of *Pseudomonas fluorescens* organisms with mutated AlgG genes is described in detail in WO 2004/011628 and Gimmestad, M., *et al,* 2003 (*supra*)*.* The generation of a number of *Azotobacter vinelandii* organisms with mutated AlgE genes is disclosed in Gimmestad, M., *et al,* 2006 (*supra*)*.* The skilled man would be able to use this teaching to produce new mutants that could be used to give rise to the alginate oligomers of use in the invention without undue burden.

A further approach is to delete or inactivate the endogenous epimerase genes from an *Azotobacter* or a *Pseudomonas* organism and then to introduce one or more exogenous epimerase genes, which may or may not be mutated (i.e. may be wild-type or modified) and the expression of which may be controlled, for example by the use of inducible or other "controllable promoters". By selecting appropriate combinations of genes, alginates of predetermined primary structure can be produced.

A still further approach would be to introduce some or all of the alginate biosynthesis machinery of *Pseudomonas* and/or *Azotobacter* into a non-alginate producing organism (e.g. *E. coli*) and to induce the production of alginate from these genetically modified organisms.

When these culture-based systems are used, the primary structure of the alginate or alginate oligomer products can be influenced by the culture conditions. It is well within the capabilities of the skilled man to adjust culture parameters such as temperature, osmolarity, nutrient levels/sources and atmospheric parameters in order to manipulate the primary structure of the alginates produced by a particular organism.

References to "G residues/G" and "M residues/M" or to guluronic acid or mannuronic acid, or guluronate or mannuronate are to be read interchangeably as references to guluronic acid/guluronate and mannuronic acid/mannuronate (specifically α-L-guluronic acid/guluronate and β-D-mannuronic acid/mannuronate), and further include derivatives thereof in which one or more available side chains or groups have been modified without resulting in a capacity to improve the efficacy of an antifungal agent against a fungus and in particular the effectiveness (or efficacy) of an antifungal agent to inhibit the growth and/or viability of a fungus that is substantially lower than that of the unmodified oligomer. Common saccharide modifying groups would include acetyl, sulphate, amino, deoxy, alcohol, aldehyde, ketone, ester and anhydro groups. The alginate oligomers may also be chemically modified to add charged groups (such as carboxylated or carboxymethylated glycans), and to alter flexibility (e.g. by periodate oxidation). The skilled man would be aware of still further chemical modifications that can be made to the monosaccharide subunits of oligosaccharides and these can be applied to the alginate oligomers of use in the invention.

An antifungal agent is any agent that has a biocidal/biostatic activity that is relatively specific and selective for fungi. In accordance with the invention, agents such as antiseptics, disinfectants and sterilisation agents are not considered to be "antifungal agents" because these agents have a broad spectrum of biocidal/biostatic activity in that their activity does not display appreciable specificity or selectivity for fungi over other cell types (e.g. bacteria, protozoa, animal and so on).

An antifungal agent may be referred to as an antimycotic agent and the terms are used herein interchangeably.

In certain contexts, e.g. in certain therapeutic, medical or clinical contexts, an antifungal agent may be an antifungal (or antimycotic) drug, which may be considered to be an antifungal agent that may be administered, including internally, to an animal subject in amounts sufficient to exert an antifungal effect without being deleterious to the long term physical health of the subject.

In certain other embodiments, e.g. in an environmental context, particularly an agricultural, food production, or engineering context, an antifungal agent may be a fungicide (or mycocide), which may be considered to be an antifungal agent that is not designed to be taken internally by an animal and instead exerts its antifungal effects in locations and at sites outside of an animal body, e.g. through application to or incorporation into inanimate (e.g. abiotic) materials, plants, seeds, plant products, food stuffs and so on, or on an uncompromised exterior surface of an animal. Thus, in some instances a fungicide as defined herein may be used in a therapeutic, medical or clinical context, for instance to prevent fungal infection/colonisation on the skin of a subject or on the surfaces of medical equipment and instruments.

In certain instances an antifungal agent does not also display antibacterial activity, i.e. a biocidal/biostatic activity that is relatively specific and selective for bacteria, e.g. an antibacterial antibiotic. In certain embodiments the antifungal agent is not an alginate oligomer as defined herein.

By way of example, antifungal agents include, but are not limited to, polyene antifungals (e.g. natamycin, rimocidin, nystatin, amphotericin B, candicin, hamycin, perimycin); azole antifungals (e.g. imidazole antifungals, in particular, miconazole, ketoconazole, clotrimazole, econazole, omoconazole, bifonazole, butoconazole, fenticonazole, isoconazole, oxiconazole, sertaconazole, sulconazole, tioconazole; triazole antifungals, in particular, fluconazole, fosfluconazole, itraconazole, isavuconazole, ravuconazole, posaconazole, voriconazole, terconazole, albaconazole; thiazole antifungals, in particular, abafungin); allylamine antifungals (e.g. terbinafine, naftifin, butenafine, amorolfine); echinocandin antifungals (e.g. anidulafungin, caspofungin, micafungin); ciclopirox; tolnaftate; and flucytosine. The antifungal drug may be in any convenient form, including any pharmaceutically acceptable salt or hydrate. The references to the antifungal drugs listed above extends to any isomeric form in which the compound may exist as well as mixtures of two or more isomers, e.g. racemic mixtures.

Antifungal agents may be a drug that may be administered systemically, e.g. amphotericin B, hamycin, ketoconazole, fluconazole, fosfluconazole, itraconazole, posaconazole, voriconazole, terbinafine, echinocandin antifungals (e.g. anidulafungin, caspofungin, micafungin), and flucytosine.

Antifungal agents may be a drug that is typically administered as a non-systemic treatment, e.g. as a topical treatment. Representative examples of such drugs include, but are not limited to, natamycin, nystatin, amphotericin B, candicin, hamycin, perimycin, miconazole, ketoconazole, clotrimazole, econazole, omoconazole, bifonazole, butoconazole, fenticonazole, isoconazole, oxiconazole, sertaconazole, sulconazole, tioconazole, fluconazole, fosfluconazole, isavuconazole, ravuconazole, terconazole, albaconazole, abafungin, allylamine antifungals (e.g. terbinafine, naftifin, butenafine, amorolfine), ciclopirox and tolnaftate.

Antifungal agents may be a polyene compound (e.g. natamycin, rimocidin, nystatin, amphotericin B, candicin, hamycin, perimycin), in particular nystatin or amphotericin B. Without wishing to be bound by theory, a possible explanation for the potentiating (e.g. synergistic) effect of alginate oligomers on polyene antifungal agents, e.g. nystatin and amphotericin B, as observed in the Examples, is that alginate oligomers have a direct disruptive effect at the fungal cell membrane, like the polyene antifungal agents, and this combination of disruptive effects is especially detrimental to the integrity of the fungal cell. Moreover, the molecular target of the mode of action of the polyene antifungal agents (ergosterol, which is similar to the sterols in animal cell membranes) is such that the polyene antifungals are especially associated with toxic side effects. As such, methods to improve their effectiveness would be especially valuable as it would allow their use at lower dosages.

Other antifungal agents also exert their antifungal effects through an indirect action on the fungal cell membrane or cell wall. In the case of the azole and allylamine antifungals, the synthesis of ergosterol is inhibited thus depleting the fungal cell membrane of this essential component. Similarly, the echinocandin antifungals inhibit the synthesis of glucan in fungi, which is an essential component of fungal cell walls. Thus it is believed that alginate oligomers may be able to potentiate the effect of such other antifungal agents in a similar way.

In certain embodiments the antifungal agent is not terbinafine or an allylamine antifungal (e.g. naftifin, butenafine, amorolfine).

By way of example, fungicides include, but are not limited to, aliphatic nitrogen fungicides (e.g. butylamine, cymoxanil, dodicin, dodine, guazatine, iminoctadine); amide fungicides (e.g. carpropamid, chloraniformethan, cyflufenamid, diclocymet, diclocymet, dimoxystrobin, fenoxanil, flumetover, furametpyr, isopyrazam, mandipropamid, metominostrobin, orysastrobin, penthiopyrad, prochloraz, quinazamid, silthiofam, triforine, xiwojunan); acylamino acid fungicides (e.g. benalaxyl, benalaxyl-M, furalaxyl, metalaxyl, metalaxyl-M, pefurazoate, valifenalate); anilide fungicides (e.g. benalaxyl, benalaxyl-M, bixafen, boscalid, carboxin, fenhexamid, fluxapyroxad, isotianil, metalaxyl, metalaxyl-M, metsulfovax, ofurace, oxadixyl, oxycarboxin, penflufen, pyracarbolid, sedaxane, thifluzamide, tiadinil, vangard); benzanilide fungicides (e.g. benodanil, flutolanil, mebenil, mepronil, salicylanilide, tecloftalam); furanilide fungicides (e.g. fenfuram, furalaxyl, furcarbanil, methfuroxam); sulfonanilide fungicides (e.g. flusulfamide); benzamide fungicides (e.g. benzohydroxamic acid, fluopicolide, fluopyram, tioxymid, trichlamide, zarilamid, zoxamide); furamide fungicides (e.g. cyclafuramid, furmecyclox); phenylsulfamide fungicides (e.g. dichlofluanid, tolylfluanid); sulfonamide fungicides (e.g. amisulbrom, cyazofamid); valinamide fungicides (e.g. benthiavalicarb, iprovalicarb); antibiotic fungicides (e.g. aureofungin, blasticidin-S, cycloheximide, griseofulvin, kasugamycin, moroxydin, polyoxins, polyoxorim, validamycin); strobilurin fungicides (e.g. fluoxastrobin); methoxyacrylate strobilurin fungicides (e.g. azoxystrobin, bifujunzhi, coumoxystrobin, enestroburin, jiaxiangjunzhi, picoxystrobin, pyraoxystrobin); methoxycarbanilate strobilurin fungicides (e.g. Ivdingjunzhi, pyraclostrobin, pyrametostrobin); methoxyiminoacetamide strobilurin fungicides (e.g. dimoxystrobin, metominostrobin, orysastrobin, xiwojunan); methoxyiminoacetate strobilurin fungicides (e.g. kresoxim-methyl, trifloxystrobin); aromatic fungicides (e.g. biphenyl, chlorodinitronaphthalenes, chloroneb, chlorothalonil, cresol, dicloran, fenjuntong, hexachlorobenzene, pentachlorophenol, quintozene, sodium pentachlorophenoxide, tecnazene); arsenical fungicides (e.g. asomate, urbacide); aryl phenyl ketone fungicides (e.g. metrafenone, yriofenone); benzimidazole fungicides (e.g. albendazole, benomyl, carbendazim, chlorfenazole, cypendazole, debacarb, fuberidazole, mecarbinzid, rabenzazole, thiabendazole); benzimidazole precursor fungicides (e.g. furophanate, thiophanate, thiophanate-methyl); benzothiazole fungicides (e.g. bentaluron, benthiavalicarb, benthiazole, chlobenthiazone, probenazole); botanical fungicides (e.g. allicin, berberine, carvacrol, carvone, osthol, santonin); bridged diphenyl fungicides (e.g. bithionol, dichlorophen, diphenylamine, hexachlorophene, parinol); carbamate fungicides (e.g. benthiavalicarb, furophanate, iodocarb, iprovalicarb, propamocarb, pyribencarb, thiophanate, thiophanate-methyl); benzimidazolylcarbamate fungicides (e.g. albendazole, benomyl, carbendazim, cypendazole, debacarb, mecarbinzid); carbanilate fungicides (e.g. diethofencarb, Ivdingjunzhi, pyraclostrobin, pyrametostrobin); conazole (imidazole) fungicides (e.g. climbazole, clotrimazole, imazalil, oxpoconazole, prochloraz, triflumizole); conazole (triazole) fungicides (e.g. azaconazole, bromuconazole, cyproconazole, diclobutrazol, difenoconazole, diniconazole, diniconazole-M, epoxiconazole, etaconazole, fenbuconazole, fluquinconazole, flusilazole, flutriafol, furconazole, furconazole-cis, hexaconazole, imibenconazole, ipconazole, metconazole, myclobutanil, penconazole, propiconazole, prothioconazole, quinconazole, simeconazole, tebuconazole, tetraconazole, triadimefon, triadimenol, triticonazole, uniconazole, uniconazole-P); copper fungicides (e.g. acypetacs-copper, Bordeaux mixture, Burgundy mixture, Cheshunt mixture, copper acetate, copper carbonate (basic), copper hydroxide, copper naphthenate, copper oleate, copper oxychloride, copper silicate, copper sulfate, copper sulfate (basic), copper zinc chromate, cufraneb, cuprobam, cuprous oxide, mancopper, oxine-copper, saisentong, thiodiazole-copper); cyanoacrylate fungicides (e.g. benzamacril, phenamacril); dicarboximide fungicides (e.g. famoxadone, fluoroimide); dichlorophenyl dicarboximide fungicides (e.g. chlozolinate, dichlozoline, iprodione, isovaledione, myclozolin, procymidone, vinclozolin); phthalimide fungicides (e.g. captafol, captan, ditalimfos, folpet, thiochlorfenphim); dinitrophenol fungicides (e.g. binapacryl, dinobuton, dinocap, dinocap-4, dinocap-6, meptyldinocap, dinocton dinopenton, dinosulfon, dinoterbon, DNOC); dithiocarbamate fungicides (e.g. amobam, asomate, azithiram, carbamorph, cufraneb, cuprobam, disulfiram, ferbam, metam, nabam, tecoram, thiram, urbacide, ziram); cyclic dithiocarbamate fungicides (e.g. dazomet, etem, milneb); polymeric dithiocarbamate fungicides (e.g. mancopper, mancozeb, maneb, metiram, polycarbamate, propineb, zineb); dithiolane fungicides (e.g. isoprothiolane, saijunmao); fumigant fungicides (e.g. dithioether, methyl bromide); hydrazide fungicides (e.g. benquinox, saijunmao); imidazole fungicides (e.g. cyazofamid, fenamidone, fenapanil, glyodin, iprodione, isovaledione, pefurazoate, triazoxide); inorganic fungicides (potassium azide, potassium thiocyanate, sodium azide, sulfur); inorganic mercury fungicides (e.g. mercuric chloride, mercuric oxide, mercurous chloride); organomercury fungicides (e.g. (3-ethoxypropyl)mercury bromide, ethylmercury acetate, ethylmercury bromide, ethylmercury chloride, ethylmercury 2,3-dihydroxypropyl mercaptide, ethylmercury phosphate, N-(ethylmercury)-p-toluenesulphonanilide hydrargaphen, 2-methoxyethylmercury chloride, methylmercury benzoate, methylmercury dicyandiamide, methylmercury pentachlorophenoxide, 8-phenylmercurioxyquinoline, phenylmercuriurea, phenylmercury acetate, phenylmercury chloride, phenylmercury derivative of pyrocatechol, phenylmercury nitrate, phenylmercury salicylate, thiomersal, tolylmercury acetate); morpholine fungicides (e.g. aldimorph, benzamorf, carbamorph, dimethomorph, dodemorph, fenpropimorph, flumorph, tridemorph); organophosphorus fungicides (e.g. ampropylfos, ditalimfos, EBP, edifenphos, fosetyl, hexylthiofos, inezin, iprobenfos izopamfos, kejunlin, phosdiphen, pyrazophos, tolclofos-methyl, triamiphos); organotin fungicides (e.g. decafentin, fentin, tributyltin oxide); oxathiin fungicides (e.g. carboxin, oxycarboxin); oxazole fungicides (e.g. chlozolinate, dichlozoline, dingjunezuo, drazoxolon, famoxadone, hymexazol, metazoxolon, myclozolin, oxadixyl, vinclozolin); polysulfide fungicides (e.g. barium polysulfide, calcium polysulfide, potassium polysulfide, sodium polysulfide); pyrazole fungicides (e.g. bixafen, fenpyrazamine, fluxapyroxad, furametpyr, isopyrazam, penflufen, penthiopyrad, pyraclostrobin, pyrametostrobin, pyraoxystrobin, rabenzazole, sedaxane); pyridine fungicides (e.g. boscalid, buthiobate, dingjunezuo, dipyrithione, fluazinam, fluopicolide, fluopyram, Ivdingjunzhi, parinol, pyribencarb, pyridinitril, pyrifenox, pyroxychlor, pyroxyfur); pyrimidine fungicides (e.g. bupirimate, diflumetorim, dimethirimol, ethirimol, fenarimol, ferimzone, nuarimol, triarimol); anilinopyrimidine fungicides (e.g. cyprodinil, mepanipyrim, pyrimethanil); pyrrole fungicides (e.g. dimetachlone, fenpiclonil, fludioxonil, fluoroimide); quaternary ammonium fungicides (e.g. berberine); quinoline fungicides (e.g. ethoxyquin, halacrinate, 8-hydroxyquinoline sulfate, quinacetol, quinoxyfen, tebufloquin); quinone fungicides (e.g. chloranil, dichlone, dithianon); quinoxaline fungicides (e.g. chinomethionat, chlorquinox, thioquinox); thiadiazole fungicides (e.g. etridiazole, saisentong, thiodiazole-copper, zinc thiazole); thiazole fungicides (e.g. ethaboxam, isotianil, metsulfovax, octhilinone, thiabendazole, thifluzamide); thiazolidine fungicides (e.g. flutianil, thiadifluor); thiocarbamate fungicides (e.g. methasulfocarb, prothiocarb); thiophene fungicides (e.g. ethaboxam, silthiofam); triazine fungicides (e.g. anilazine); triazole fungicides (e.g. amisulbrom, bitertanol, fluotrimazole, huanjunzuo, triazbutil); triazolopyrimidine fungicides (e.g. ametoctradin); urea fungicides (e.g. bentaluron, pencycuron, quinazamid); zinc fungicides (e.g. acypetacs-zinc, copper zinc chromate, cufraneb, mancozeb, metiram, polycarbamate, polyoxorim-zinc, propineb, zinc naphthenate, zinc thiazole, zineb, ziram); acibenzolar, acypetacs, allyl alcohol, benzalkonium chloride, bethoxazin, bromothalonil, chitosan, chloropicrin, DBCP, dehydroacetic acid, diclomezine, diethyl pyrocarbonate, ethylicin, fenaminosulf, fenitropan, fenpropidin, furfural, hexachlorobutadiene, methyl iodide, methyl isothiocyanate, nitrostyrene, nitrothal-isopropyl, OCH, 2-phenylphenol, phthalide, piperalin, propamidine, proquinazid, pyroquilon, sodium orthophenylphenoxide, spiroxamine, sultropen, thicyofen or tricyclazole. The fungicide may be in any convenient form, including any functionally acceptable salt or hydrate. The references to the fungicides listed above extends to any isomeric form in which the compound may exist as well as mixtures of two or more isomers, e.g. racemic mixtures.

In certain examples the fungicide is not copper hydroxide, cresol, dichlorophen, dipyrithione, dodicin, ethylicin, fenaminosulf, hexachlorophene, hydrargaphen, 8-hydroxyquinoline sulfate, kasugamycin, octhilinone, probenazole, saisentong, tecloftalam, thiodiazole-copper, thiomersal or zinc thiazole.

As used herein the term fungus includes any fungus, e.g. any eukaryotic organism with a cell wall containing chitin, or any organism classified as belonging to the taxonomic kingdom Fungi. More specifically a fungus may be a member of the taxonomic phyla Ascomycota (i.e. from the taxonomic class Neolectomycetes, Pneumocystidomycetes, Schizosaccharomycetes, Taphrinomycetes, Arthoniomycetes, Dothideomycetes, Geoglossomycetes, Eurotiomycetes, Laboulbeniomycetes, Lecanoromycetes, Leotiomycetes, Lichinomycetes, Orbiliomycetes, Pezizomycetes, Sordariomycetes, Saccharomycetes); Basidiomycota (i.e. from the taxonomic class Agaricomycetes, Dacrymycetes, Tremellomycetes, Agaricostilbomycetes, Attractiellomycetes, Classiculomycetes, Cryptomycocolacomycetes, Cystobasidiomycetes, Microbotryomycetes, Mixiomycetes, Pucciniomycetes, Ustilaginomycetes, or Exobasidiomycetes); Chytridiomycota (i.e. from the taxonomic class Chytridiomycetes or Monoblepharidomycetes); Glomeromycota (i.e. from the taxonomic class Glomeromycetes); Zygomycota (i.e. from the taxonomic class Trichomycetes or Zygomycetes); Microsporidia (i.e. from the taxonomic class Aquasporidia, Marinosporidia or Terresporidia); Blastocladiomycota (i.e. from the taxonomic class Blastocladiomycetes); and Neocallimastigomycota (i.e. from the taxonomic class Neocallimastigomycetes). The term "fungus" extends to the spores that may be produced by certain species of fungus, e.g. the "fungus" may be a sporangiospore, a zygospore, an acospore, a basidiospore, an aeciospore, a urediospore, a teliospore, a conidiospore, or a mitospore.

A fungus may be a unicellular species or a species that may exist in a unicellular form at some point in its lifecycle. A fungus may therefore be a yeast. A fungus may be a species that exists as a part of a multicelled hyphae or mycelium or a species that may exist as a part of a multicelled hyphae or mycelium at some point in its lifecycle. The multicelled hyphae or mycelium may be microscopic or macroscopic. A fungus may therefore be a mould or a mushroom. Of note are fungus that are dimorphic, i.e. they may exist in a unicellular (yeast) form under certain conditions (e.g. at certain levels of nutrients, carbon dioxide, oxygen, pH, temperature, etc.) and may exist as part of a multicelled hyphae or mycelium under certain other conditions or different levels of the abovementioned conditions. It is common for a fungus that is an animal pathogen to exist in the environment as a part of a multicelled hyphae or mycelium, but in a unicellular form in the animal. Conversely, it is common for a fungus that is a plant pathogen to exist in the environment in a unicellular form, but as a part of a multicelled hyphae or mycelium on or in the plant.

A fungus may be an animal and/or a plant pathogen, a animal and/or a plant parasite, or involved in the spoilage or decomposition of organic materials (e.g. foodstuffs and cellulose-based products). A fungus may be an opportunistic pathogen in that usually it is benign to healthy subjects with an uncompromised immune system, but such a fungus can establish an infection in subjects whose immune system is compromised in some way. A fungus may also be a fungus that produces a mycotoxin that affects animals, typically by poisoning them or inducing allergic reactions.

By way of example a fungus may be the causative agent of an aspergillosis (i.e. fungi from the taxonomic genus Aspergillus, e.g. *Aspergillus fumigatus, Aspergillus flavus, Aspergillus clavatus, Aspergillus terrus, Aspergillus niger);* a candidiasis (i.e. fungi from the taxonomic genus Candida, e.g. *Candida albicans, Candida glabrata, Candida tropicalis, Candida lusitaniae, Candida dubliniensis, Candida parapsilossis, Candida krusei, Candida rugosa*); a coccidioidomycosis (e.g. *Coccidioides immitis, Coccidioides posadasii*); a cryptococcosis (e.g. *Cryptococcus neoformans, Cryptococcus gattii, Cryptococcus laurentii, Cryptococcus albidus*); a histoplasmosis (e.g. *Histoplasma capsulatum, Histoplasma duboisii*); blastomycosis (e.g. *Blastomyces dermatitidis)*; a mycetoma (e.g. *Actinomadura pelletieri, Acremonium strictum, Actinomadura madurae, Aspergillus nidulans, Noetestudina rosatii, Phaeoacremonium krajdenii, Pseudallescheria boydii, Curvularia lunata, Exophiala jeanselmei, Leptosphaeria senegalensis, Leptosphaeria tompkinsii, Madurella grisea, Madurella mycetomatis, Pyrenochaeta romeroi*); paracoccidioidomycosis (e.g. *Paracoccidioides brasiliensis*); pneumocystosis (e.g. *Pneumocystis jirovecii*); fusariosis (e.g. the *Fusarium solani complex: Fusarium oxysporum, Fusarium verticillioides, Fusarium proliferatum, Fusarium monilifrome*); a phaeohyphomycosis (e.g. fungi from the genus Alternaria, *Exophiala jeanselmei*); an alternariosis (i.e. fungi from the genus Alternaria, e.g. *Alternaria alternata*); rhinosporidiosis (e.g. *Rhinosporidium seeberi*); a microsporidiosis (e.g. *Enterocytozoon bieneusi, Encephalitozoon intestinalis*); basidiobolomycosis (e.g. *Basidiobolus ranarum*); a conidiobolomycosis (e.g. *Conidiobolus coronatus, Conidiobolus incongruus*); a mucormycosis (e.g. *Rhizopus oryzae, Mucor indicus Absidia corymbifera, Syncephalastrum racemosum*); a trichosporonosis (e.g. *Trichosporon spp, Trichosporon asahii, Trichosporon inkin, Trichosporon asteroides, Trichosporon cutaneum, Trichosporon mucoides, Trichosporon ovoides, Trichosporon pullulans, Trichosporon loubieri, Trichosporon japonicum);* a chromoblastomycosis (*Fonsecaea pedrosoi, Fonsecaea compacta, Phialophora verrucosa*); geotrichosis (e.g. *Geotrichum candidum*); allescheriasis (e.g. *Pseudallescheria boydii*); sporotrichosis (e.g. *Sporothrix schenckii*); penicilliosis (e.g. *Penicillium marneffei*); lobomycosis (e.g. *Lacazia loboi*); a dermatophytosis (i.e. fungi from the genera Epidermophyton, Microsporum and Trichophyton, *e.g. Epidermophyton floccosum, Microsporum canis, Microsporum audouinii, Microsporum gypseum, Trichophyton interdigitale*/*mentagrophytes, Trichophyton verrucosum, Trichophyton violaceum Trichophyton canis, Trichophyton tonsurans, Trichophyton schoenleini, Trichophyton rubrum, Trichophyton concentricum*); a piedra (e.g. *Hortaea werneckii, Piedraia hortae, Malassezia furfur, Trichosporon spp, Trichosporon beigelii*)*,* a pityrosporum folliculitis / malassezia folliculitis (i.e. fungi from the genera Malassezia, e.g. *Malassezia globosa, Malassezia restricta*). Other fungal species capable of acting as an animal pathogen include, *Malassezia pachydermatis, Scedosporium prolificans, Acremonium kiliense,* and *Paecilomyces lilacinus*

Preferably a fungus is a species from the taxonomic genus Candida, e.g. *Candida albicans, Candida glabrata, Candida tropicalis, Candida lusitaniae, Candida dubliniensis, Candida parapsilossis, Candida krusei* and *Candida rugosa,* in particular *Candida albicans, Candida glabrata, Candida tropicalis, Candida parapsilosis, Candida krusei* and *Candida lusitaniae,* and most particularly *Candida albicans.*

Preferably a fungus is a species from the taxonomic genus Aspergillus, e.g. *Aspergillus niger, Aspergillus fumigatus, Aspergillus flavus, Aspergillus clavatus* and *Aspergillus terrus,* in particular *Aspergillus flavus.*

Preferably a fungus is a species from the taxonomic genus Cryptococcus, e.g. *Cryptococcus neoformans, Cryptococcus gattii, Cryptococcus laurentii, Cryptococcus albidus,* in particular *Cryptococcus neoformans.*

Preferably a fungus is a species from the taxonomic genera Malassezia (e.g. *Malassezia pachydermatis* or *Malassezia furfur*), Trichosporon (e.g. *Trichosporon cutaneum),* Fusarium (e.g. the *Fusarium solani complex: Fusarium oxysporum, Fusarium verticillioides, Fusarium proliferatum, Fusarium monilifrome),* Acremonium (e.g. *Acremonium kiliense, Acremonium strictum*), Paecilomyces (e.g. *Paecilomyces lilacinus*), Rhizopus (e.g. *Rhizopus oryzae*), Mucor (e.g. *Mucor indicus),* Scedosporium (e.g. *Scedosporium prolificans*) and Absidia (e.g. *Absidia corymbifera*).

By way of further example a fungus may be the causative agent of cankers/anthracnose (e.g. apple canker, *Nectria galligena;* butternut canker, *Sirococcus clavigignenti-juglandacearum;* cypress canker, *Seiridium cardinale;* dogwood anthracnose, *Discula destructiva;* honey locust canker, *Thyronectria austro-americana;* mulberry canker, *Gibberella baccata;* oak canker, *Diplodia quercina;* pine pitch canker, *Fusarium pini;* plane anthracnose, *Apiognomonia veneta;* rapeseed stem canker, *Leptosphaeria maculans;* rose canker, *Leptosphaeria coniothyrium* and *Cryptosporella umbrina;* scleroderris canker, *Gremmeniella abietina;* willow anthracnose, *Marssonina salicicola*); brown rot of stone fruits (e.g. *Monilinia fructicolal*); Phomopsis leaf (e.g. *Phomopsis viticola*); kole-roga (e.g. *Phytophthora palmivora*); botrytis bunch rot (e.g. *Botrytis cinerea*); black mould (e.g. *Aspergillus niger*); bitter rot (e.g. *Glomerella cingulata*); cladosporium rot / soft rot (e.g. *Cladosporium cladosporioides*); kernal rot / fusariosis on maize (e.g. *Fusarium sporotrichioides*); sour rot (e.g. *Geotrichum candidum*); strawberry fruit rot (*Pestalotia longisetula*); rust (e.g. fungi from the families Chaconiaceae, Coleosporiaceae, Cronartiaceae, Melampsoraceae, Mikronegeriaceae, Phakopsoraceae, Phragmidiaceae, Pileolariaceae, Pucciniaceae, Pucciniosiraceae, Pucciniastraceae, Raveneliaceae, Sphaerophragmiaceae, Uropyxidaceae, in particular, *Gymnosporangium juniperivirginianae, Cronartium ribicola, Hemileia vastatrix, Puccinia graminis, Puccinia coronata, Phakopsora meibomiae, Phakopsora pachyrhizi, Uromyces phaseoli, Puccinia hemerocallidis, Puccinia persistens subsp. triticina, Puccinia sriiformis, Puccinia graminis, Uromyces appendeculatus*); apple scab (e.g. *Venturia inaequalis*); black scab (e.g. Synchytrium endobioticum); Fusarium head blight (e.g. fungi from the genus Fusarium, in particular *Fusarium avenaceum, Fusarium culmorum, Fusarium graminearum, Fusarium poae, Fusarium nivale*); pear scab, (e.g. *Venturia pirina, Fusicladium pyrorum*); poinsettia scab (e.g. *Sphaceloma poinsettiae*); powdery mildew (e.g. fungi from the family Erysiphaceae, in particualr *Erysiphe necator, Blumeria graminis, Leveillula taurica, Podosphaera leucotricha, Podosphaera fusca, Microsphaera syringae, Podosphaera aphanis, Sawadaea tulasnei*); Black Sigatoka (e.g. *Mycosphaerella fijiensis*); Yellow Sigatoka (e.g. *Mycosphaerella musicola*), sugarcane smut (e.g. *Sporisorium scitamineum*); corn smut (e.g. *Ustilago maydis*); loose smut of barley (e.g. *Ustilago nuda*); loose smut of wheat (e.g. *Ustilago tritici*); covered smut of barley (e.g. *Tilletia tritici, Tilletia. laevis, Ustilago hordei*); TCK smut (e.g. *Tilletia controversa*); false smut of rice (e.g. *Ustilaginoidea virens*); loose smut of oats (e.g. *Ustilago avenae*); frogeye leaf spot (e.g. *Botryosphaeria obtusa*); sheath blight (e.g. *Rhizoctonia solani*); or rice blast (e.g. *Pyricularia grisea* or *Magnaporthe grisea*)*.*

Many plant pathogens are found in the genera Fusarium, Ustilago, Alternaria, and Cochliobolus. Examples of plant pathogenic species within those genera include, but are not limited to *Fusarium graminearum, Fusarium oxysporum f.sp. cubense, Fusarium avenaceum, Fusarium culmorum, Fusarium graminearum, Fusarium poae, Fusarium nivale, Ustilago maydis, Ustilago nuda, Ustilago tritici, Ustilago hordei, Ustilaginoidea virens, Ustilago avenae, Alternaria alternata, Alternaria arborescens, Alternaria arbusti, Alternaria blumeae, Alternaria brassica, Alternaria brassicicola, Alternaria brunsii, Alternaria carotiincultae, Alternaria conjuncta, Alternaria euphorbiicola, Alternaria gaisen, Alternaria infectoria, Alternaria japonica, Alternaria panax, Alternaria petroselini, Alternaria radicina, Alternaria raphani, Alternaria saponariae, Alternaria selini, Alternaria solani, Alternaria smyrnii, Cochliobolus carbonum, Cochliobolus heterostrophus, Cochliobolus lunatus* and *Cochliobolus stenospilus.*

By way of still further example a fungus may be a wood decay fungus (e.g. *Serpula lacrymans, Meruliporia incrassata* (both true dry rot), *Fibroporia vaillantii* (mine fungus), and *Coniophora puteana* (cellar fungus), *Phellinus contiguus,* a penicillium rot (e.g. *Penicillium chrysogenum*), bread mould (*Rhizopus stolonifer*) or a soft rot/blue mould (e.g. *Penicillium expansum*)*.*

Myctoxin producing fungi include those that produce an aflatoxin (e.g. Aspergillus species, in particular, *Aspergillus flavus* and *Aspergillus parasiticus*)*,* an ochratoxin (e.g. Aspergillus and Penicillium species, in particular, *Aspergillus ochraceus, Aspergillus carbonarius* and *Penicillium viridicatum*), a citrinin (e.g. Aspergillus, Monascus and Penicillium species, in particular, *Aspergillus niveus, Aspergillus ochraceus, Aspergillus oryzae, Aspergillus terreus, Monascus ruber, Monascus purpureus, Penicillium citrinum, Penicillium camemberti*)*,* an ergot alkaloid (e.g. Claviceps species, in particular, *Claviceps africana, Claviceps fusiformis, Claviceps paspali, Claviceps purpurea*)*,* a patulin (e.g. Aspergillus and Penicillium species, in particular, *Penicillium expansum*), a trichothecene (e.g. Fusarium, Myrothecium, Trichoderma, Trichothecium, Cephalosporium, Verticimonosporium, and Stachybotrys species), or a fusarium toxin, which includes fumonisins, trichothecenes, zearalenone, beauvercin and enniatins, butenolide, equisetin, and fusarinspatulin (e.g. Fusarium species).

"Improving the efficacy of the antifungal agent" includes any aspect of improving or enhancing the anti-fungal effect of the anti-fungal agent, e.g. so that the anti-fungal effect of the anti-fungal agent is increased or enhanced in any way over the anti-fungal effect of the anti-fungal agent seen in the absence of the alginate oligomer. This may be seen for example in a stronger effect of the anti-fungal agent in inhibiting growth and/or viability of the fungi, a requirement for less anti-fungal agent in order to achieve the same effect seen in the absence of alginate oligomer, or a increased effectiveness seen as increased speed or rate of action, an inhibitory effect being seen in less time than in the absence of oligomer.

Accordingly in certain embodiments of the various aspects of the invention above, the amount of antifungal agent (e.g. used) is less than the amount in the absence of the alginate oligomer. Thus to achieve the same (or an equivalent or comparable antifungal effect etc.) less antifungal agent is used or required. An advantage of the present invention is thus that the dose of the antifungal agent may be reduced as compared to the dose used in the absence of the alginate oligomer.

The references to "improving the effectiveness of an anti-fungal agent to inhibit the growth and/or viability of a fungus" etc. accordingly may include that the alginate oligomer renders the anti-fungal agent, at least twice as, or at least four times, at least eight times, at least sixteen times or at least thirty two times more effective at inhibiting fungal growth (e.g. acting as a fungistatic agent). Put in a different way, the oligomer may at least double, at least quadruple, at least octuple, at least sexdecuple or at least duotrigenuple the effectiveness of the anti-fungal agent to inhibit growth of the fungi. The inhibitory effect of the anti-fungal agent against a particular fungus can be measured by assessing the Minimum Inhibitory Concentration (MIC) of that antifungal agent for that fungus (Jorgensen et al., Manual of Clinical Microbiology, 7th ed. Washington, D.C: American Society for Microbiology, 1999; 1526-43), i.e. that concentration of anti-fungal agent that completely inhibits growth of that fungus. A halving of the MIC corresponds to a doubling in the inhibitory effect of the anti-fungal agent. A quartering of the MIC corresponds to a quadrupling of the inhibitory effect. As can be seen from the Examples, alginate oligomers and antifungal agents have a combinatorial, e.g. synergistic, effect that makes fungi more susceptible to that antifungal agent. In one embodiment the alginate oligomer will measurably reduce the MIC value of the antifungal agent for the fungus, e.g. the MIC value will be at least 50%, 25%, 20%, 15%, 10%, 5%, 2% or 1% of the MIC value of the antifungal agent for the fungus before treatment in accordance with the invention.

This invention also allows the concentration of the anti-fungal agent administered to a subject or applied to a site or location to be reduced whilst maintaining the same effectiveness. This can be beneficial if the anti-fungal agent is expensive or associated with side effects (such as is often the case with antifungal drugs). Minimising the use of anti-fungal agents is also desirable to minimise development of resistance. In accordance with the invention the use of an alginate oligomer as described above, e.g. at the same or substantially the same time or prior to administering the anti-fungal agent permits the anti-fungal agent to be used at a concentration that is less than 50%, less than 25%, less than 10% or less than 5% of the amount normally administered/applied to achieve a particular level of inhibition of the growth of fungi in the absence of the alginate oligomer.

Thus use of alginate oligomers according to the present invention may potentiate the effect of an antifungal agent (or increase or improve its efficacy) and so it may enable an antifungal agent already known to be effective against a particular fungus to be used at a reduced dose. It may also render usable (or effective) an antifungal agent previously thought not to be usable/effective against a particular fungus, or an antifungal agent which is not normally effective against a given fungus. Put differently, it may overcome the resistance of a fungus to an antifungal agent.

In this regard the disclosure herein may be considered to provide the following:
A method of overcoming resistance to at least one antifungal agent in an fungus said method comprising contacting said fungus with (particularly with an effective amount of) an alginate oligomer together with (particularly with an effective amount of) the antifungal agent. This method may be an *in vitro* or an *ex vivo* method.

An alginate oligomer for use together with at least one antifungal agent in treating a subject infected, suspected to be infected, or at risk of infection, with a fungus that is resistant to said antifungal agent to overcome resistance to the antifungal agent in said fungus.

Use of an alginate oligomer for the manufacture of a medicament for use together with at least one antifungal agent in treating subject infected, suspected to be infected, or at risk of infection, with a fungus that is resistant to said antifungal agent to overcome resistance to the antifungal agent in said fungus.

A product containing an alginate oligomer and an antifungal agent as a combined preparation for separate, simultaneous or sequential use in treating a subject infected, suspected to be infected, or at risk of infection, with a fungus that is resistant to said antifungal agent to overcome resistance to the antifungal agent in said fungus.

A method for combating colonisation of a site with a fungus that is resistant to an antifungal agent, said method comprising contacting said site and/or said fungus with (particularly with an effective amount of) an alginate oligomer together with (particularly with an effective amount of) the antifungal agent to which said fungus is resistant. Such a method may particularly be an *in vitro* or an *ex vivo* method.

A method to combat a fungus that is resistant to an antifungal agent (which includes a population of fungi as well as an individual or single fungus or fungal cell), said method comprising contacting said fungus or a site at which said fungus is or may be located with (particularly with an effective amount of) an alginate oligomer together with (particularly with an effective amount of) the antifungal agent to which said fungus is resistant. Such a method may particularly be an *in vitro* method.

In these methods there may be a step in which it is determined (e.g. ascertained or identified) that the fungus is resistant to a particular antifungal agent(s). In a step in place of, or in addition to, the previously described step, there may be a step in which it is determined that the fungus is a fungus that is already known to be resistant to an antifungal agent. Any convenient test can be used here, for instance those described below, or any technique for identifying known and characterised fungi (e.g. fungi already identified as being resistant to an antifungal agent). In a further step it may be ascertained whether or not a particular resistance is acquired or intrinsic, e.g. by comparison to typical or wild type fungi of the same species.

By "growth of an fungus" it is meant both an increase in the size of a fungus or in the amount and/or volume of the constituents of a fungus (e.g. the amount of nucleic acid, the amount of protein, the number of nuclei, the numbers or size of organelles, the volume of cytoplasm) and an increase in the numbers of the fungus, i.e. an increase in the replication of the fungus.

Typically growth of a fungus is accompanied by the enlargement of the organism. The growth of fungus can be measured with routine techniques. For instance, microscopic examination of cell morphology over time, or assays to measure changes in the quantities of protein or nucleic acid (e.g. DNA) in general, or the changes in the quantities of specific proteins or nucleic acids, can be used. The skilled man would easily be able to select suitable markers to follow. Conveniently, so called housekeeping genes (e.g. α-actin, GAPDH (glyceraldehyde 3-phosphate dehydrogenase), SDHA (succinate dehydrogenase), HPRT1 (hypoxanthine phosphoribosyl transferase 1), HBS1L (HBS1-like protein), AHSP (alphahaemoglobin stabilising protein), and β2M (beta-2-microglobulin)) and 16S RNA can be monitored.

By "replication of a fungus" it is meant the act by which the fungus reproduces. Typically this is by binary fission where a cell divides into two. To support the division of the cell into two, binary fission is normally preceded by enlargement of the dividing cell and an increase in the amount and/or volume of cellular constituents. Replication results in an increase in the number of cells and so may be followed by any method of assessing cell numbers in a population. Another option is to follow the process in real time by visual examination with a microscope. The time it takes for cell to replicate (i.e. produce another version of itself) is the generation time. Generation time will depend on the conditions in which the fungus is found. The rate of replication can be expressed in terms of the generation time.

By "inhibiting the growth of a fungus " it is meant that measurable growth (e.g. replication) of a fungus, or the rate thereof, is reduced. Preferably measurable growth (e.g. replication) of a fungus, or the rate thereof, is reduced by at least 50%, more preferably at least 60%, 70%, 80% or 90%, e.g. at least 95%. Preferably, measurable growth (e.g. replication) is ceased. Growth in terms of cell size increase or expansion etc. may be inhibited independently of replication and vice versa.

The term "viability of a fungus" means the ability of a fungus to survive under given conditions. Survival can be considered equivalent to remaining alive. Determining the viability of a fungus can be done using the techniques detailed below for measuring microorganism cell death (and viability).

Thus, "inhibiting the viability" of a fungus can include any effect which reduces the viability of a fungus, or which renders it less likely to survive, or non-viable. In particular this term covers killing or destroying a fungus.

The term "killing a fungus" refers to the act of causing a fungus to cease to be alive, i.e. to become dead. A fungus is considered to be alive if it can be induced to replicate and/or grow, or at least display morphological changes, when placed in a medium that would normally support the growth of that fungus and/or the fungus is metabolising nutrients to release energy to support cellular functions. Typically, a fungus can be considered to be dead if cell membrane integrity is lost.

Many routine assays are available to determine if a fungus is alive (viable) or dead. One option is to place the fungus in conditions that would normally support the growth of that fungus and monitor the growth of the fungus by appropriate standard means, e.g. by monitoring the size of the fungus, the morphology of the fungus, the number of fungi in the colony over time, the consumption of nutrients in the culture media, etc.

Another option is to assess the fungal cells for morphologies characteristic of cell death, e.g. necrotic or apoptotic bodies, membrane blebs, nuclear condensation and cleavage of DNA into regularly sized fragments, ruptured cell walls or membranes and leakage of cell contents into the extracellular environment.

Other methods exploit the characteristic loss of cell membrane integrity in dead fungal cells. Membrane impermeable dyes (e.g. trypan blue and propidium iodide) are routinely used to assess membrane integrity. These dyes are excluded from intact fungal cells and so no staining occurs in such fungi. If cell membrane integrity is compromised, these dyes can access the fungal cells and stain intracellular components. Alternatively, or in addition, dyes that only stain fungal cells with intact membranes are used to give an indication of the viability of the cell. The Live/Dead Assay of Invitrogen Ltd is an assay that uses two dyes, one to stain dead cells, the other to stain live cells. Another approach to assessing membrane integrity is to detect the release of cellular components into the culture media, e.g. lactate dehydrogenase.

A still further option is to measure the metabolism of fungal cells. This can be done routinely in a number of ways. For instance the levels of ATP can be measured. Only living cells with intact membranes can synthesis ATP and because ATP is not stored in cells, levels of ATP drop rapidly upon cell death. Monitoring ATP levels therefore gives an indication of the status of the fungal cell. A yet further option is to measure the reducing potential of the fungal cell. Viable fungal cells metabolising nutrients use reducing reactions, by applying a marker that gives different outputs whether in reduced or oxidised form (e.g. a fluorescent dye) to the fungal cell, the fungal cell's reducing potential can be assessed. Fungal cells that lack the ability to reduce the marker can be considered to be dead. The MTT and MTS assays are convenient examples of this type of assay.

By "resistant to an antifungal agent" it is meant that the fungus displays a substantially greater tolerance (reduced susceptibility) to an antifungal agent as compared to a reference fungus sensitive to the antifungal agent or a typical, or a wild type, version of the fungus. Such a substantially greater tolerance may be a statistically significant decrease in susceptibility to the antifungal agent, as measured for example in standard assays, such as MIC assays. In some cases, a fungus can be completely unaffected by exposure to an antifungal agent. In this instance the fungus can be considered fully resistant to that antifungal agent.

A suitable reference fungus is *Saccharomyces cerevisiae* although many others are known in the art and are readily available. Typical, or wild type, versions of a fungus can be obtained easily from laboratories and culture collections throughout the world.

Susceptibility (and conversely resistance and tolerance) to an antifungal agent can be measured in any convenient way, e.g. with dilution susceptibility tests and/or disk diffusion tests. The skilled man would appreciate that the extent of the difference in tolerance/susceptibility sufficient to constitute resistance will vary depending on the antifungal agent and organism under test and the test used. However, a resistant fungus will preferably be at least twice, e.g. at least 3, 4, 5, 6, 10, 20, or 50 times as tolerant to the antifungal agent as the reference fungus sensitive to the antifungal agent or a typical or a wild type version of the fungus. Preferably resistance of a particular fungus to an antifungal agent is determined using fungi which are not in a bioflm or which do not have a biofilm phenotype.

The minimum inhibitory concentration (MIC) assay is a convenient dilution susceptibility test to use. This assay measures the relevant tolerance of a fungus to antifungal agents by determining the lowest concentration of antifungal agent that causes complete inhibition of growth. A fungus resistant to an antifungal agent will have a substantially greater MIC value for the antifungal agent than that of the reference fungus sensitive to the antifungal agent or a typical, or a wild type, version of the fungus, e.g. the resistant fungus will have a MIC value for the antifungal agent that is at least twice or at least four times, at least eight times, at least sixteen times, at least thirty two times or at least sixty four times higher. Put in a different way, the MIC value of the resistant fungus for the antifungal agent may be at least double, at least quadruple, at least octuple, at least sexdecuple or at least duotrigenuple the MIC value of the reference fungus sensitive to the antifungal agent or a typical or a wild type version of the fungus

Viewed alternatively, and in the context of an *in vivo* use (e.g. the treatment of a fungal infection) wherein the fungus is resistant to an antifungal agent, a fungus may be considered resistant to an antifungal agent if the fungus has a MIC value for the antifungal agent that is greater than the maximum safe circulating concentration of the antifungal agent in the subject (which may be determined easily by the skilled man). More functionally, a fungus may be considered to be resistant to an antifungal agent if an infection associated with that fungus is unresponsive (i.e. there is no change in the clinical indicia of the infection) to the maximum safe dose of the antifungal agent.

The fungus targeted by the methods or uses of the invention may be resistant to more than one antifungal agent, or more particularily it may be resitant to more than one class of antifungal agent, e.g. the fungus may be resistant to at least 2 or 3, or at least 4, 5, 6, 7, 8, 9 or 10 antifungal agents or classes thereof. Those fungi that are resistant to more than 3 classes of antifungal agent are "multidrug resistant (MDR)" or "have an MDR phenotype" .

"Overcoming resistance" should be construed accordingly as a measurable reduction in the above-described indicators of the resistance (or measurable increase in susceptibility or measurable decrease in tolerance) to the antifungal agent displayed by the fungus. Therefore "overcoming resistance" can alternatively be expressed as "reducing resistance". It is a reference to the observed phenotype of the target fungus and should not necessarily be considered to equate to a reversal, to any extent, at the mechanistic level of any particular resistance mechanism. The effects of alginate oligomers in overcoming resistance to an antifungal agent(s) or in potentiating (etc.) the effects of an antifungal agent(s) may be seen irrespective of the mechanism of resistance to the antifungal agent in question.

In one embodiment the alginate oligomer will measurably reduce the MIC value of the resistant fungus for the antifungal agent, e.g. the MIC value will be at least 50%, 25%, 20%, 15%, 10%, 5%, 2% or 1% of the MIC value of the fungus for the antifungal agent before treatment in accordance with the invention. In other examples the alginate oligomer overcomes resistance to at least two, e.g. at least 3, 4, 5, 6, 7, 8, 9, 10 or all of the structurally and/or functionally different antifungal agents or classes of antifungal agents to which the fungus is resistant. However, it is not required, or implied, that all of the resistance of any given resistant strain is overcome. The alginate oligomer may for example be effective in overcoming resistance to certain antifungal agents or classes thereof in a given resistant strain (e.g. to polyenes and/or allylamines and/or azoles and/or echinocandins) and this may be useful, even though resistance to other antifungal agents may remain. This example will preferably entail the use of a plurality of antifungal agents corresponding in number and identity to some or all of the antifungal resistances overcome.

In other examples the method of the invention overcomes resistance in a fungus to at least one antifungal agent that is a conventional treatment of that fungus. Put differently, the alginate oligomer may overcome resistance in a fungus to an antifungal agent to which that fungus has acquired or developed resistance. In these examples the alginate oligomer overcomes at least one acquired resistance in a fungus that has acquired resistance to at least one, e.g. at least 2, 3, 4, 5, 6, 7, 8, 9 or 10 structurally and/or functionally different antifungal agents or classes thereof. Preferably all of the acquired antifungal agent resistance of the fungus is overcome. It will be clear to the skilled reader that the present disclosure therefore makes possible the treatment of a fungus with an antifungal agent that had become ineffective in the treatment of that fungus. However, as noted above, not all resistance in an resistant phenotype may be acquired and the disclosure is not limited to this. Thus the present disclosure may be used in the treatment of fungi that are innately resistant to an antifungal agent.

The location of the fungus which may targeted in any aspect of the present invention is not restricted, and thus as indicated above, not only are therapeutic uses covered, but also non-therapeutic or non-clinical uses where the fungus or the site or location at risk of infection or contamination is not present on or within the body of a human or non-human animal (e.g. a clinical subject/patient), but may for example be present at an abiotic (e.g. inanimate) site or location or a non-clinical biotic site or location (e.g. on or in a plant, or a part thereof) i.e. the invention may be carried out *in vitro* (which term is considered to include use in relation to *ex vivo* biotic materials). Thus, the methods of the invention as set out above are in certain embodiments not practiced or carried out in or on the human or animal body (e.g. wherein the step of using the alginate oligomer and/or antifungal agent does not occur in or on the human or animal body). In the context of this invention any method that is not a method practiced on or in the body of a human or an animal (e.g. by therapy or surgery) may be viewed as an *in vitro* method. Conversly, *in vivo* methods are methods carried out on or in the human or non-human animal body, which include the treatment of inanimate materials contained in (e.g. implanted in) the body of a human or non-human animal. For the avoidance of doubt, methods of the invention invloving the treatment of plants, parts thereof (including seeds, fruits and flowers), plant products and other biotic surfaces/materials that are not on or in a living human or animal body at the point of treatment are *in vitro* methods.

The fungus may be present on a surface. The surface is not limited and includes any surface on which a fungus may occur. The surface may be biotic or abiotic, and inanimate (or abiotic) surfaces include any such surface which may be exposed to fungal contact or colonisation (e.g. contamination). Thus particularly included are surfaces on medical equipment, or machinery, e.g. industrial machinery, or any surface exposed to an aquatic environment (e.g. marine equipment, or ships or boats or their parts or components), or any surface exposed to any part of the environment, e.g. pipes or on buildings. Such inanimate surfaces exposed to microbial contact or colonisation include in particular any part of: food or drink processing, preparation, storage or dispensing machinery or equipment, air conditioning apparatus, industrial machinery, e.g. in chemical or biotechnological processing plants, storage tanks, medical or surgical equipment and cell and tissue culture equipment. Any apparatus or equipment for carrying or transporting or delivering materials is susceptible to fungal contamination. Such surfaces will include particularly pipes (which term is used broadly herein to include any conduit or line). Representative inanimate or abiotic surfaces include, but are not limited to food processing, storage, dispensing or preparation equipment or surfaces, tanks, conveyors, floors, drains, coolers, freezers, equipment surfaces, walls, valves, belts, pipes, air conditioning conduits, cooling apparatus, food or drink dispensing lines, heat exchangers, boat hulls or any part of a boat's structure that is exposed to water, dental waterlines, oil drilling conduits, contact lenses and storage cases.

As noted above, medical or surgical equipment or devices represent a particular class of surface on which fungal colonisation may occur. This may include any kind of line, including catheters (e.g. central venous and urinary catheters), prosthetic devices (e.g. heart valves, artificial joints, false teeth, dental crowns, dental caps and soft tissue implants (e.g. breast, buttock and lip implants)). Any kind of implantable (or "in-dwelling") medical device is included (e.g. stents, intrauterine devices, pacemakers, intubation tubes (e.g. endotracheal or tracheostomy tubes), prostheses or prosthetic devices, lines or catheters). An "in-dwelling" medical device may include a device in which any part of it is contained within the body, i.e. the device may be wholly or partly in-dwelling.

The surface can be made of any material. For example it may be metal, e.g. aluminium, steel, stainless steel, chrome, titanium, iron, alloys thereof, and the like. The surface can also be plastic, for example, polyolefin (e.g. polyethylene, (UltraHigh Molecular Weight) polyethylene, polypropylene, polystyrene, poly(meth)acrylate, acrylonitrile, butadiene, ABS, acrylonitrile butadiene, etc.), polyester (e.g. polyethylene terephthalate, etc.), and polyamide (e.g. nylon), combinations thereof, and the like. Other examples include acetal copolymer, polyphenylsulfone, polysulfone, polythermide, polycarbonate, polyetheretherketone, polyvinylidene fluoride, poly(methyl methacrylate) and poly(tetrafluoroethylene). The surface can also be brick, tile, ceramic, porcelain, wood, vinyl, linoleum, or carpet, combinations thereof, and the like. The surfaces can also be food, for example, beef, poultry, pork, vegetables, fruits, fish, shellfish, combinations thereof, and the like. The "treatment" of any such surface (i.e. the application to any such surface of an alginate oligomer together with an antifungal agent) to combat infection or colonisation by a fungus is encompassed by the present invention.

In an infection by a fungus, which may be treated according to the present invention, the fungus may occur in or on a surface in a human or non-human animal subject or plant. Furthermore, outside the context of medical treatment, fungi may also occur on biotic surfaces. Thus the invention includes the treatment of biotic surfaces. A biotic or animate surface may include any surface or interface in or on an animal or plant body or parts thereof, for instance limbs, organs, seeds, flowers, fruits, roots, bark and leaves. It may accordingly be viewed as a "physiological" or "biological" surface. It may be any internal or external body surface, including of any tissue or organ, which, in the case of an animal body, may include haematological or haematopoietic tissue (e.g. blood). Dead or dying (e.g. necrotic) or damaged (e.g. inflamed or disrupted or broken) tissue is particularly susceptible to fungal colonisation, and such tissue is encompassed by the term "animate" or "biotic". The surface may be a mucosal or non-mucosal surface. Animal and plant products, e.g. pelts, leather, hide, wool, wood, cotton, linen, jute, silk, bamboo, cork and so on may also be considered biotic. The soil, especially cultivated soil, may also be considered a biotic material on account of its content of organic material.

In the context of the human or non-human animal body, representative biotic surfaces include, but are not limited to, any surface in the oral cavity (e.g. teeth, gingiva, gingival crevice, periodontal pocket) the reproductive tract (e.g. cervix, uterus, fallopian tubes), the peritoneum, middle ear, prostate, urinary tract, vascular intima, the eye (i.e. ocular tissue, e.g. the conjunctiva, corneal tissue, lachrymal duct, lachrymal gland, eyelid) the respiratory tract, lung tissue (e.g. bronchial and alveolial), heart valves, gastrointestinal tract, skin, scalp, nails and the interior of wounds, particularly chronic wounds and surgical wounds, which may be topical or internal wounds. Other surfaces include the exterior of organs, particularly those undergoing transplantation, for example, heart, lungs, kidney, liver, heart valve, pancreas, intestine, corneal tissue, arterial and venous grafts and skin.

In the context of the plant body, which may be, for example, a bryophte (e.g. a moss, a liverwort, a hornwort), a fern, a gymnosperm or an angiosperm (e.g. a monocot or a dicot), representative biotic surfaces include, but are not limited to, any surface of the roots, rhizomes, fronds, stems, branches, leaves, needles, spines, seeds, seed pods, seed shells, bulbs, cones, fruits, berries, drupes, follicles, legumes, capsules, kernels, sporangiums, buds, husks, flowers, petals, carpels, stamens, stigmas, styles, anthers, filaments, bark and tendrils. Preferably the plant will be a crop plant, especially those of which are or provide foodstuffs for animals, e.g. humans, more especially cereals (e.g. oats, barley, maize, rice, wheat, sorghum, millet, triticale, fonio, buckwheat, quinoa), sugar cane, oil seed plants (e.g. rape, soybean, palm, sunflower, peanut, cotton, coconut, olive, castor), apple, pear, plum, peach, nectarine, strawberry, raspberry, blackcurrant, redcurrant, whitecurrant, gooseberry, blueberry, cranberry, greengage, kiwi, mango, passion fruit, melon, tomato, potato, carrot, banana, cacao, lime, lemon, orange, grapefruit, mandarin, tangerine, satsuma, clementine, pineapple, tea, coffee, grape, almond, walnut, cashew, hazelnut, lentil, pea, bean, cabbage, onion, lettuce, pepper, cucumber, asparagus, broccoli, cauliflower and sweet potato. In other embodiments the plant will provide non-edible materials. For instance the plant will be a source of timber or wood (e.g. oak, pine, walnut, beech, birch, spruce, fir, cork, balsa), cotton, linen, latex rubber or bamboo.

The location may also be a location that is not a surface. In other words the fungus can be found within an material as well as on its surface. The material can be chemically heterogeneous as well as chemically homogenous. The material can also be constructed or formed from or comprise different parts or components. The material can be a part of a larger material or entity. The material may be or comprise the materials from which the above mentioned surfaces are formed. In some instances the material can be considered to be an object, which terms covers volumes of liquids wherever found. The material may comprise any of the above described surfaces. The material may be abiotic or biotic (inanimate or animate) as is discussed above in relation to surfaces. For instance, the material might be, completely or in part, a solid, a liquid, a semi solid, a gel or a gel-sol.

Thus, for example, the fungus might be present in the body fluids of an animal (e.g. blood, plasma, serum, cerebrospinal fluid, GI tract contents, semen, sputum and other pulmonary secretions); animal tissues (e.g. adrenal, hepatic, renal, pancreatic, pituitary, thyroid, immune, ovarian, testicular, prostate, endometrial, ocular, mammary, adipose, epithelial, endothelial, neural, muscle, pulmonary, epidermis, osseous); plant parts (e.g. the roots, rhizomes, fronds, stems, branches, leaves, needles, spines, seeds, seed pods, seed shells, bulbs, cones, fruits, berries, drupes, follicles, legumes, capsules, kernels, sporangiums, buds, husks, flowers, petals, carpels, stamens, stigmas, styles, anthers, filaments, bark, tendrils, xylem and phloem), plant body fluids (xylem sap and phloem sap); cell and tissue culture media; plant culture media; cell and tissue cultures; plant cultures; clinical/scientific waste materials (which can comprise any of the preceding materials); soil; composts; pharmaceuticals (e.g. tablets, pills, powders, lozenges, sachets, cachets, elixirs, suspensions, emulsions, solutions, syrups, aerosols, sprays, compositions for use in nebulisers, ointments, soft and hard gelatine capsules, suppositories, sterile injectable solutions, sterile packaged powders); animal or human food stuffs (e.g. meat, fish, shellfish, fruits, vegetables, cereals, diary products, fruit juices, vegetable juices, sauces, stocks, soups, confectionary, alcoholic beverages, condiments); personal hygiene products (e.g. toothpaste, mouthwash, shampoo, soap, deodorant, shower gel); cosmetics (e.g. lip gloss, eye shadow, foundation); drinking water supplies; waste water supplies; agricultural feedstuffs and water supplies; insecticide, pesticide and herbicide formulations; industrial lubricants; engineering materials (e.g. wood, timber, lumber, paper, concrete, cement, sand, porcelain, stone, ceramics, plaster, paint, varnish, silicon sealants, grout, mortar, bricks, plastics) and so on. Liquids, semi solids, gels or gel-sols are of note. The animal body fluids and tissues may be treated *in vitrolex vivo* as well as it being possible to treat the same *in vivo.*

In accordance with the invention the fungus will not be in a biofilm (e.g. will be growing planktonically or in a mycelium or a hypha). Put differently, the fungus will not be in a biofilm mode of growth; or will be in a non-biofilm mode of growth. Thus, in certain embodiments, the methods of the invention may further comprise a step in which the fungi being targeted will be determined as being, or alternatively not being in, or involving, a biofilm.

By "biofilm" it is meant a community of microorganisms characterized by a predominance of sessile cells that are attached to a substratum or interface or to each other (some motile cells may also be present) and that are embedded in a matrix of extracellular polymers (more specifically extracellular polymers that they have produced) characterised in that the microorganisms of this colony exhibit an altered phenotype with respect to growth rate and gene transcription (for example as compared to their "non-biofilm" or free-floating, planktonic, mycelial, or hyphal counterparts). By "in a biofilm" it is meant that the fungus is within (completely or in part), on or associated with the polymer matrix of a biofilm. Viewed differently, fungi that are "not in a biofilm" are organisms that are either in isolation, e.g. planktonic, or if in an aggregation of a plurality of organisms, that aggregation is unorganised and/or is devoid of the matrix characteristic of a biofilm, e.g. a mycelium or a hypha. In each case, the individual fungi do not exhibit an altered phenotype that is observed in their biofilm dwelling counterparts.

A biofilm in which a fungus may be found may contain a homogenous population of fungi (i.e. contain a single type of fungus) or may contain a heterogeneous population (i.e. contain a plurality of types of fungus and/or other microorganisms). Examples of possible fungal species that may be present are recited above, but a biofilm may also comprise any other microbial organism, that is any organism that is microscopic, namely too small to be seen by the naked eye. In particular as used herein the term includes viruses, as well as the organisms more typically thought of as microorganisms, particularly bacteria, fungi, archaea, algae and protists. The term thus particularly includes organisms that are typically unicellular, but which may have the capability of organising into simple cooperative colonies or structures such as filaments, hyphae or mycelia (but not true tissues) under certain conditions. The microorganism may be prokaryotic or eukaryotic, and may be from any class, genus or species of microorganism. Examples of prokaryotic microorganisms include, but are not limited to, bacteria, including the mycoplasmas, (e.g. Gram-positive, Gram-negative bacteria or Gram test non-responsive bacteria) and archaeobacteria. Eukaryotic microorganisms include fungi, algae and others that are, or have been, classified in the taxonomic kingdom Protista or regarded as protists, and include, but are not limited to, for example, protozoa, diatoms, protoophyta, and fungus-like moulds. The microorganism may be aerobic or anaerobic. The microorganism may be pathogenic or non-pathogenic, or a be spoilage or an indicator microorganism. In particular preferred embodiments the microorganism is pathogenic.

In the medical (e.g.therapeutic) embodiments of the invention the subject may be any human or non-human animal subject, but more particularly may be a vertebrate, e.g. an animal selected from mammals, birds, amphibians, fish and reptiles. The animal may be a livestock or a domestic animal or an animal of commercial value, including laboratory animals or an animal in a zoo or game park. Representative animals therefore include dogs, cats, rabbits, mice, guinea pigs, hamsters, horses, pigs, sheep, goats, cows, chickens, turkeys, guinea fowl, ducks, geese, parrots, budgerigars, pigeons, salmon, trout, cod, haddock, sea bass and carp. Veterinary uses of the invention are thus covered. The subject may be viewed as a patient. Preferably the subject is a human.

The term "in a subject" is used broadly herein to include sites or locations inside a subject or on a subject, e.g. an external body surface, and may include in particular infection of a medical device e.g. an implanted or "in-dwelling" medical device. The term "in a patient" should be interpreted consistently with this.

The location of the subject's infection is not restricted and may be any of the sites or locations in a subject described above. Administering the alginate oligomer and the antifungal agent to the subject preferably results in the infected location being contacted with an alginate oligomer and antifungal agent in amounts sufficient to treat the infection.

The subject's infection may be acute, or alternatively chronic, e.g. an infection that has persisted for at least 5 or at least 10 days, particularly at least 20 days, more particularly at least 30 days, most particularly at least 40 days.

In this aspect of the invention the infection may occur on a surface in or on the subject (i.e. a biotic surface as discussed above) and/or a surface of a medical device, particularly an implantable or "in-dwelling" medical device, representative examples of which are discussed above.

In one embodiment the methods or uses of the invention may comprise a step in which the subject is identified (e.g. diagnosed) as having or suspected to have a fungal infection or being a candidate that is at risk of or susceptible to an fungal infection.

In particular embodiments the invention may provide for the treatment of mycoses, e.g. fungal sinusitis (which incorporates allergic fungal rhinosinusitis and saprophytic sinusitis); otomycosis (fungal ear infection); fungal keratitis (fungal eye infection); onychomycosis (fungal nail infection); fungal meningitis; systemic fungal infections; invasive fungal infections; disseminated fungal infections; opportunistic fungal infections; aspergillosis (e.g. aspergilloma, chronic pulmonary aspergillosis, allergic bronchopulmonary aspergillosis); candidiasis (e.g. oral candidiasis (oral thrush), candida esophagitis, perlèche (angular cheilitis), candidal vulvovaginitis (vaginal yeast infection/thrush), candidal intertrigo, diaper candidiasis, congenital cutaneous candidiasis, perianal candidiasis, candidal paronychia, erosio interdigitalis blastomycetica, chronic mucocutaneous candidiasis, systemic candidiasis, candidid, antibiotic candidiasis (iatrogenic candidiasis); coccidioidomycosis (e.g. acute pulmonary coccidioidomycosis, chronic pulmonary coccidioidomycosis, disseminated coccidioidomycosis, coccidioidal meningitis); cryptococcosis (e.g. acute pulmonary cryptococcomycosis, chronic pulmonary cryptococcomycosis, disseminated cryptococcomycosis, cryptococcal meningitis); histoplasmosis (e.g. asymptomatic primary histoplasmosis, acute symptomatic pulmonary histoplasmosis, chronic pulmonary histoplasmosis, disseminated histoplasmosis); blastomycosis (e.g. acute pulmonary blastomycosis, chronic pulmonary blastomycosis, disseminated blastomycosis); mycetoma; paracoccidioidomycosis; pneumocystosis; fusariosis; phaeohyphomycosis (e.g. subcutaneous phaeohyphomycosis; paranasal sinus phaeohyphomycosis, cerebral phaeohyphomycosis); alternariosis (e.g. subcutaneous alternariosis, cutaneous alternariosis); rhinosporidiosis; microsporidiosis; basidiobolomycosis (e.g. subcutaneous basidiobolomycosis, gastrointestinal basidiobolomycosis, disseminated basidiobolomycosis); conidiobolomycosis (e.g. subcutaneous conidiobolomycosis, disseminated conidiobolomycosis); mucormycosis (e.g. rhinocerebral mucormycosis, GI mucormycosis, pulmonary mucormycosis); trichosporonosis (e.g. cutaneous trichosporonosis, GI trichosporonosis, disseminated trichosporonosis); chromoblastomycosis; geotrichosis (e.g. oral geotrichosis, pulmonary geotrichosis, GI geotrichosis); allescheriasis (e.g. pulmonary allescheriasis, disseminated allescheriasis); sporotrichosis (e.g. pulmonary sporotrichosis, fixed cutaneous sporotrichosis, lymphocutaneous sporotrichosis, osteoarticular sporotrichosis, disseminated sporotrichosis); penicilliosis; lobomycosis; dermatophytosis/ringworm (e.g. tinea pedis (athlete's foot), tinea unguium, tinea corporis, tinea cruris (jock itch), tinea manuum, tinea capitis, tinea barbae, tinea faciei); piedra (e.g. tinea versicolor, tinea nigra, tinea albegena) or pityrosporum folliculitis / malassezia folliculitis.

The fungal infection may be a superficial mycosis (i.e. infections limited to the outer surface of the hair and skin), a cutaneous mycosis, a subcutaneous mycosis or a systemic, invasive or disseminated (which terms are used interchangeably) mycosis. The infection may be considered an opportunistic infection, by which it is meant an infection by a fungus species that is considered usually benign relative to a healthy subject, i.e. one with a healthy (uncompromised) immune system.

Fungi play a role in the acquisition, development and complication of respiratory diseases (e.g. cystic fibrosis, pneumonia, COPD, COAD, COAP), septicaemia, septic shock, sepsis, meningitis, or poisoning/allergies caused by fungally derived toxins. Accordingly, in particular embodiments the invention may provide for the treatment of respiratory diseases, e.g. cystic fibrosis, (fungal) pneumonia, COPD, COAD, COAP, (fungal) septicaemia, (fungal) septic shock, (fungal) sepsis, (fungal) meningitis, or poisoning/allergies caused by fungally derived toxins.

A fungal infection can occur in any subject but some subjects will be more susceptible to infection than others. Subjects who are susceptible to fungal infection include, but are not limited to, subjects whose epithelial and/or endothelial barrier is weakened or compromised, subjects whose secretion-based defences to microbial infection have been abrogated, disrupted, weakened or undermined, and subjects who are immunocompromised, immunodeficient or immunosuppressed (i.e. a subject in whom any part of the immune system is not working normally, or is working sub-normally, in other words in whom any part of the immune response, or an immune activity is reduced or impaired, whether due to disease or clinical intervention or other treatment, or in any way). These subjects are susceptible to opportunistic fungal infections.

Representative examples of subjects who are susceptible to fungal infection include, but are not limited to, subjects with a pre-established infection (e.g. with bacteria, viruses, fungi or parasites such as protozoa), especially subjects with HIV, bacteraemia, sepsis or septic shock; subjects with immunodeficiency, e.g. subjects preparing for, undergoing or recovering from cancer chemotherapy and/or radiotherapy, organ (e.g. bone marrow, liver, lung, heart, heart valve, kidney, etc.) transplant subjects (including autograft, allograft and xenograft patients) and subjects with AIDS; subjects undergoing or recovering from antibiotic therapy; subjects undergoing or recovering from steroid therapy; subjects resident in a healthcare institution, e.g. hospital, especially subjects in intensive care or critical care (i.e. those units concerned with the provision of life support or organ support systems to patients); subjects on respiratory ventilators; subjects suffering from trauma; subjects with burns; subjects with acute and/or chronic wounds; neonatal subjects; elderly subjects; subjects with cancer (defined broadly herein to include any neoplastic condition; malignant or non-malignant), especially those with cancers of the immune system (e.g. leukaemias, lymphomas and other haematological cancers); subjects with diabetes; subjects with malnutrition; subjects with alcoholism; subjects suffering from auto-immune conditions such as rheumatoid arthritis, diabetes mellitus type I, Crohn's disease, especially those undergoing immunosuppression treatment for those diseases; subjects with reduced or abrogated epithelial or endothelial secretion (e.g. mucous, tears, saliva) and/or secretion clearance (e.g. subjects with poorly functioning cilia on mucosal tissue and/or patients with hyperviscous mucous (e.g. smokers and subjects with COPD, COAD, COAP, bronchitis, cystic fibrosis, emphysema, lung cancer, asthma, pneumonia or sinusitis)) and subjects fitted with a medical device.

Thus, subjects in whom fungal infections may particularly be combated according to the present invention include patients who are impaired, whether due to poor perfusion, repetitive trauma, poor nutrition, poor oxygenation or white cell dysfunction.

Fungal infections are commonly encountered in healthcare institutions due in part to the close proximity of subjects with fungal infections and those that have compromised defences against microorganisms, but also because of the widespread use of antibiotics. Fungi, e.g. from the genera Candida, Aspergillus, Malassezia, Trichosporon, Fusarium, Acremonium, Paecilomyces, Rhizopus, Mucor, Scedosporium and Absidia, are often involved in nosocomial infections and accordingly the invention can be seen as providing treatments for fungal nosocomial infections, e.g. nosocomial infections involving *Candida albicans, Candida glabrata, Candida tropicalis, Candida lusitaniae, Candida dubliniensis, Candida parapsilossis, Candida krusei, Candida rugosa, Aspergillus fumigatus, Aspergillus flavus, Aspergillus clavatus, Aspergillus terrus, Malassezia pachydermatis, Malassezia furfur, Trichosporon cutaneum,* the *Fusarium solani complex: Fusarium oxysporum, Fusarium verticillioides, Fusarium proliferatum, Fusarium monilifrome, Acremonium kiliense, Acremonium strictum, Paecilomyces lilacinus, Rhizopus oryzae, Mucor indicus, Scedosporium prolifican* or *Absidia corymbifera.* Very often, but not always, these infections are invasive (systemic or disseminated) infections of the subjects.

Particularly susceptible to fungal infections, whether resident in a healthcare institution or not, are subjects that have undergone physical trauma. The trauma itself might cause a weakening in or compromisation of an epithelial and/or endothelial barrier of the subject or the subject may become immunocompromised in response to the trauma (a shock response). The term "trauma" refers broadly to cellular attack by foreign bodies and/or physical injury of cells. Included among foreign bodies are microorganisms, particulate matter, chemical agents, and the like. Included among physical injuries are mechanical injuries; thermal injuries, such as those resulting from excessive heat or cold; electrical injuries, such as those caused by contact with sources of electrical potential; and radiation damage caused, for example, by prolonged, extensive exposure to infrared, ultraviolet or ionizing radiations.

Of particular note are subjects that have a blast injury, which may be considered a trauma resulting directly or indirectly from exposure to an explosion.

Also of particular note are subjects that have a burn. Any burn, in particular a severe burn, has a significant impact on the integrity of the epithelial and/or endothelial barrier of the subject and the subject will often become immunocompromised in response to the burn (a shock response).

Typical burn-causing agents are extremes of temperature (e.g. fire and liquids and gases at extreme temperature), electricity, corrosive chemicals, friction and radiation. The extent and duration of exposure, together with the intensity/strength of the agent, result in burns of varying severity. Scalding (i.e. trauma associated with high temperature liquids and/or gases) is considered to be a burn.

Epidermal burn severity is commonly classified in two ways. Most common is the classification by degree. First-degree burns are usually limited to erythema (redness) in the general area of the injury and a white plaque at the site of injury. The cellular trauma of these burns extends only as deep as the epidermis. Second-degree burns also display erythema in the general area of the injury but with superficial blistering of the epidermis. The cellular trauma of second-degree burns involves the superficial (papillary) dermis and may also involve the deep (reticular) dermis layer. Third-degree burns are those in which the epidermis is lost with damage to the hypodermis. Damage is typically extreme including charring. Sometimes eschar, (dry, black necrotic tissue) will be present. Third-degree burns may require grafting. In fourth-degree burns catastrophic damage of the hypodermis occurs, e.g. the hypodermis is completed lost, with damage extending to the underlying muscle, tendon, and ligament tissue. Charring and eschar are observed. Grafting is required if the burn does not prove to be fatal.

Another common classification system is the classification by thickness. "Superficial thickness" burns correspond to first degree burns. The spectrum of second degree burns is covered by two classes of "partial thickness" burns. "Partial thickness-superficial" are burns that affect the epidermis only as far as the papillary dermis. "Partial thickness-deep" are burns that affect the dermis as far as the reticular dermis. "Full thickness" burns correspond to third and fourth degree burns.

Some physical injuries, e.g. some burns, and cellular attacks by foreign bodies result in the formation of a wound. More specifically a wound may be considered to be a breach in, or denudement of, a tissue. Wounds may also be caused by a spontaneously forming lesion such as a skin ulcer (e.g. a venous, diabetic or pressure ulcer), an anal fissure or a mouth ulcer.

Wounds are typically defined as either acute or chronic. Acute wounds are wounds that proceed orderly through the three recognised stages of the healing process (i.e. the inflammatory stage, the proliferative stage and the remodelling phase) without a protracted timecourse. Chronic wounds, however, are those wounds that do not complete the ordered sequence of biochemical events of the healing process because the wound has stalled in one of the healing stages. Commonly, chronic wounds are stalled in the inflammatory phase. In accordance with a particular aspect of the present invention, a chronic wound is a wound that has not healed within at least 40 days, particularly at least 50 days, more particularly at least 60 days, most particularly at least 70 days.

As discussed above, wounds are an ideal environment for an fungal infection, particularly chronic infection, due to their lack of an epithelial barrier and the availability of substrate and surface for microbial attachment and colonisation. Problematically, infection of a wound often delays healing further and thus renders that wound more susceptible to established infection. The invention is therefore effective in the treatment and prevention of fungal infection of wounds and the treatment of wounds, especially chronic wounds, represents one preferred aspect of the present invention.

Therefore, also described herein is an alginate oligomer for use together with (or in combination or conjunction with) an antifungal agent in the treatment or prevention of an infection of a subject by a fungus, particularly chronic infection by a fungus, in the above-mentioned subjects, in particular in subjects with respiratory diseases or disorders (e.g. cystic fibrosis, COPD, COAD, COAP, pneumonia), wounds, burns and/or traumas.

Through the ability to treat and prevent infection of wounds by a fungus the alginate oligomers and antifungal agents of use in the invention as defined herein can remove one of the obstacles to wound healing and therefore the alginate oligomers and antifungal agents defined above are also effective in the promotion of healing of acute and chronic wounds infected with or at risk of infection with a fungus.

By promotion of healing it is meant that the treatment accelerates the healing process of the wound in question (i.e. the progression of the wound through the three recognised stages of the healing process). The acceleration of the healing process may manifest as an increase in the rate of progression through one, two or all of the healing stages (i.e. the inflammatory stage, the proliferative stage and/or the remodelling phase). If the wound is a chronic wound that is stalled in one of the healing stages the acceleration might manifest as the restarting of the linear, sequential healing process after the stall. In other words, the treatment shifts the wound from a non-healing state to a state where the wound begins to progress through the healing stages. That progression after the restart may be at a normal rate or even a slower rate compared with the rate a normal acute wound would heal.

The alginate oligomers and antifungal agents of use in the invention may be used together (or in combination or conjunction) to treat or prevent fungal infections wherever they may occur in or on the body of a subject. Thus the infection may be an infection of a medical device by a fungus, particularly an in-dwelling medical device, e.g. endotracheal and tracheostomy tubes.

The alginate oligomers and antifungal agents of use in the invention may also be used together (or in combination or conjunction) as oral healthcare agents, for example in the control of oral thrush.

Conveniently, the alginate oligomers and/or antifungal agents can be applied by any oral health/oral hygiene delivery system. This may be through the use of toothpastes, dental gels, dental foams and mouthwashes. Removable dentures and other removable dental prostheses may be treated outside of the oral cavity with the same compositions or other suitable pharmaceutically acceptable compositions. The alginate oligomers and/or antifungal agents can also be incorporated into compositions that are applied to the oral cavity (or applied to removable dentures and other removable dental prostheses outside of the oral cavity) to form a coating that persists on surfaces over time, or that releases the alginate oligomers and/or antifungal agents from the coated surfaces over time, and which inhibit the growth of fungi in the oral cavity and on the surfaces of removable dentures and other removable dental prostheses.

The alginate oligomers and antifungal agents of use in the invention may also be used together (or in combination or conjunction) as skincare and/or haircare agents, for example in the control of superficial, cutaneous or subcutaneous mycoses (e.g. tinea pedis, tinea unguium, tinea corporis, tinea cruris, tinea manuum, tinea capitis, tinea barbae, tinea faciei, tinea versicolor, tinea nigra, tinea albegena, pityrosporum folliculitis / malassezia folliculitis, perlèche, candidal intertrigo, diaper candidiasis, congenital cutaneous candidiasis, perianal candidiasis, candidal paronychia, erosio interdigitalis blastomycetica).

Conveniently, the alginate oligomers and/or antifungal agents can be applied by any skincare and/or haircare delivery system. This may be through the use of shampoos, soaps, shower gels, hair conditioners, skin creams, emollients, ointments, lotions, oils, hair gels, hair sprays, foams and waxes.

In specific embodiments of the invention the alginate oligomers and antifungal agents of use in the invention may be used together (or in combination or conjunction) in the treatment or prevention of pneumonia (in particular ventilator associated pneumonia) associated with fungi; respiratory diseases associated with fungi (which may include COPD, COAD, COAP, pneumonia, cystic fibrosis and asthma); and device related fungal infections associated with implantable or prosthetic medical devices (e.g. prosthetic valve endocarditis or the infection of lines or catheters or artificial joints or tissue replacements (including e.g dental implants) or endotracheal or tracheotomy tubes).

As mentioned previously, in one aspect the invention provides an in vitro method for combating colonisation of a site with fungi which is not in a biofilm.

"Combating colonisation" includes both preventative and reactionary measures or treatments and therefore covers the prevention as well as the reduction, limitation, or elimination of, existing colonisation, e.g. includes a delay in colonisation.

The term "combat a fungus" includes both preventative and reactionary measures or treatments and therefore includes killing or otherwise preventing or reducing the growth of a fungus. In particular, the formation of a population of fungi may be prevented or the growth of the population may be controlled. This may result in the reduction, limitation, or elimination of the population, or a delay in its formation.

The site or location of the fungus or fungal colonisation (or potential fungal colonisation or location etc.) is not restricted and can be any of the various sites or locations described or mentioned above, e.g. it can be *in vitro* or *in vivo,* but particularly in this aspect of the invention it will be an *"in vitro"* or *"ex vivo"* site or location (e.g. an inanimate or abiotic site or location, or a non-clinical biotic location). However, the site or location may be in or on a subject and in which case the alginate oligomer and the antifungal agent are typically administered to the subject in physiologically and/or pharmaceutically acceptable forms.

In one particular embodiment the various aspects of the invention can be applied to the decontamination of clinical, scientific and industrial waste materials. In another particular embodiment the various aspects of the invention can be used to decontaminate transplant tissue (e.g. heart, lungs, kidney, liver, heart valve, pancreas, intestine, corneal tissue, arterial and venous grafts and skin) and medical devices (e.g. endotracheal and tracheostomy tubes) prior to implantation.

In other embodiments the various aspects of the invention can be applied to the control of fungal populations in the environment, in particular in the technical fields of agriculture, food production and engineering where fungal colonisation and subsequent spoilage/damage can be costly and even dangerous to human health.

Of particular note is the application of the invention to the combat of fungi during the cultivation of plants and the production of plant products. In this area the methods of the invention may be used to control fungi in or on the living plants themselves to maximise their productivity, but they may also be applied to the products of harvest (e.g. seeds, fruits, flowers, leaves) or the products produced therefrom to combat spoilage. The soil may also be treated to combat existing and future colonisation.

The term "in a plant" is used broadly herein to include sites or locations inside a plant or on a plant, e.g. an external plant surface or an internal plant tissue.

The location of the plant's infection (or colonisation) is not restricted and may be any of the sites or locations in a plant described above. Administering the alginate oligomer and the antifungal agent to the plant preferably results in the infected (or colonised) location being contacted with an alginate oligomer and antifungal agent in amounts sufficient to treat the infection (or colonisation).

The plant's infection (or colonisation) may be acute, or alternatively chronic, e.g. an infection that has persisted for at least 5 or at least 10 days, particularly at least 20 days, more particularly at least 30 days, most particularly at least 40 days.

In one embodiment the methods or uses of the invention may comprise a step in which the plant is identified as having or suspected to have a fungal infection (or colonisation) or being a candidate that is at risk of, or susceptible to, an fungal infection (or colonisation).

Food spoilage is a serious problem and the methods of the invention can be applied to the food production industry at many points, e.g. by applying the alginate oligomers and the antifungal agents together to foodstuffs and/or using these compounds together as ingredients in the foodstuffs and/or in their packaging.

In a still further embodiment the various aspects of the invention can be considered to cover the use of the alginate oligomers together with the antifungal agents as anti-fungal preservative treatments/additives for materials, especially solutions and liquids. This has a particular application in the field of engineering where structural and/or cosmetic damage to materials can occur if fungal colonisation is allowed to establish. Thus, the incorporation of the alginate oligomers together with the antifungal agents into engineering materials or the application of these compounds to engineering materials is useful to prevent this damage. Engineering materials (e.g. wood, timber, lumber, paper, concrete, cement, sand, porcelain, stone, ceramics, plaster, paint, varnish, silicon sealants, grout, mortar, bricks, plastics) may therefore be supplied after being treated with the alginate oligomers together with the antifungal agents or with these compounds being included as additives. In addition, the alginate oligomers together with the antifungal agents may be applied or added to these materials at any time, e.g. to prevent fungal colonisation or to treat fugal colonisation if it has occurred.

As noted above, the term "contacting" encompasses any means of delivering the alginate oligomer and the antifungal agent to the fungus, whether directly or indirectly.

More particularly the fungus will be contacted with an effective amount of the alginate oligomer and the antifungal agent, more particularly an amount of the alginate oligomer and an amount of the antifungal agent that together (or in combination or conjunction) inhibit the viability and/or growth of the fungus, and therefore treat or prevent the infection/colonisation. In other embodiments those amounts are sufficient to overcome the resistance of the fungus to the antifungal agent.

An "effective amount" of the alginate oligomer and the antifungal agent is that amount of alginate oligomer and that amount of the antifungal agent that together (or in combination or conjunction) provide measurable inhibition of the growth of a fungus, or population thereof, that is being targeted. In certain embodiments the "effective amount" of the alginate oligomer and the antifungal agent is that amount of alginate oligomer and that amount of the antifungal agent that together (or in combination or conjunction) provide measurable reduction in the resistance (or measurable increase in susceptibility or measurable decrease in tolerance) to the antifungal displayed by the fungus (e.g. using the above-described indicators of resistance).

A "pharmaceutically effective amount" of the alginate oligomer and the antifungal agent is that amount of alginate oligomer and that amount of the antifungal agent that together (or in combination or conjunction) provide a measurable treatment or prevention of the infection by a fungus that is being targeted. In certain embodiments the "pharmaceutically effective amount" of the alginate oligomer and the antifungal agent is that amount of alginate oligomer and that amount of the antifungal agent that together (or in combination or conjunction) provide a measurable reduction in the resistance (or measurable increase in susceptibility or measurable decrease in tolerance) to the antifungal agent displayed by the fungus (e.g. using the above-described indicators of resistance) in a subject.

The skilled man would easily be able to determine what an effective/pharmaceutically effective amount of alginate oligomer and antifungal agent would be on the basis of routine dose response protocols and, conveniently, the routine techniques for assessing microbial growth inhibition etc., as discussed above. The skilled man would, without undue burden, also be able to optimise these amounts to maximise the combinatorial effects of the alginate oligomer and antifungal agent in his target system.

In a therapeutic context, suitable doses of alginate oligomer and antifungal agent will vary from subject to subject and can be determined by the physician or veterinary practitioner in accordance with the weight, age and sex of the subject, the identity of the fungus being targeted, severity of the condition, the mode of administration and also the particular alginate oligomer or antifungal agent selected. Typically the alginate oligomers of use in the invention will be applied to the location undergoing treatment at a local concentration of at least 0.5%, preferably at least 2% or at least 4%, more preferably at least 6% and most preferably at least 10% weight by volume. Typically the antifungal agent of use in the invention will be applied to the location undergoing treatment at a local concentration of at least 0.0001 µg/ml, preferably at least 0.001, 0.01, 0.1, 0.5, 1, 2, 4, 8, 16, 32, 64, 128, 256, 512, 1024, 2048 or 4096 µg/ml.

Similar considerations apply outside of the therapeutic applications of the invention and likewise suitable doses of alginate oligomer and antifungal agent will vary depending on the specific application and can be determined by the relevant skilled man (e.g. farmer, food scientist, engineer) without undue burden. Again, examples of suitable local concentrations would be, for the alginate oligomers of use in the invention, at least 0.5%, preferably at least 2% or at least 4%, more preferably at least 6% and most preferably at least 10% weight by volume, and for the antifungal agent, at least 0.0001 µg/ml, preferably at least 0.001, 0.01, 0.1, 0.5, 1, 2, 4, 8, 16, 32, 64, 128, 256, 512, 1024, 2048 or 4096 µg/ml.

"Treatment" when used in relation to the treatment of a medical condition/infection (which reference extents to "colonisation" in certain aspects of the invention) in a subject in accordance with the invention is used broadly herein to include any therapeutic effect, i.e. any beneficial effect on the condition or in relation to the infection. Thus, not only included is eradication or elimination of the infection, or cure of the subject or infection, but also an improvement in the infection or condition of the subject. Thus included for example, is an improvement in any symptom or sign of the infection or condition, or in any clinically accepted indicator of the infection/condition (for example a decrease in wound size or an acceleration of healing time). Treatment thus includes both curative and palliative therapy, e.g. of a pre-existing or diagnosed infection/condition, i.e. a reactionary treatment.

"Prevention" as used herein refers to any prophylactic or preventative effect. It thus includes delaying, limiting, reducing or preventing the condition (which reference includes infection, contamination and population, as applicable, in the different aspects of the invention) or the onset of the condition, or one or more symptoms or indications thereof, for example relative to the condition or symptom or indication prior to the prophylactic treatment. Prophylaxis thus explicitly includes both absolute prevention of occurrence or development of the condition, or symptom or indication thereof, and any delay in the onset or development of the condition or symptom or indication, or reduction or limitation of the development or progression of the condition or symptom or indication.

Specifically, the alginate oligomers and the antifungal agent of use in the invention can be taken/applied together (or in combination or conjunction) as a prophylactic treatment, for example to prevent, or at least minimise the risk, of infection or colonisation by the fungus.

The aspect of the invention concerning the combating (treatment or prevention) of infection by fungus is of particular utility in the care of hospitalised patients as the risk of contracting an nosocomial infection (commonly known as hospital related/acquired infection or healthcare-associated infection) by a fungus can be minimised with a prophylactic regime of the alginate oligomers and antifungal agents defined herein. This aspect of the invention is also of particular utility in the care of subjects suffering from trauma, subjects with a burn, subjects with wounds and subjects with an implantable/in-dwelling medical device, all of which, as discussed above, are more susceptible to infection by fungi than a subject that is not affected similarly.

Generally, subjects in need of treatment or prophylaxis according to the invention will be diagnosed as suffering with or at risk from infection by a fungus e.g. identified as having or at risk of developing an infection by a fungus.

Specifically, the alginate oligomers and antifungal agents of use in the invention can be taken together (or in combination or conjunction) as a prophylactic treatment to prevent, or at least to minimise the risk, of developing an infection by the fungus including for example the infection of a wound by the fungus; infections of the respiratory tract and lungs by a fungus (for example in the context of cystic fibrosis, COPD, COAD, COAP, pneumonia, or other respiratory diseases) or infection of a medical (e.g. in-dwelling) device by the fungus.

The invention encompasses the use of a single alginate oligomer or a mixture (multiplicity/plurality) of different alginate oligomers. Thus, for example, a combination of different alginate oligomers (e.g. two or more) may be used.

The invention encompasses the use of a single antifungal agent or a mixture (multiplicity/plurality) of different antifungal agents. Thus, for example, a combination of different antifungal agents (e.g. two or more) may be used.

In one advantageous embodiment of the invention the alginate oligomers and antifungal agent may be used in the methods of the invention in conjunction or combination with a further anti-microbial agent (hereinafter "further anti-microbial agent")

In the context of a therapeutic use, such an anti-microbial agent may be any clinically-useful anti-microbial agent and particularly an antibiotic or an antiviral or an antifungal agent. In the context of non-therapeutic uses, the anti-microbial agent may again be any anti-microbial agent used for such purposes, e.g. any disinfectant or antiseptic or cleaning or sterilising agent. The agents may be used separately, or together in the same composition, simultaneously or sequentially or separately, e.g. at any desired time interval.

Thus, by way of representative example, the further anti-microbial agent may be used after the alginate oligomer and/or the antifungal agent, but a preceding or simultaneous or intervening use may be beneficial in some circumstances.

The choice of anti-microbial agent will of course need to be appropriate for the location undergoing treatment, but for instance anti-microbial agents, e.g. antibiotics, antifungals, antivirals, antiseptics and/or sterilising conditions such as irradiation (e.g. UV, X-ray, gamma) extremes of temperature, and extremes of pH may be used.

Representative antibiotics include the aminoglycosides (e.g. amikacin, gentamicin, kanamycin, neomycin, netilmicin, streptomycin, tobramycin); the carbacephems (e.g. loracarbef); the 1st generation cephalosporins (e.g. cefadroxil, cefazolin, cephalexin); 2nd generation cephalosporins (e.g. cefaclor, cefamandole, cephalexin, cefoxitin, cefprozil, cefuroxime); 3rd generation cephalosporins (e.g. cefixime, cefdinir, cefditoren, cefoperazone, cefotaxime, cefpodoxime, ceftazidime, ceftibuten, ceftizoxime, ceftriaxone); 4th generation cephalosporins (e.g. cefepime); the macrolides (e.g. azithromycin, clarithromycin, dirithromycin, erythromycin, troleandomycin); the monobactams (e.g. aztreonam); the penicillins (e.g. amoxicillin, ampicillin, carbenicillin, cloxacillin, dicloxacillin, nafcillin, oxacillin, penicillin G, penicillin V, piperacillin, ticarcillin); the polypeptide antibiotics (e.g. bacitracin, colistin, polymyxin B); the quinolones (e.g. ciprofloxacin, enoxacin, gatifloxacin, levofloxacin, lomefloxacin, moxifloxacin, norfloxacin, ofloxacin, trovafloxacin); the sulfonamides (e.g. mafenide, sulfacetamide, sulfamethizole, sulfasalazine, sulfisoxazole, trimethoprim- sulfamethoxazole); the tetracyclines (e.g. demeclocycline, doxycycline, minocycline, oxytetracycline, tetracycline); the glycylcyclines (e.g. tigecycline); the carbapenems (e.g. imipenem, meropenem, ertapenem, doripenem, panipenem/betamipron, biapenem, PZ-601); other antibiotics include chloramphenicol; clindamycin, ethambutol; fosfomycin; isoniazid; linezolid; metronidazole; nitrofurantoin; pyrazinamide; quinupristin/dalfopristin; rifampin; spectinomycin; and vancomycin.

Representative antiseptics include, but are not limited to chlorine bleach (sodium hypochlorite), quaternary ammonium compounds (e.g. benzalkonium chloride, cetyl trimethylammonium bromide, cetylpyridinium chloride), hydrogen peroxide, phenol compounds (e.g. TCP), alcohols (e.g. ethanol), Virkon™, iodine compounds (e.g. povidone-iodine), silver compounds (e.g. elemental silver nano/microparticles) and tricolsan (5-chloro-2-(2,4-dichlorophenoxy)phenol).

Antimicrobial surfactants are another class of antiseptics. These are compounds that disrupt microbial cell membranes and other structural components and therefore inhibit growth and/or viability of microorganisms. Antimicrobial surfactants and their use in antimicrobial compositions is well known in the art should further guidance be needed the discussion of antimicrobial surfactants in "Preservative-free and self-preserving cosmetics and drugs - Principles and practice", Ed. Kabara and Orth, Marcel Dekker, NY, NY, 1997. Antimicrobial surfactants may be anionic, cationic, non-ionic or amphoteric. Examples of antimicrobial anionic surfactants include, but are not limited to, sodium dodecyl sulfate (sodium lauryl sulfate), sodium dodecyl aminopropionic acid, sodium ricinoleate, bile acids, alkylaryl sulfonates, Grillosan DS7911, disodium undecylenic acid monoethanol amidosulfosuccinate. Examples of antimicrobial cationic surfactants include, but are not limited to, the quaternary ammionium compounds, the aminimides and chlorhexidine compounds. Examples of antimicrobial non-ionic surfactants include, but are not limited to, the monoesters of fatty acids, polyethyleneglycomonoesters of alkyldihydroxybenzoic acids, glucosamine derivatives and diethanolamides of N-lauroyl dipeptides. Examples of antimicrobial amphoteric surfactants include, but are not limited to, the alkyl betaines, the alkylamidopropylbetaines, the alkyl aminopropionates, the alkyliminodipropionates and the alkylimidazolines.

Representative antifungals include those listed above, especially those stated as preferred.

Representative antivirals include, but are not limited to, abacavir, acyclovir, adefovir, amantadine, amprenavir, arbidol, atazanavir, atripla, boceprevir, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine , imunovir, idoxuridine, imiquimod, indinavir, inosine, interferon type III, interferon type, II interferon type I, lamivudine, lopinavir, loviride, maraviroc, moroxydine, nelfinavir, nevirapine, nexavir, oseltamivir, penciclovir, peramivir, pleconaril, podophyllotoxin, raltegravir, ribavirin, rimantadine, ritonavir, saquinavir , stavudine , tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine.

The further anti-microbial agent may conveniently be applied before, simultaneously with, following or between the alginate oligomer and/or the antifungal agent. Conveniently the further anti-microbial agent is applied at substantially the same time as the alginate oligomer and/or the antifungal agent or afterwards. For example, the further anti-microbial agent may be applied at least 1 hour, preferably at least 3 hours, more preferably at least 5 and most preferably at least 6 hours after the alginate oligomer and/or the antifungal agent is administered. In other embodiments the further antimicrobial may conveniently be applied or administered before the alginate oligomer and/or the antifungal agent, e.g. at least 1 hour, at least 3 hours, at least 6 hours before the alginate oligomer and/or the antifungal agent. In these embodiments the alginate oligomer and/or the antifungal agent can be applied or administered with or without a further application of the further antimicrobial. To optimise the anti-microbial effect of the further anti-microbial agent it can be given (e.g. administered or delivered) repeatedly at time points appropriate for the agent used. The skilled person is able to devise a suitable dosage or usage regimen. In long term treatments the alginate oligomer and/or the antifungal agent can also be used repeatedly. The alginate oligomer can be applied as frequently as the antifungal agent and/or the further anti-microbial agent, but will typically be less frequently. The frequency required will depend on the location of the fungus, colony composition and the further anti-microbial used and the skilled person is able to optimise the dosage or usage patterns to optimise results.

In an advantageous embodiment the alginate oligomer and/or the antifungal agent may be used or applied after physical removal or reduction (e.g. debridement) of the colony/population comprising the fungus causing the infection at the location undergoing treatment.

Following removal of, or an attempt to remove, the fungus, the location may be contacted with the alginate oligomer for between 0 and 24 hours, particularly 2 and 12 hours, more particularly 4 and 8 hours, most particularly 5 and 7 hours, e.g. 6 hours. Following this, the antifungal agent, and if desired the further anti-microbial agent, may be applied. Such a scenario may be desirable or particularly applicable in a clinical setting. In the case of wounds infected by an fungus, the duration of incubation can be conveniently be designed to correspond to scheduled changes of the wound dressing.

Physical removal of the fungus can be carried out with any suitable surgical, mechanical or chemical means. Conveniently this can be the use of a liquid, gel, gel-sol, semi-solid compositions or gas applied at pressure to the colony/population, sonication, laser, or by abrasive implement. A composition used in the removal itself or as a wash solution before, during or afterwards may conveniently contain the alginate oligomer and/or the antifungal agent.

Accordingly, there is disclosed herein a debridement or wash composition e.g. solution for wounds containing an alginate oligomer, particularly any alginate oligomer as herein defined, and/or an antifungal agent, particularly any antifungal drug as herein defined, for use in the treatments of the invention. Such a debridement composition will typically be a sterile solution, particularly an aqueous sterile solution or an oil-based sterile solution, and may additionally contain proteolysis enzymes (e.g. collagenase, trypsin, pepsin, elastase) and/or an abrasive solid phase (e.g. colloidal silica, ground pumice, ground plant or animal shell).

Use of the alginate oligomers and the antifungal agent in combination or conjunction with immunostimulatory agents may also be beneficial in the application of the invention in a clinical situation. These immunostimulatory agents may conveniently be used at timepoints corresponding to those described above in relation to further anti-microbial agents and may optionally be used in combination with an alginate oligomer and/or the antifungal agent and/or a further anti-microbial agent Suitable immunostimulatory agents include, but are not limited to cytokines e.g. TNF, IL-1, IL-6, IL-8 and immunostimulatory alginates, such as high M-content alginates as described for example in US 5,169,840, WO91/11205 and WO03/045402, but including any alginate with immunostimulatory properties.

Use of the alginate oligomers and the antifungal agent in combination or conjunction with growth factors, e.g. PDGF, FGF, EGF, TGF, hGF and enzymes may also be beneficial in the medical uses of the invention. Representative examples of suitable enzymes include but are not limited to proteases, e.g. serine proteases, metalloproteases and cysteine proteases (examples of these types of proteases are listed in EP0590746); nucleases, e.g. DNase I and II, RNase A, H, I, II, III, P, PhyM, R; lipases and enzymes capable of degrading polysaccharides.

Use of the alginate oligomers and the antifungal agent in combination or conjunction with a physiologically tolerable mucosal viscosity reducing agent could also be beneficial, e.g. a nucleic acid cleaving enzyme (e.g. a DNase such as DNase I), gelsolin, a thiol reducing agent, an acetylcysteine, sodium chloride, an uncharged low molecular weight polysaccharide (e.g. dextran), arginine (or other nitric oxide precursors or synthesis stimulators), or an anionic polyamino acid (e.g. poly ASP or poly GLU). Ambroxol, romhexine, carbocisteine, domiodol, eprazinone, erdosteine, letosteine, mesna, neltenexine, sobrerol, stepronin, tiopronin are specific mucolytics of note.

Use of the alginate oligomers and the antifungal agent in combination or conjunction with bronchodilators may also be beneficial in the medical uses of the invention, in the treatment of respiratory diseases associated with fungi especially (which may include COPD, COAD, COAP, pneumonia, cystic fibrosis, emphysema and asthma). Representative examples of suitable bronchodilators include but are not limited to the β2 agonists (e.g. pirbuterol, epinephrine, salbutamol, salmeterol, levosalbutamol, clenbuterol), the anticholinergics (e.g. ipratropium, oxitropium, tiotropium) and theophylline.

Use of the alginate oligomers and the antifungal agent in combination or conjunction with corticosteroids may also be beneficial in the medical uses of the invention, in the treatment of respiratory diseases associated with fungi especially (which may include COPD, COAD, COAP, pneumonia, cystic fibrosis, emphysema and asthma). Representative examples of suitable corticosteroids include but are not limited to prednisone, flunisolide, triamcinolone, fluticasone, budesonide, mometasone, beclomethasone, amcinonide, budesonide, desonide, fluocinonide, fluocinolone, halcinonide. hydrocortisone, cortisone, tixocortol, prednisolone, methylprednisolone, prednisone, betamethasone, dexamethasone, fluocortolone, aclometasone, prednicarbate, clobetasone, clobetasol, and fluprednidene.

The alginate oligomers and the antifungal agent can be used optionally with any other therapeutically active agent it may be desired to use, e.g. an anti-microbial agent, an anti-inflammatory agent (e.g. an anti-inflammatory steroid), an immunostimulatory agent, a mucosal viscosity reducing agent, a growth inhibitor or an enzyme or an alpha blocker, a bronchodilator or a corticosteroid. The combined use of an alginate oligomer and an antifungal agent with a further therapeutically active agent (e.g. an anti-microbial or anti-inflammatory agent, an immunostimulatory agent, a mucosal viscosity reducing agent, a growth inhibitor or an enzyme or an alpha blocker, a bronchodilator or a corticosteroid) may improve the clinical effects of the active agent and this may advantageously allow the dose (e.g. the usual or normal dose) of the further therapeutically active agent to be reduced e.g. it may be used at its normal or usual dose or at a lower dose, for example at up to 50% (or at 50%) of its normal dose.

In the case of therapeutic use, the alginate oligomers and antifungal agents of use in the invention may be administered to the subject in any convenient form or by any convenient means, e.g. by topical, oral, parenteral, enteral, parenteral routes or by inhalation. Preferably the alginate and antifungal agents will be administered by topical, oral or parenteral routes or by inhalation. The alginate oligomers and antifungal agents need not be in the same composition and need not be administered via the same route.

The skilled man will be able to formulate the alginate oligomers and the antifungal agents of use in the invention into pharmaceutical compositions that are adapted for these routes of administration according to any of the conventional methods known in the art and widely described in the literature.

Also described herein is a pharmaceutical composition for use in any of the above-mentioned methods or uses comprising an alginate oligomer as defined herein together with at least one pharmaceutically acceptable carrier, diluent or excipient. This composition may also comprise an antifungal agent as defined herein.

Also described herein is a pharmaceutical composition for use in any of the above-mentioned methods or uses comprising an antifungal agent as defined herein together with at least one pharmaceutically acceptable carrier, diluent or excipient. This composition may also comprise an alginate oligomer as defined herein.

As discussed above, the alginate oligomers and the antifungal agents proposed for use according to the invention may be used in combination with each other, for example to be administered together, in a single pharmaceutical formulation or composition, or separately (i.e. for separate, sequential or simultaneous administration). Thus, the alginate oligomers and the antifungal agents of the use in invention may be combined, e.g. in a pharmaceutical kit or as a combined ("combination") product.

The present disclosure therefore also provides products (e.g. a pharmaceutical kit or a combined ("combination") product) or compositions (e.g. a pharmaceutical composition) wherein the product or composition comprises the alginate oligomer as herein defined and the antifungal agent.

Further active agents may also be incorporated. The above and following discussion of additional active agents and excipients and the like is directly applicable in its entirety to this part of the disclosrure.

Thus as noted above, further aspects of the present invention provide products containing the alginate oligomer and the antifungal agent as a combined preparation for the uses defined herein. Such products may optionally further contain a further active agent.

The active ingredient may be incorporated, optionally together with other active agents, with one or more conventional carriers, diluents and/or excipients, to produce conventional galenic preparations such as tablets, pills, powders (e.g. inhalable powders), lozenges, sachets, cachets, elixirs, suspensions, emulsions, solutions, syrups, aerosols (as a solid or in a liquid medium), sprays (e.g. nasal sprays), compositions for use in nebulisers, ointments, soft and hard gelatine capsules, suppositories, pessaries, sterile injectable solutions, sterile packaged powders, and the like. Sterile inhalable compositions are of particular note for use in the treatment of respiratory diseases associated with fungi (which may include COPD, COAD, COAP, pneumonia, cystic fibrosis, emphysema and asthma). Sterile injectable solutions are of particular note for use in the treatment of systemic fungal infections.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances such as hard fat or suitable mixtures thereof. Excipients and diluents of note are mannitol and hypertonic salt water (saline).

The compositions may additionally include lubricating agents, wetting agents, emulsifying agents, suspending agents, preserving agents, sweetening agents, flavouring agents, and the like. Additional therapeutically active agents may be included in the pharmaceutical compositions, as discussed above in relation to combination therapies above.

Parenterally administrable forms, e.g., intravenous solutions, should be sterile and free from physiologically unacceptable agents, and should have low osmolarity to minimize irritation or other adverse effects upon administration and thus solutions should preferably be isotonic or slightly hypertonic, e.g. hypertonic salt water (saline). Suitable vehicles include aqueous vehicles customarily used for administering parenteral solutions such as sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975). The solutions can contain preservatives, antimicrobial agents, buffers and antioxidants conventionally used for parenteral solutions, excipients and other additives which are compatible with the biopolymers and which will not interfere with the manufacture, storage or use of products.

For topical administration the alginate oligomer and/or the antifungal agent can be incorporated into creams, ointments, gels, transdermal patches and the like. The alginate oligomers and/or the antifungal agent can also be incorporated into medical dressings, for example wound dressings e.g. woven (e.g. fabric) dressings or non-woven dressings (e.g. gels or dressings with a gel component). The use of alginate polymers in dressings is known, and such dressings, or indeed any dressings, may further incorporate the alginate oligomers of use in the invention.

Accordingly, in a further specific example, the disclosure further provides a wound dressing comprising an alginate oligomer (which may be any alginate oligomer as herein defined) and/or an antifungal agent (which may be any antifungal agent as herein defined, although preferably an antifungal drug) for use, where appropriate, in the treatments of the invention.

Further topical systems that are envisaged to be suitable are *in situ* drug delivery systems, for example gels where solid, semi-solid, amorphous or liquid crystalline gel matrices are formed *in situ* and which may comprise the alginate oligomer (which may be any alginate oligomer as herein defined) and/or an antifungal agent (which may be any antifungal agent as herein defined, although preferably an antifungal drug). Such matrices can conveniently be designed to control the release of the alginate oligomer and/or the antifungal agent from the matrix, e.g. release can be delayed and/or sustained over a chosen period of time. Such systems may form gels only upon contact with biological tissues or fluids. Typically the gels are bioadhesive. Delivery to any body site that can retain or be adapted to retain the pre-gel composition can be targeted by such a delivery technique. Such systems are described in WO 2005/023176.

For application to oral, buccal and dental surfaces, toothpastes, dental gels, dental foams and mouthwashes are mentioned specifically. Thus, in one particular example is included an oral health care, or oral hygiene, composition, comprising an alginate oligomer (which may be any alginate oligomer as defined herein) and an antifungal agent (which may be any antifungal agent as herein defined, although preferably an antifungal drug), particularly a mouthwash, toothpaste, dental gel or dental foam for use, where appropriate, in the treatments of the invention.

For application to external body surfaces, skincare and haircare agents are mentioned specifically. Thus, in one particular example is included a skincare and/or haircare composition comprising an alginate oligomer and an antifungal agent (which may be any alginate oligomer or antifungal agent as defined herein), particularly a shampoo, soap, shower gel, hair conditioner, skin cream, emollient, ointment, lotion, oil, hair gel, hair spray, foam and wax.

Inhalable compositions are also of note. The formulation of compositions suitable for inhalation is routine for the skilled man and has long been standard practice in the treatment of respiratory diseases. Inhalable compositions may, for instance, take the form of inhalable powders, solutions or suspensions. The skilled man would be able to select the most appropriate type of delivery system for his needs and be able to prepare a suitable formulation of the alginates and/or antifungal drugs of use in the invention for use in that system. Propellant-free nebulisable solutions and inhalable powder formulations are particularly preferred, e.g. formulations wherein the antifungal drug and/or the alginate oligomer are solubilised in sterile water.

As noted above, a preferred composition of use in the invention is a debridement composition that is used in a debridement process to remove a fungus or colony or population thereof, for example from a tissue. Typically such a composition will be liquid, but gels, gel-sols, or semi-solid compositions might be used. The composition might be used to remove the fungus (e.g. by application to the infected tissue under pressure) and/or may be used to bathe the infected tissue before, during and/or after debridement by other means such as by surgical, mechanical or chemical processes. The skilled person is readily able to formulate debridement compositions.

In the case of a fungus on an inanimate surface or in an inanimate material, the alginate oligomer and/or antifungal agent may be applied to the surface or material to be treated in any convenient composition or formulation, or by any convenient means. Thus the alginate oligomer and/or antifungal agent may be in liquid, gel, gel-sol, semi-solid or solid form (e.g. solutions, suspensions, homogenates, emulsions, pastes, powders, aerosols, vapours). Typically the compositions for treating such inanimate surfaces or materials will be a non-pharmaceutically acceptable composition. The choice of composition form will be dictated by the identity of the fungus on the surface or in the material and location of the surface or material. For instance, if the location is a fluid line it might be convenient to apply a fluid composition. It might also be preferred to use a composition that persists on the surface or in the part of the fluid line to be treated but that will not leach into the fluid of normal use, e.g. an adhesive gel. Sprayable compositions are envisaged to be very convenient, but liquid formulations for use in dips, washes and baths might be more appropriate in some applications.

The skilled person is readily able to prepare suitable compositions from his common general knowledge. For instance, the alginate oligomer and/or antifungal agent may be added to a paint formulation and applied to the surface to be treated, e.g. a boat hull or other part of a boat's structure that is exposed to water, or to a building or any part thereof, a tank (e.g. a storage or processing tank) or indeed to any part of any industrial machinery. The composition may instead be a simple aqueous and/or alcoholic solution comprising the antifungal agent. If desired, a surfactant, a polyol or an oil may also be included to facilitate wetting or adhesion. Suitable compositions may conveniently also comprise a further pesticide (e.g. an anti-microbial agent as described above, or a herbicide, insecticide, miticide/acaricide, molluscicide or nematicide). The antimicrobial may be an antibiotic, chlorine bleach, TCP, ethanol, Virkon™, povidone-iodine, silver compounds, antimicrobial surfactants, triclosan, etc. As the compositions need not be pharmaceutically acceptable (or even physiologically acceptable in certain embodiments), harsh antimicrobials and pesticides can be used subject to considerations of surface damage, environmental contamination, user safety and contamination of the treated surface and interaction with the other components of the composition.

Likewise, in the case of a fungus on or in a non-clinical biotic location (e.g. plants and parts thereof, seeds, fruits, flowers and leaves, pelts, leather, hide, wool, wood, cotton, linen, jute, silk, bamboo, cork, soil) the alginate oligomer and/or antifungal agent may also be applied to the surface or material to be treated in any convenient composition or formulation, or by any convenient means. Thus the alginate oligomer and/or antifungal agent may be in liquid, gel, gel-sol, semi-solid or solid form (e.g. solutions, suspensions, homogenates, emulsions, pastes, powders, aerosols, vapours). Typically the compositions for treating such non-clinical biotic surfaces or materials will be a non-pharmaceutically acceptable composition although in the context of application to living tissue (e.g. plants and parts thereof such as seeds, fruits, flowers and leaves) it will typically be physiologically acceptable in that the composition will not damage the location to which it is applied.

The choice of composition form will be dictated by the identity of the fungus on the surface or in the material and location of the surface or material. Sprayable compositions are envisaged to be very convenient, but liquid formulations for use in dips, washes and baths might be more appropriate in some applications. It might also be preferred to use a composition that persists on the surface to be treated e.g. an adhesive gel. The skilled person is readily able to prepare suitable compositions from his common general knowledge. For instance the alginate oligomer and/or antifungal agent containing compositions of use in this aspect of the invention will typically also include a solvent, for example water, an alcohol (e.g. a C₁₋₆ alcohol), or a water/alcohol mixture. If desired, a surfactant, a polyol or an oil may also be included to facilitate wetting or adhesion. Suitable compositions may conveniently also comprise a further pesticide (e.g. an anti-microbial agent as described above or a herbicide, insecticide, miticide/acaricide, molluscicide or nematicide). As the compositions need not be pharmaceutically acceptable, harsh antimicrobials and pesticides can be used subject to considerations of surface damage, environmental contamination, user safety and contamination of the treated surface and interaction with the other components of the composition. In some applications a physiologically acceptable formulation might be required.

The compositions of use in the invention may be formulated so as to provide quick (immediate), sustained or delayed release of the active ingredient after administration to the subject/surface by employing procedures well known in the art. Adhesive compositions are also preferred. Adhesive, sustained and/or delayed release formulations may be particularly convenient, especially in applications where the prevention of infection/contamination is desired, e.g. in the treatment of crops, engineering materials, seeds and bulbs destined for future planting ("saved seeds/bulbs"), foodstfuffs and other plant and animal derived products to prevent spoilage.

The relative content of the alginate oligomer and the antifungal agent in the compositions of use in the invention can vary depending on the dosage required and the dosage regime being followed and this will depend on the subject to be treated and the location and identity of the fungus, and/or the constituents of the colony or population comprising the fungus. Preferably, the composition will comprise an amount of alginate oligomer and an amount of the antifungal agent that together will provide measurable inhibition of the growth of the fungus, or population thereof, that is being targeted, e.g. growth (e.g. replication) of a fungus, or the rate thereof, is reduced by at least 50%, more preferably at least 60%, 70%, 80% or 90%, e.g. at least 95%. Put in a different way, the composition will comprise an amount of alginate oligomer and an amount of antifungal agent that will provide a measurable treatment or prevention of the infection by a fungus that is being targeted. In certain embodiments the composition will comprise an amount of alginate oligomer and an amount of the antifungal agent that together will provide measurable reduction in the resistance (or measurable increase in susceptibility or measurable decrease in tolerance) to the antifungal displayed by the fungus, e.g. an amount of alginate oligomer that will at least double, at least quadruple, at least octuple, at least sexdecuple or at least duotrigecuple the susceptibility of the fungus, to the antifungal agent.

Preferably the composition or product will comprise sufficient alginate oligomer that upon administration to a subject or application to a location, the local concentration at the target location of the oligomer will be at least 2%, preferably at least 4%, 6% or 8% and most preferably at least 10% (weight by volume). The antifungal agent preferably will be present in an amount that is sufficient to provide a local concentration at the target location of at least 0.0001 µg/ml, preferably at least 0.001, 0.03125, 0.0625, 0.01, 0.125, 0.1, 0.25, 0.5, 1, 2, 4, 8, 16, 64, 128, 256, 512, 1024, 2048 or 4096 µg/ml. The skilled man would know that the amounts of alginate oligomer and/or antifungal agent can be reduced if a multiple dosing regime is followed or increased to minimise the number of administrations or applications.

The compositions and products of use in the invention will typically comprise 1% to 99%, 5% to 95%, 10% to 90% or 25% to 75% alginate oligomer and 1% to 99%, 5% to 95%, 10% to 90% or 25% to 75% antifungal agent, allowance being made for other ingredients.

The disclosure provides products susceptible to contamination/colonisation by fungi whose susceptible surfaces have been pretreated with an alginate oligomer and an antifungal agent as defined herein.

By "pretreated" it is meant that the susceptible surface is exposed to an alginate oligomer and/or an antifungal agent prior to an exposure to an fungus in such a way that the alginate oligomer and/or antifungal agent persists on the surface for a duration sufficient to prevent colonisation by an fungus for an appreciable duration of time. Preferably the alginate oligomer and/or the antifungal agent will persist for substantially the useful life of the surface, e.g. the pretreatment results in a substantially permanent coating of an alginate oligomer and/or an antifungal agent. Thus a pre-treated surface/product is one to which the alginate oligomer and/or antifungal agent is applied and on which it remains. Such a product/surface may be a coated and/or impregnated product/surface. Preferably a coating will comprise a plurality, i.e. at least two, layers of alginate oligomer and/or antifungal agent.

Non-limiting examples of products and surfaces susceptible to colonisation by fungi are described above. Particular mention may be made of medical devices (e.g. endotracheal or tracheostomy tubes), food or drink processing, storage or dispensing equipment, building materials (e.g. wood, timber, lumber, bricks, tiles, plasterboard, preformed concrete, paper), foodstuffs, cell culture media, seeds and bulbs. Pretreatment can be achieved by any convenient means, for example any form of applying the alginate oligomer and/or antifungal agent to the surface, notably coating the surface, e.g. spray drying, polymer coating with a polymer incorporating the alginate oligomer and/or antifungal agent, and painting, varnishing or lacquering with paint, varnish or lacquer formulations containing the alginate oligomer and/or antifungal agent. Such a "coating" composition (e.g. a paint, varnish or lacquer) containing an alginate oligomer and/or antifungal agent represents a further part of the disclosure. Alternatively, the alginate oligomer and/or antifungal agent can be incorporated or impregnated into the material from which the object or its susceptible parts are manufactured. This approach is suited to objects, or constituent parts thereof, manufactured from polymers such as plastics and silicones, e.g. the medical and surgical devices and building materials described above. Products comprising an inanimate surface or a non-clinical biotic surface comprising an alginate oligomer and/or antifungal agent coating or coating composition, or incorporating, or impregnated with, an alginate oligomer and/or antifungal agent are therefore contemplated.

Non-limiting examples of such products and surfaces are described above. Of particular note are medical and surgical devices. This may include any kind of line, including catheters (e.g. central venous and urinary catheters), prosthetic devices, e.g. heart valves, artificial joints, false teeth, dental crowns, dental caps, dental implants, and soft tissue implants (e.g. breast, buttock and lip implants). Any kind of implantable (or "in-dwelling") medical device is included (e.g. stents, intrauterine devices, pacemakers, intubation tubes (e.g. endotracheal or tracheostomy tubes), prostheses or prosthetic devices, lines or catheters).

Further products include food processing, storage, dispensing or preparation equipment or surfaces, tanks, conveyors, floors, drains, coolers, freezers, equipment surfaces, walls, valves, belts, pipes, air conditioning conduits, cooling apparatus, food or drink dispensing lines, heat exchangers, boat hulls or any part of a boat's structure that is exposed to water, dental waterlines, oil drilling conduits, contact lenses and storage cases, building materials (e.g. wood, timber, lumber, bricks, tiles, plasterboard, preformed concrete, paper), foodstuffs, cell culture media, seeds and bulbs.

The invention will be further described with reference to the following non-limiting Examples.

### EXAMPLES

### Example 1 - Effect of G-block Alginate Oligomers on the Minimum Inhibitory Concentrations of the Antifungal Agents Nystatin and Amphotericin B for Candida albicans

### Materials and Methods

The antifungal agents used (nystatin and amphotericin B) were pharmaceutical grade and purchased from Sigma Aldrich and USP Reference Standards, respectively. OligoG CF-5/20 G-block alginate oligomers (DP 5 to 20, average molecular weight 2600, 90-95% G residues) were provided by AlgiPharma AS, Norway. The Mueller-Hinton broth used was LAB114 from Lab M Limited. The M19 was prepared in house (peptone (Oxoid) 9.4 g/L; yeast extract (Oxoid) 4.7 g/L; beef extract (Difco) 2.4 g/L; glucose (BDH) 10 g/L; pH is adjusted to 6.1 with HCI before sterile filtration). YM agar/broth was 271120 from Difco.

The test strain used in this Example is *Candida albicans,* CCUG 39343.

A robotic MIC Assay was performed as follows. OligoG CF-5/20 were dissolved in medium (Mueller-Hinton broth or M19 broth depending on experiment) to 1.25 times of the desired assay concentrations (2, 6 and 10%). The antifungal agents under test were dissolved in medium without OligoG CF-5/20 and in medium with OligoG CF-5/20 at a concentration of 1.25 times the highest desired assay concentrations. For experiments where the effect of adding different concentrations of Na⁺ was investigated, NaCl was dissolved in medium to the desired concentrations (10, 30 and 50 mM).

Two-fold serial dilutions of antifungal agents were made in medium with different concentrations of OligoG CF-5/20, and the solutions were placed in four parallel wells in Nunc 384-well micro plates (30 µl per well in Nunc 242757 microplates). A group of 8 wells with no addition of antifungal agents for each OligoG CF-5/20 concentration was included on each microplate as growth reference. To each well in the 384-well assay plates was added 7.5 µl of medium inoculated with a frozen stock culture of *Candida albicans* (strain CCUG 39343).

The frozen stock culture was prepared by growing the Candida strain at 34°C for 48h on YM-agar. 1-3 colonies from the plates were grown in 6 ml YM-broth at 34°C for 14h before freezing in 6% glycerol at -80°C. Each batch of frozen stock culture was then characterized in separate growth experiments using Mueller-Hinton or M19 broth to determine the minimum amount of inoculum giving satisfactory growth after 48h under the conditions relevant for the MIC assay. This inoculation procedure is used in order to reduce day to day variation in bioassays.

The micro plates were placed in plastic bags and incubated without shaking at 34 °C. The optical density at 600 nm in the microwells was measured after approximately 13, 18, 24 and 34 hours of incubation, and the relative growth yield in each well was calculated based on the growth in the reference groups. The MIC value was set to the highest concentration giving less than 30 % growth in all 4 parallel wells within the sample groups.

### Results and Discussion

Results observed at 24 hour incubation are shown in Table 1

The ability of OligoG CF-5/20 to potentiate the effect of nystatin and amphotericin B on C. *albicans* was investigated in MIC assays. Furthermore, to rule out that a potential impact is caused by the Na⁺ ions present in the OligoG CF-5/20 molecules, MIC assays were also performed with nystatin or amphotericin B in combination with varying NaCl concentrations. Nystatin and amphotericin B are both polyene antifungal agents which interact specifically with ergosterol in the yeast cell membrane and create pores therein. These pores may facilitate the transport of otherwise retained components in and out of the cell. The presence of excess ions could then potentially have a potentiating effect with the antifungal agents. The concentrations of NaCl used are similar to what is observed in 2, 6 and 10% solutions of OligoG CF-5/20.

The addition of OligoG CF-5/20 leads to a reduction in MIC values for both nystatin and amphotericin B and in both media tested. The reduction is most pronounced for amphotericin B in both cases, being 32x and 16x in M19 and Mueller-Hinton respectively when comparing 0 and 10% G-block. The same numbers for nystatin is 16x and 8x.

In M19 there is a slight effect of adding NaCl, but the observed reduction in MIC is in each case smaller than what is obtained by addition of OligoG CF-5/20 (4x compared to 16x-32x). In Mueller-Hinton broth NaCl has no effect on the MIC values for either antifungal agent. Thus, these experiments clearly show that there is no indirect effect of adding Na⁺ together with the OligoG CF-5/20 and the observed reductions in MIC values is caused directly by OligoG CF-5/20.

Results obtained in this study show that co-administration of G-block alginate oligomers with the polyene antifungal agents nystatin and amphotericin B potentiates the effect of these antifungal agents on *C. albicans* as compared to their administration alone.

**Table 1. Minimum Inhibitory Concentration (MIC) values (µg/ml) of nystatin and amphotericin B for Candida albicans (strain CCUG 39343), in the presence of varying concentrations of OligoG CF-5/20 or NaCl as determined after incubation for 24 hours.**

| | **M19** | | **Mueller-Hinton** | |
|---|---|---|---|---|
| **OligoG CF 5/20** | **Nystatin** | **Amphotericin B** | **Nystatin** | **Amphotericin B** |
| 0% | 6.4 | 1.2 | 3.2 | 0.15 |
| 2% | 1.6 | 0.3 | 3.2 | 0.15 |
| 6% | 0.8 | 0.075 | 0.8 | 0.0375 |
| 10% | 0.4 | 0.0375 | 0.4 | 0.0094 |

| **NaCl** | **Nystatin** | **Amphotericin B** | **Nystatin** | **Amphotericin B** |
|---|---|---|---|---|
| 0 mM | 6.4 | 1.2 | 6.4 | 0.15 |
| 10 mM | * | 0.3 | 6.4 | 0.15 |
| 30 mM | 3.2 | 0.3 | 6.4 | 0.15 |
| 50 mM | 1.6 | 0.3 | 6.4 | 0.15 |

| | | | | |
|---|---|---|---|---|
| *MIC could not be determined due to irregular OD₆₀₀ measurements | | | | |

### Example 2 - Effect of G-block Alginate Oligomers on the Minimum Inhibitory Concentrations of the Antifungal Agents Nystatin, Fluconazole and Terbinafine for various Candida species - Robotic Screening

### Materials and Methods

The robotic assay described in Example 1 was employed in this Example. Strains were grown in Mueller-Hinton broth (LAB114 from Lab M Limited) and MIC values (µg ml-1) were determined for each antifungal after incubation for 48 h in the presence of 0, 2, 6 and 10% OligoG CF-5/20 G-fragments. The antifungal agents used (nystatin, fluconazole and terbinafine) were pharmaceutical grade and purchased from Sigma Aldrich. OligoG CF-5/20 G-fragments were provided by AlgiPharma AS, Norway.

Candida strains used were as follows:
*C. albicans* CCUG 39343 (strain from culture collection)
*C. parapsillosis* ATCC 22019T (strain from culture collection)
*C. krusei* 141/03 (candidosis)
*C. krusei* 249/03(2) (ulceration)
*C. lusitaniae* 994/01(2) (candidosis)
*C. tropicalis* 12 (vaginal)
*C. tropicalis* 75 (vaginal)
*C. tropicalis* 519468 (urinary)
*C. tropicalis* 544123 (urinary)
*C. tropicalis* 250/03 (candidosis)
*C. tropicalis* AG1 (oral)
*C. tropicalis* T2.2 (oral)

Candida strains other than *C. albicans* CCUG 39343 and *C. parapsillosis* ATCC 22019T were provided by M. Henriques, University of Minho, Portugal, and the reference numbers used for these strains are internal designations.

MIC values presented in Table 2 are based on four independent experiments.

### Results and Discussion

Results are shown in Table 2. MIC values for two different types of antifungal agents (nystatin and fluconazole) for all species and strains tested were reduced by the addition of OligoG CF-5/20. This study highlights the potential of G-block alginate oligomers to potentiate antifungal agents across a variety of fungal species and classes of antifungal.

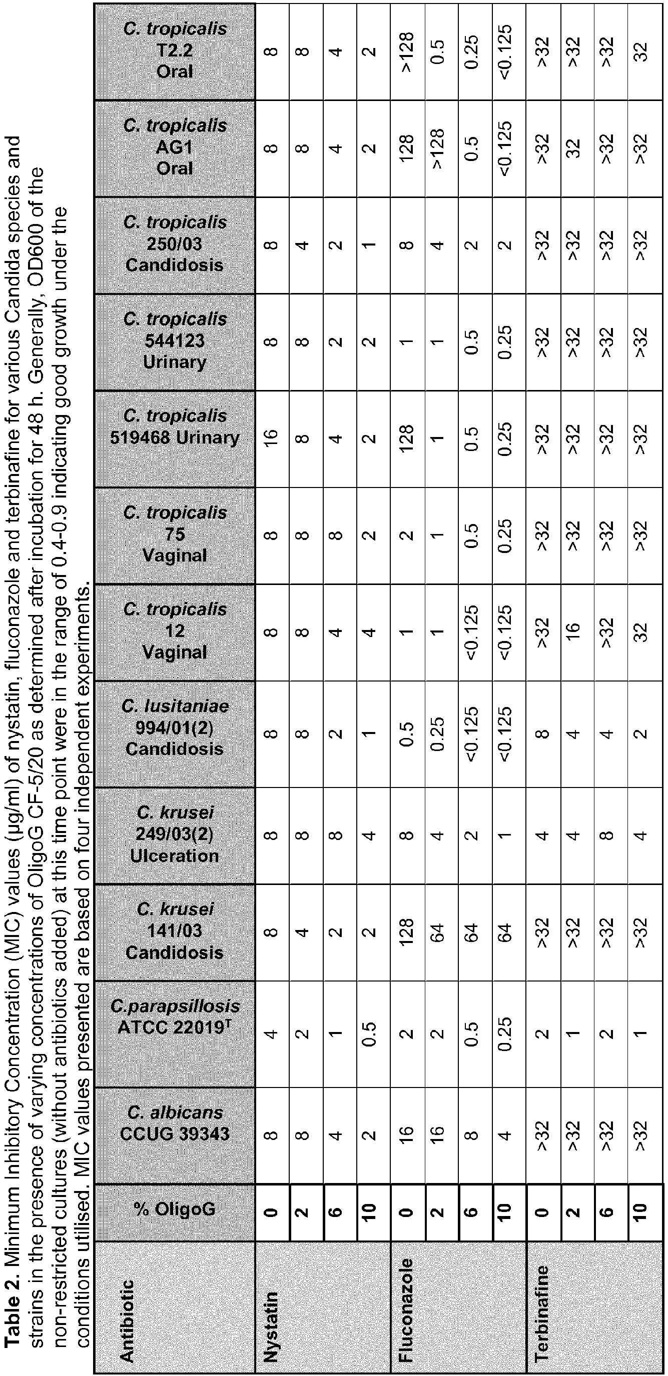

### Example 3 - Effect of G-block Alginate Oligomers on the Minimum Inhibitory Concentrations of the Antifungal Agent Fluconazole for various Candida species - Standard Minimum Inhibitory Concentration Testing

### Materials and Methods

The Candida strains, antifungal agent (fluconazole) and OligoG CF-5/20 used in this Example is the same as described in Example 2.

The Minimum Inhibitory Concentration assay used in this Example was based on Jorgensen et al. (Manual of Clinical Microbiology, 7th ed. Washington, D.C: American Society for Microbiology, 1999; 1526-43). Following retrieval from -80°C storage, fungal colonies were grown on blood agar with 5% sheep blood and were used to inoculate tryptone soya broth (TSB) for overnight growth.

Overnight fungal cultures as described above were diluted in sterile water until the OD625 was between 0.08 and 0.10 to confirm that the cell density was equivalent to 0.5 McFarland standard.

Two-fold serial dilutions of fluconazole were prepared in RPMI medium or RPMI medium with Oligo CF-5/20 at 2%, 6% or 10% and were placed in duplicate wells of flat-bottom 96-well microtiter plates (100 µl in each well).

Fungal cultures at 0.5 McFarland standard were diluted ten-fold in RPMI medium and 5 µl added to the media containing wells of the microtiter plates. Plates were wrapped in parafilm and incubated at 34°C for 48 hours. MIC values were determined as the lowest concentration at which there was no visible growth.

### Results and Discussion

Results observed at 48 hours incubation are shown in Table 3.

MIC values for fluconazole in 5 of the 8 strains tested were reduced by the addition of OligoG CF-5/20. This study highlights the potential of G-block alginate oligomers to potentiate antifungal therapies.

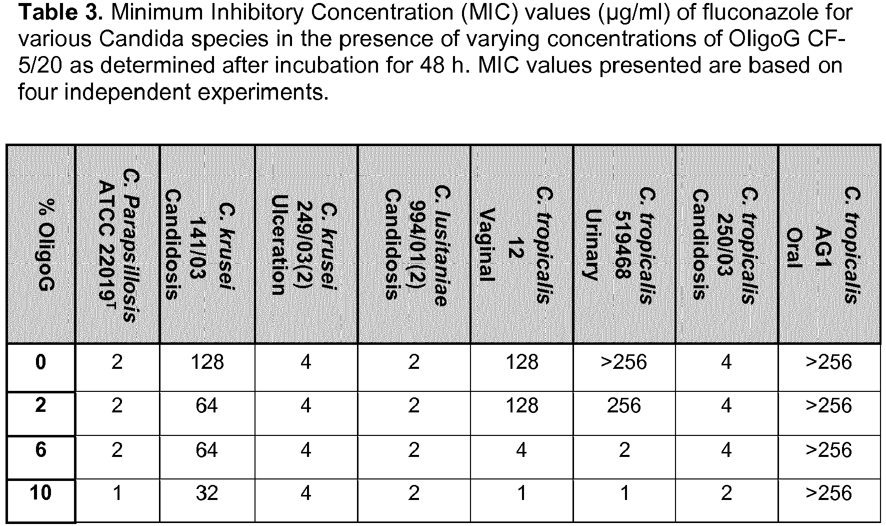

### Example 4 - Effect of G-block Alginate Oligomers on the Minimum Inhibitory Concentrations of Various Antifungal Agents for Various Aspergillus Species - Robotic Screening

### Materials and Methods

The antifungal agents used (nystatin, amphotericin B, miconazole, voriconazole, fluconazole and terbinafine were pharmaceutical grade and purchased from Sigma Aldrich and USP Reference Standards). OligoG CF-5/20 G-block alginate oligomers were provided by AlgiPharma AS, Norway. The Mueller-Hinton broth used was LAB114 from Lab M Limited. YM agar/broth was 271120 from Difco.

Fungal strains used were as follows:
*Aspergillus niger* CCUG 18919 (strain from culture collection; blueberry)
*Aspergillus fumigatus* CCUG 17460 (strain from culture collection)
*Aspergillus flavus* CCUG 28296 (strain from culture collection; shoe sole)
*Aspergillus sp.* was grown at 30°C for 96h on YM-agar. Spores and aerial mycelium was cut out from the agar, suspended in 1 ml YM-broth and dispersed with glass beads (1 mm) in a mini bead beater for 2 min. Glycerol was added to the suspension to 10% and frozen at -80°C.

Each batch of frozen stock culture was then characterized in separate growth experiments using Mueller-Hinton broth to determine the minimum amount of inoculum giving satisfactory growth after 48h under the conditions relevant for the MIC assay. This inoculation procedure is used in order to reduce day to day variation in bioassays.

Robotic minimum inhibitory concentration (MIC) assay was performed as follows. OligoG CF-5/20 was dissolved in medium (Mueller-Hinton broth) to 1.25 times of the desired assay concentrations (2, 6 and 10%). Antifungal agents were dissolved in medium without OligoG CF-5/20 and in medium with OligoG CF-5/20 at a concentration of 1.25 times the highest desired assay concentrations.

Two-fold serial dilutions of antifungal agents were made in medium with different concentrations of OligoG CF-5/20, and the solutions were placed in four parallel wells in Nunc 384-well micro plates (30 µl per well in Nunc 242757 microplates). A group of 8 wells with no addition of antifungal agents for each OligoG CF-5/20 concentration was included on each micro plate as growth reference. Each well in the 384-well assay plates was added 7.5 µl of the MH medium inoculated with frozen stock culture of the relevant strains.

The microplates were placed in plastic bags and incubated without shaking at 34°C. The optical density at 600 nm in the microwells was measured after approximately 24 and 48 hours of incubation. The MIC value was set to the highest concentration giving less than 30 % growth in all 4 parallel wells within the sample groups.

### Results and Discussion

Results observed at 48 hour incubation are shown in Table 4

The ability of OligoG CF-5/20 to potentiate the effect of antifungal agents from different classes, i.e. the polyene antifungals (nystatin and amphotericin B), the azole antifungals (fluconazole, miconazole, voriconazole) and the allylamine antifungals (terbinafine) on various Aspergillus species was investigated in MIC assays.

The addition of OligoG CF-5/20 leads to a reduction in MIC values of nystatin, amphotericin B, miconazole, voriconazole and terbinafine for all Aspergillus species tested. OligoG CF-5/20 was able to reduce MIC values of fluconazole for one of the species of Aspergillus tested (*Aspergillus flavus*).

Results obtained in this study show that co-administration of G-block alginate oligomers can potentiate the effect of different types of antifungal agents. In combination with the results from the other Examples it can be seen that this potentiation effect is also observed across different species and genera of fungi.

**Table 4. Minimum Inhibitory Concentration (MIC) values (µg/ml) of nystatin, amphotericin B, miconazole, voriconazole, fluconazole and terbinafine for various Aspergillus species in the presence of varying concentrations of OligoG CF-5/20 after incubation for 48 hours.**

| **Antibiotic** | **% OligoG** | **A. niger CCUG 18919** | **A. fumigatus CCUG 17460** | **A. flavus CCUG 23451** |
|---|---|---|---|---|
| **Nystatin** | 0 | 8 | 8 | 8 |
| | 2 | 4 | 8 | 8 |
| | 6 | 2 | 4 | 4 |
| | 10 | 0.5 | 2 | 2 |
| **AmphotericinB** | 0 | 0.1875 | 0.75 | 0.75 |
| | 2 | 0.1875 | 0.375 | 0.375 |
| | 6 | 0.09375 | 0.375 | 0.375 |
| | 10 | 0.046875 | 0.1875 | 0.1875 |
| **Fluconazole** | 0 | >128 | >128 | 64 |
| | 2 | >128 | >128 | 64 |
| | 6 | >128 | 128 | 32 |
| | 10 | 128 | 128 | 32 |
| **Miconazole** | 0 | 1 | 4 | 1 |
| | 2 | 0.5 | 2 | 0.25 |
| | 6 | 0.25 | 2 | 0.25 |
| | 10 | 0.25 | 2 | 0.25 |
| **Voriconazole** | 0 | 0.25 | 0.125 | 0.0625 |
| | 2 | 0.0625 | 0.125 | 0.0625 |
| | 6 | <0.03125 | 0.0625 | <0.03125 |
| | 10 | <0.03125 | <0.03125 | <0.03125 |
| **Terbinafine** | 0 | 0.5 | 4 | 0.125 |
| | 2 | 0.25 | 4 | 0.0625 |
| | 6 | 0.0625 | 4 | <0.03125 |
| | 10 | 0.125 | 2 | <0.03125 |

### Example 5 - Effect of ipratropium bromide, salbutamol, budesonide and formoterol on the Minimum Inhibitory Concentrations of Combinations of Nystatin/Amphotericin B and G-block Alginate Oligomers for Candida albicans.

### Materials and Methods

The robotic assay described in Example 1 was employed in this Example. Additionally, the water soluble asthma/COPD medicaments ipratropium bromide monohydrate, salbutamol and budesonide were dissolved in Mueller-Hinton broth with and without OligoG CF-5/20, and with or without antifungal agents to obtain the desired concentrations. Formoterol fumarate dihydrate, which is water insoluble, was dissolved in DMSO and added directly to the 384-plates. The final DMSO concentration in the cultivation medium was 2% which does not significantly influence growth of the indicator organism. The concentration utilized for the different components were based on information from Manocha et al. (2006). Ipratropium bromide monohydrate, salbutamol, budesonide and formoterol fumarate dihydrate were pharmaceutical grade and obtained from Sigma Aldrich.

### Results and Discussion

To test whether selected representatives of commonly used asthma/COPD medicaments might have an impact on the MIC values of combinations of nystatin/amphotericin B and G-block alginate oligomers for *Candida albicans,* the effect of addition of these medicaments in the MIC assay described in Example 1 was determined. In this assay only Mueller-Hinton broth was used since this is the medium specified for standardized MIC assays (Jorgensen et al., 1999).

Ipratropium bromide and salbutamol are soluble in water and were used at 0.01 and 1 mM in the assays, whereas budesonide has limited solubility in water and was used only at 0.01 mM. Formoterol is insoluble in water, and was therefore dissolved in DMSO and thereafter added directly to the assay plates to a final concentration of 0.5 mM. This resulted in a final DMSO content of 2%.

Results observed at 24 hours incubation are shown in Table 5.

Results from the assays show a reduction in MIC values of 4x for both nystatin and amphotericin B by the addition of 10% OligoG CF-5/20 compared to without OligoG CF-5/20. This is a smaller reduction than what was obtained in the initial experiments (Table 5), and the absolute MIC values are also slightly different. The reason for these deviations is not known. However, the trend in OligoG CF-5/20 increasing the effect of both antifungal agents is clear.

The results shown in Table 5 indicate that ipratropium bromide (anticholinergic) and salbutamol (short acting beta-2-agonist) at 0.01 mM and 1 mM, and budesonide (corticosteroid) at 0.01 mM and formoterol (long acting beta-2-agonist) at 0.5 mM do not significantly influence the MIC values of nystatin or amphotericin B in combination with OligoG CF5/20 for C. *albicans.* Where variations in MIC values occur by the addition of one of these four compounds, it is generally a factor of two which is within the resolution of the assay (the antifungal agents were used in two-fold serial dilutions).

This study indicates that none of these compounds influence the antifungal potentiating effects of G-block alginate oligomers. Addition of ipratropium bromide, salbutamol, budesonide and formoterol at the given concentrations did not affect growth of C. *albicans* (data not shown).

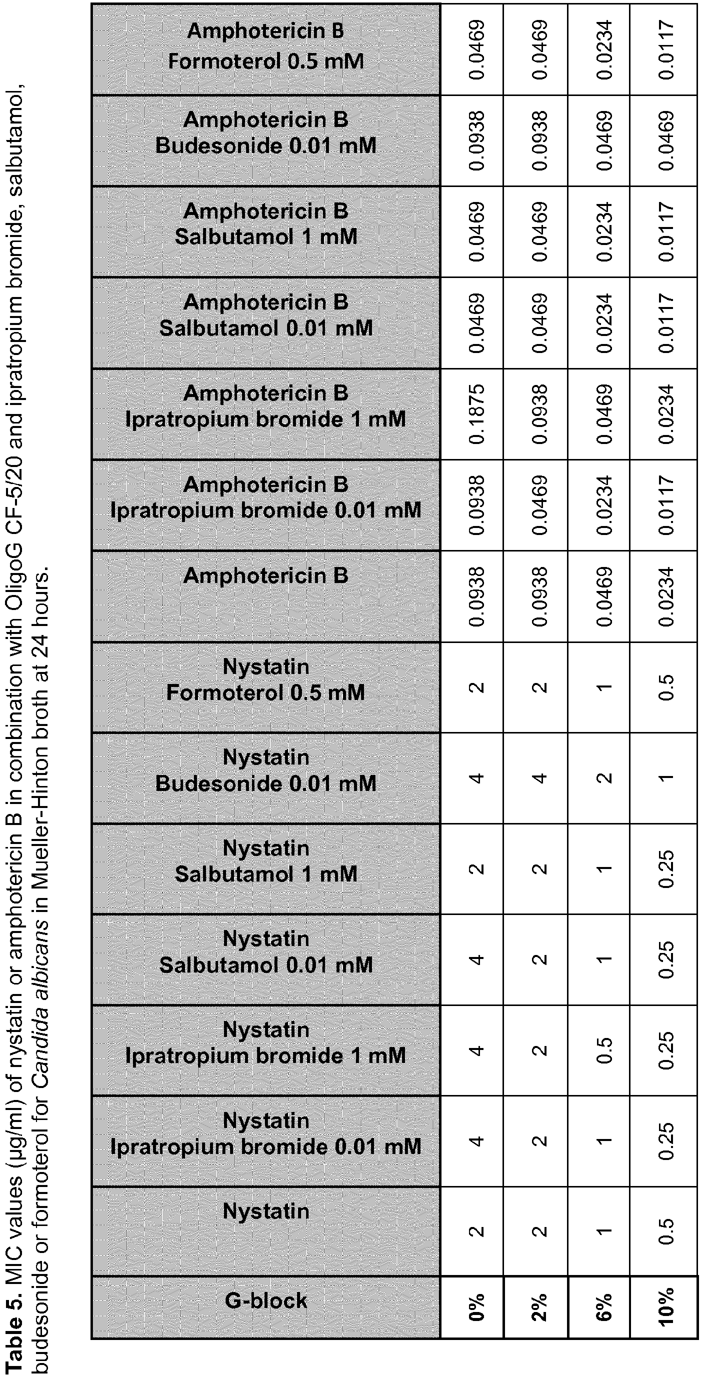

### Example 6 - Effect of G-block Alginate Oligomers on the Minimum Inhibitory Concentrations of Various Antifungal Agents for Cryptococcus neoformans - Robotic Screening

### Materials and Methods

The antifungal agents used (nystatin, amphotericin B, miconazole and fluconazole) were pharmaceutical grade and purchased from Sigma Aldrich and USP Reference Standards). OligoG CF-5/20 G-block alginate oligomers were provided by AlgiPharma AS, Norway. The Mueller-Hinton broth used was LAB114 from Lab M Limited. YM agar/broth was 271120 from Difco. Sabouraud media was prepared in-house (glucose, 20 g/l; tryptone, 5 g/l; peptone,5 g/l). RPMI was from Sigma and supplemented with 2% glucose.

Fungal strains used were as follows:
*Cryptococcus neoformans* CCUG 23451 (human cerebrospinal fluid)

Cryptococcus sp. was grown at 30°C for 96h on YM-agar. Spores and aerial mycelium was cut out from the agar, suspended in 1 ml YM-broth and dispersed with glass beads (1 mm) in a mini bead beater for 2 min. The suspension was added glycerol to 10% and frozen at -80°C. Each batch of frozen stock culture was then characterized in separate growth experiments using Mueller-Hinton and RPMI to determine the minimum amount of inoculum giving satisfactory growth after 48h under the conditions relevant for the MIC assay. This inoculum procedure is used in order to reduce day to day variation in bioassays.

Robotic minimum inhibitory concentration (MIC) assay was performed as follows. OligoG CF-5/20 was dissolved in medium (Mueller-Hinton, RPMI with 2% glucose, Sabouraud or YM) to 1.25 times of the desired assay concentrations (2, 6 and 10%). Antifungal agents were dissolved in medium without OligoG CF-5/20 and in medium with OligoG CF-5/20 at a concentration of 1.25 times the highest desired assay concentrations.

Two-fold serial dilutions of antifungal agents were made in medium with different concentrations of OligoG CF-5/20, and the solutions were placed in four parallel wells in Nunc 384-well micro plates (30 µl per well in Nunc 242757 microplates). A group of 8 wells with no addition of antifungal agents for each OligoG CF-5/20 concentration was included on each micro plate as growth reference. Each well in the 384-well assay plates was added 7.5 µl of the medium innoculated with frozen stock culture of the relevant strains.

The microplates were placed in plastic bags and incubated without shaking at 34°C. The optical density at 600 nm in the microwells was measured after certain time points from 24h to 96h of incubation. The MIC value was set to the highest concentration giving less than 30 % growth in all 4 parallel wells within the sample groups.

### Results and Discussion

The ability of OligoG CF-5/20 to potentiate the effect of antifungal agents from different classes, i.e. the polyene antifungals (nystatin and amphotericin B) and the azole antifungals (fluconazole and miconazole) on *Cryptococcus neoformans* was investigated in MIC assays.

Results observed at 72 hour incubation are shown in Table 6 and results observed at 96 hour incubation are shown in Table 7. In the analysis of these results, the following points should be noted. In MH and YM media the growth of *C. neoformans* is so low when 6% and 10% OligoG is added that MIC cannot be determined. In Sabouraud medium, growth is too low with 10% OligoG to determine MIC. In RPMI with 2% glucose growth is acceptable; however precipitations in the medium when combining 10% OligoG with certain of the antifungal agents disturbs MIC readings at the highest OligoG concentrations used and in these cases MIC could not be determined.

For amphotericin B there is a reduction in MIC in all media tested when OligoG is added to the medium. For nystatin there is a reduction in MIC in all media tested when OligoG is added to the medium. For fluconazole there is a reduction in MIC in all media tested when OligoG is added to the medium. For miconazole there is a reduction in MIC in RPMI with glucose and in MH medium when OligoG is added to the medium. Overall, these data show that the addition of OligoG CF-5/20 leads to a reduction in MIC values of nystatin, amphotericin B, miconazole and fluconazole in *Cryptococcus neoformans.*

The results obtained in this study show that co-administration of G-block alginate oligomers can potentiate the effect of different types of antifungal agents on *Cryptococcus neoformans.* In combination with the results from the other Examples it can be seen that this potentiation effect is also observed across different species and genera of fungi.

**Table 6. Minimum Inhibitory Concentration (MIC) values (µg/ml) of nystatin, amphotericin B, miconazole and fluconazole for Cryptococcus neoformans CCUG 23451 in the presence of varying concentrations of OligoG CF-5/20 after incubation for 72 hours. NG, no growth; ND, not determined.**

| | | **Medium** | | | |
|---|---|---|---|---|---|
| **Antifungal agent** | **%OligoG** | **RPMI 2% GLU** | **MH** | **Sabouraud** | **YM** |
| Amphotericin B | 0 | >1.5 | NG | 0.1875 | >1.5 |
| | 2 | 1.5 | NG | 0.09375 | 1.5 |
| | 6 | 0.75 | NG | 0.01171875 | NG |
| | 10 | ND | NG | NG | NG |
| Miconazole | 0 | 4 | NG | <0.015625 | 0.5 |
| | 2 | 2 | NG | 0.0625 | 0.5 |
| | 6 | 0.5 | NG | 0.0078125 | NG |
| | 10 | ND | NG | NG | NG |
| Fluconazole | 0 | 2 | NG | 1 | 1 |
| | 2 | 1 | NG | 0.5 | 0.5 |
| | 6 | 0.5 | NG | 0.5 | NG |
| | 10 | ND | NG | NG | NG |
| Nystatin | 0 | 8 | NG | 2 | 8 |
| | 2 | 8 | NG | 0.5 | 4 |
| | 6 | 4 | NG | 0.25 | NG |
| | 10 | ND | NG | NG | NG |

**Table 7. Minimum Inhibitory Concentration (MIC) values (µg/ml) of nystatin, amphotericin B, miconazole and fluconazole for Cryptococcus neoformans CCUG 23451 in the presence of varying concentrations of OligoG CF-5/20 after incubation for 96 hours. NG, no growth; ND, not determined.**

| | | **Medium** | | | |
|---|---|---|---|---|---|
| **Antifungal agent** | **% OligoG** | **2% GLU** | **MH** | **Sabouraud** | **YM** |
| Amphotericin B | 0 | >1.5 | 1.5 | 0.375 | >1.5 |
| | 2 | >1.5 | 0.1875 | 0.09375 | 1.5 |
| | 6 | 1.5 | NG | 0.0234 | NG |
| | 10 | 0.75 | NG | NG | NG |
| Miconazole | 0 | 4 | 1 | 0.0625 | 0.5 |
| | 2 | 2 | 0.125 | 0.25 | 1 |
| | 6 | 1 | NG | 0.5 | NG |
| | 10 | ND | NG | NG | NG |
| Fluconazole | 0 | 2 | 2 | 1 | 1 |
| | 2 | 1 | 0.5 | 0.5 | 1 |
| | 6 | 1 | NG | 0.5 | NG |
| | 10 | 1 | NG | NG | NG |
| Nystatin | 0 | 8 | 4 | 2 | 16 |
| | 2 | 8 | 1 | 0.5 | 8 |
| | 6 | 8 | NG | 0.25 | NG |
| | 10 | ND | NG | NG | NG |

## Claims

1. An *in vitro* method for combating colonisation of a site with a fungus, which is not in a biofilm, said method comprising contacting the site and/or the fungus, which is not in a biofilm, with an alginate oligomer of 2 to 50 monomer residues at least 80% of which are G residues together with at least one antifungal agent, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not in a biofilm, is enhanced.

2. An alginate oligomer of 2 to 50 monomer residues at least 80% of which are G residues for use together with at least one antifungal agent in treating a subject infected, suspected to be infected, or at risk of infection, with a fungus, wherein the fungus is not in a biofilm, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not in a biofilm, is enhanced.

3. A product containing an alginate oligomer of 2 to 50 monomer residues at least 80% of which are G residues and an antifungal agent as a combined preparation for separate, simultaneous or sequential use in treating a subject infected, suspected to be infected, or at risk of infection, with a fungus , wherein the fungus is not in a biofilm, wherein
(i) said fungus is *Candida albicans* and said antifungal agent is nystatin, amphotericin B or fluconazole;
(ii) said fungus is *Candida tropicalis, Candida parapsilosis, Candida krusei* or *Candida lusitaniae* and said antifungal agent is nystatin or fluconazole;
(iii) said fungus is *Candida lusitaniae* and said antifungal agent is terbinafine;
(iv) said fungus is *Aspergillus niger* or *Aspergillus fumigatus* and said antifungal agent is nystatin, amphotericin B, miconazole, voriconazole, or terbinafine;
(v) said fungus is *Aspergillus flavus* and said antifungal agent is nystatin, amphotericin B, fluconazole, miconazole, voriconazole, or terbinafine; or
(vi) said fungus is *Cryptococcus neoformans* and said antifungal agent is nystatin, amphotericin B, miconazole or fluconazole,
and wherein the efficacy of said antifungal agent to inhibit the growth and/or viability of said fungus, when not it a biofilm, is enhanced.

4. The alginate oligomer of claim 2 or the product of claim 3, wherein the infection with a fungus which is not in a biolfim is of an internal or external body surface selected from a surface in the oral cavity, the reproductive tract, the urinary tract, the respiratory tract, the gastrointestinal tract, the peritoneum, the middle ear, the prostate, vascular intima, the eye, including the conjunctiva or corneal tissue, lung tissue, heart valves, skin, scalp, nails, the interior of wounds or the surface of adrenal, hepatic, renal, pancreatic, pituitary, thyroid, immune, ovarian, testicular, prostate, endometrial, ocular, mammary, adipose, epithelial, endothelial, neural, muscle, pulmonary, epidermis and osseous tissue, or in a body fluid selected from blood, plasma, serum, cerebrospinal fluid, GI tract contents, sputum, pulmonary secretions and semen, or in or on body tissue selected from adrenal, hepatic, renal, pancreatic, pituitary, thyroid, immune, ovarian, testicular, prostate, endometrial, ocular, mammary, adipose, epithelial, endothelial, neural, muscle, pulmonary, epidermis and osseous tissue.

5. The alginate oligomer or product of any one of claims 2 to 4, wherein the subject is a subject with a pre-established infection, an immunocompromised subject, a subject undergoing intensive or critical care, a subject suffering from trauma, a subject with a burn, a subject with an acute and/or chronic wound, a neonatal subject, an elderly subject, a subject with cancer, a subject with malnutrition, a subject with alcholism, a subject suffering from an auto-immune condition, a subject with reduced or abrogated epithelial or endothelial secretion and/or secretion clearance, a subject undergoing or recovering from antibiotic therapy, a subject undergoing or recovering from steroid therapy or a subject fitted with a medical device.

6. The alginate oligomer or product of claim 5, wherein the subject is selected from
(i) a subject with a condition selected from HIV, sepsis, septic shock, AIDS, a cancer of the immune system, rheumatoid arthritis, diabetes mellitus type I, Crohn's disease, COPD, COAD, COAP, bronchitis, cystic fibrosis, emphysema, lung cancer, asthma, pneumonia or sinusitis,
(ii) a subject preparing for, undergoing, or recovering from chemotherapy and/or radiotherapy, an organ transplant subject,
(iv) a subject resident in a healthcare institution,
(v) a smoker, or
(vi) a subject with a respiratory condition or disease.

7. The method of claim 1, wherein the fungus which is not in a biofilm is on a surface selected from surfaces of food or drink processing, preparation, storage or dispensing machinery or equipment, surfaces of air conditioning apparatus, surfaces of industrial machinery, surfaces of storage tanks, surfaces of medical or surgical equipment, surfaces of aquatic/marine equipment or the surfaces of buildings and other structures.

8. The method of claim 7, wherein the surface is selected from food processing, storage, dispensing or preparation equipment or surfaces, tanks, conveyors, floors, drains, coolers, freezers, equipment surfaces, walls, valves, belts, pipes, air conditioning conduits, cooling apparatus, food or drink dispensing lines, heat exchangers, boat hulls, dental waterlines, oil drilling conduits, contact lenses, contact lens storage cases, catheters, prosthetic devices or implantable medical devices.

9. The method of claim 1, wherein the fungus which is not in a biofilm is in a material selected from clinical/scientific waste, soil, compost, animal and plant products, animal or human food stuffs, personal hygiene products, cosmetics, drinking water supplies, waste water supplies, agricultural feedstuffs and water supplies, insecticide formulations, pesticide formulations, herbicide formulations, industrial lubricants, engineering materials, cell and tissue culture media, cell and tissue cultures, plant culture media and plant cultures.

10. The method, alginate oligomer or product of any one of claims 1 to 9, wherein the alginate oligomer has a number average degree of polymerisation of 2 to 35, 2 to 30, 2 to 25 or 2 to 20.

11. The method, alginate oligomer or product of any one of claims 1 to 9, wherein the alginate oligomer is a 2- to 35-mer, 2- to 30-mer, 3- to 35-mer, 3- to 28-mer, 4-to 25-mer, 5- to 20-mer, 6- to 22-mer, 8- to 20-mer, or 10- to 15-mer.

12. The method, alginate oligomer or product of any one of claims 1 to 11 wherein the alginate oligomer has at least 90% or at least 95% G residues.

13. The method, alginate oligomer or product of any one of claims 1 to 12, wherein at least 80% of the G residues are arranged in G-blocks.

## Patentansprüche

1. *In-vitro-*Verfahren zum Bekämpfen einer Kolonisation einer Stelle mit einem Pilz, der nicht in einem Biofilm ist, wobei das Verfahren das In-Kontakt-Bringen der Stelle und/oder des Pilzes, der nicht in einem Biofilm ist, mit einem Alginatoligomer aus 2 bis 50 Monomerresten, von denen mindestens 80% G-Reste sind, zusammen mit mindestens einem Antimykotikum umfasst, wobei
(i) der Pilz *Candida albicans* ist und das Antimykotikum Nystatin, Amphotericin B oder Fluconazol ist;
(ii) der Pilz *Candida tropicalis, Candida parapsilosis, Candida krusei* oder *Candida lusitaniae* ist und das Antimykotikum Nystatin oder Fluconazol ist;
(iii) der Pilz *Candida lusitaniae* ist und das Antimykotikum Terbinafin ist;
(iv) der Pilz *Aspergillus niger* oder *Aspergillus fumigatus* ist und das Antimykotikum Nystatin, Amphotericin B, Miconazol, Voriconazol oder Terbinafin ist;
(v) der Pilz *Aspergillus flavus* ist und das Antimykotikum Nystatin, Amphotericin B, Fluconazol, Miconazol, Voriconazol oder Terbinafin ist; oder
(vi) der Pilz *Cryptococcus neoformans* ist und das Antimykotikum Nystatin, Amphotericin B, Miconazol oder Fluconazol ist,
und wobei die Wirksamkeit des Antimykotikums, das Wachstum und/oder die Viabilität des Pilzes, wenn nicht in einem Biofilm, zu hemmen, verstärkt wird.

2. Alginatoligomer aus 2 bis 50 Monomerresten, von denen mindestens 80% G-Reste sind, zur Verwendung zusammen mit mindestens einem Antimykotikum bei der Behandlung eines Subjekts, das mit einem Pilz infiziert ist, das vermutlich mit einem Pilz infiziert ist oder bei dem das Risiko einer Infektion mit einem Pilz besteht, wobei der Pilz nicht in einem Biofilm ist, wobei
(i) der Pilz *Candida albicans* ist und das Antimykotikum Nystatin, Amphotericin B oder Fluconazol ist;
(ii) der Pilz *Candida tropicalis, Candida parapsilosis, Candida krusei* oder *Candida lusitaniae* ist und das Antimykotikum Nystatin oder Fluconazol ist;
(iii) der Pilz *Candida lusitaniae* ist und das Antimykotikum Terbinafin ist;
(iv) der Pilz *Aspergillus niger* oder *Aspergillus fumigatus* ist und das Antimykotikum Nystatin, Amphotericin B, Miconazol, Voriconazol oder Terbinafin ist;
(v) der Pilz *Aspergillus flavus* ist und das Antimykotikum Nystatin, Amphotericin B, Fluconazol, Miconazol, Voriconazol oder Terbinafin ist; oder
(vi) der Pilz *Cryptococcus neoformans* ist und das Antimykotikum Nystatin, Amphotericin B, Miconazol oder Fluconazol ist,
und wobei die Wirksamkeit des Antimykotikums, das Wachstum und/oder die Viabilität des Pilzes, wenn nicht in einem Biofilm, zu hemmen, verstärkt wird.

3. Produkt, enthaltend ein Alginatoligomer aus 2 bis 50 Monomerresten, von denen mindestens 80% G-Reste sind, und ein Antimykotikum als kombiniertes Präparat zur separaten, gleichzeitigen oder aufeinander folgenden Verwendung bei der Behandlung eines Subjekts, das mit einem Pilz infiziert ist, das vermutlich mit einem Pilz infiziert ist oder bei dem das Risiko einer Infektion mit einem Pilz besteht, wobei der Pilz nicht in einem Biofilm ist, wobei
(i) der Pilz *Candida albicans* ist und das Antimykotikum Nystatin, Amphotericin B oder Fluconazol ist;
(ii) der Pilz *Candida tropicalis, Candida parapsilosis, Candida krusei* oder *Candida lusitaniae* ist und das Antimykotikum Nystatin oder Fluconazol ist;
(iii) der Pilz *Candida lusitaniae* ist und das Antimykotikum Terbinafin ist;
(iv) der Pilz *Aspergillus niger* oder *Aspergillus fumigatus* ist und das Antimykotikum Nystatin, Amphotericin B, Miconazol, Voriconazol oder Terbinafin ist;
(v) der Pilz *Aspergillus flavus* ist und das Antimykotikum Nystatin, Amphotericin B, Fluconazol, Miconazol, Voriconazol oder Terbinafin ist; oder
(vi) der Pilz *Cryptococcus neoformans* ist und das Antimykotikum Nystatin, Amphotericin B, Miconazol oder Fluconazol ist,
und wobei die Wirksamkeit des Antimykotikums, das Wachstum und/oder die Viabilität des Pilzes, wenn nicht in einem Biofilm, zu hemmen, verstärkt wird.

4. Alginatoligomer nach Anspruch 2 oder Produkt nach Anspruch 3, wobei die Infektion mit einem Pilz, der nicht in einem Biofilm ist, von einer inneren oder äußeren Körperoberfläche, ausgewählt aus einer Oberfläche in der Mundhöhle, dem Fortpflanzungstrakt, dem Harntrakt, dem Atemtrakt, dem Magendarmtrakt, dem Bauchfell, dem Mittelohr, der Prostata, der Gefäßintima, dem Auge, beinhaltend das Bindehaut- oder Hornhautgewebe, Lungengewebe, Herzklappen, Haut, Kopfhaut, Nägel, dem Inneren von Wunden oder von der Oberfläche von Nebennieren-, Leber-, Nieren-, Bauchspeicheldrüsen-, Hirnanhangdrüsen-, Schilddrüsen-, Immun-, Eierstock-, Hoden-, Prostata-, Gebärmutterschleimhaut -, Augen-, Brust-, Fett-, Epithel-, Endothel-, Nerven-, Muskel-, Lungen-, Epidermis- und Knochengewebe oder in einem Körperfluid, ausgewählt aus Blut, Plasma, Serum, Rückenmarkflüssigkeit, Inhalt des Magendarmtranks, Auswurf, Lungensekreten und Sperma, oder in oder auf Körpergewebe, ausgewählt aus Nebennieren-, Leber-, Nieren-, Bauchspeicheldrüsen-, Hirnanhangdrüsen-, Schilddrüsen-, Immun-, Eierstock-, Hoden-, Prostata-, Gebärmutterschleimhaut-, Augen-, Brust-, Fett-, Epithel-, Endothel-, Nerven-, Muskel-, Lungen-, Epidermis- und Knochengewebe, ist.

5. Alginatoligomer oder Produkt nach einem der Ansprüche 2 bis 4, wobei das Subjekt ein Subjekt mit einer bereits gesicherten Infektion, ein immungeschwächtes Subjekt, ein Subjekt, das eine Intensivpflege oder kritische Pflege absolviert, ein Subjekt, das an einem Trauma leidet, ein Subjekt mit einer Verbrennung, ein Subjekt mit einer akuten und/oder chronischen Wunde, ein neonatales Subjekt, ein älteres Subjekt, ein Subjekt mit Krebs, ein Subjekt mit Mangelernährung, ein Subjekt mit Alkoholismus, ein Subjekt, das an einer Autoimmunerkrankung leidet, ein Subjekt mit reduzierter oder außer Kraft gesetzter Epithel- oder Endothelsekretion und/oder Sekretabbau, ein Subjekt, das eine Antibiotikatherapie absolviert oder sich davon erholt, ein Subjekt, das eine Steroidtherapie absolviert oder sich davon erholt, oder ein Subjekt, das mit einer medizinischen Vorrichtung ausgestattet ist.

6. Alginatoligomer oder Produkt nach Anspruch 5, wobei das Subjekt ausgewählt ist aus
(i) einem Subjekt mit einer Erkrankung, ausgewählt aus HIV, Sepsis, septischem Schock, AIDS, einem Krebs des Immunsystems, rheumatoider Arthritis, Diabetes mellitus Typ I, Morbus Crohn, COPD, COAD, COAP, Bronchitis, Mukoviszidose, Emphysem, Lungenkrebs, Asthma, Lungenentzündung oder Sinusitis;
(ii) einem Subjekt, das sich auf eine Chemotherapie und/oder Strahlentherapie, vorbereitet, eine Chemotherapie und/oder Strahlentherapie absolviert oder sich von einer Chemotherapie und/oder Strahlentherapie erholt, einem Organtransplantationssubjekt,
(iv) einem Subjekt, das in einer Gesundheitseinrichtung untergebracht ist;
(v) einem Raucher oder
(vi) einem Subjekt mit einer Lungenerkrankung oder einem Lungenleiden.

7. Verfahren nach Anspruch 1, wobei der Pilz, der nicht in einem Biofilm ist, auf einer Oberfläche ist, die ausgewählt ist aus Oberflächen von Maschinen oder Vorrichtungen zur Verarbeitung, Zubereitung, Lagerung oder Ausgabe von Lebensmitteln oder Getränken, Oberflächen von Klimaanlagen, Oberflächen von Industriemaschinen, Oberflächen von Lagertanks, Oberflächen von medizinischen oder chirurgischen Vorrichtungen, Oberflächen von aquatischen/maritimen Vorrichtungen oder den Oberflächen von Gebäuden und anderen Strukturen.

8. Verfahren nach Anspruch 7, wobei die Oberfläche ausgewählt ist aus Vorrichtungen oder Oberflächen für die Verarbeitung, Lagerung, Ausgabe oder Zubereitung von Lebensmitteln, Tanks, Förderern, Böden, Abflüssen, Kühlern, Gefrierschränken, Oberflächen von Vorrichtungen, Wänden, Ventilen, Gurten, Rohren, Klimaanlagenleitungen, Kühlvorrichtungen, Lebensmittel- oder Getränkeausgabebändern, Wärmetauschern, Bootsrümpfen, Dentalwasserleitungen, Ölbohrungsleitungen, Kontaktlinsen, Kontaktlinsenaufbewahrungsbehältern, Kathetern, Prothesen oder implantierbaren medizinischen Vorrichtungen.

9. Verfahren nach Anspruch 1, wobei der Pilz, der nicht in einem Biofilm ist, in einem Material ist, das ausgewählt ist aus Klinik-/Forschungsabfall, Erde, Kompost, tierischen und pflanzlichen Produkten, Lebensmitteln für Tier oder Mensch, Körperpflegeprodukten, Kosmetika, Trinkwasser, Abwasser, landwirtschaftlichen Futtermitteln und Wasser, Insektizidformulierungen, Pestizidformulierungen, Herbizidformulierungen, Industrieschmierstoffen, technischen Werkstoffen, Zell- und Gewebekulturmedien, Zell- und Gewebekulturen, Pflanzenkulturmedien und Pflanzenkulturen.

10. Verfahren, Alginatoligomer oder Produkt nach einem der Ansprüche 1 bis 9, wobei das Alginatoligomer einen zahlengemittelten Polymerisationsgrad von 2 bis 35, 2 bis 30, 2 bis 25 oder 2 bis 20 aufweist.

11. Verfahren, Alginatoligomer oder Produkt nach einem der Ansprüche 1 bis 9, wobei das Alginatoligomer ein 2- bis 35-mer, 2- bis 30-mer, 3- bis 35-mer, 3- bis 28-mer, 4- bis 25-mer, 5- bis 20-mer, 6- bis 22-mer, 8- bis 20-mer oder 10- bis 15-mer ist.

12. Verfahren, Alginatoligomer oder Produkt nach einem der Ansprüche 1 bis 11, wobei das Alginatoligomer zu mindestens 90% oder zu mindestens 95% G-Reste aufweist.

13. Verfahren, Alginatoligomer oder Produkt nach einem der Ansprüche 1 bis 12, wobei mindestens 80 % der G-Reste in G-Blöcken angeordnet sind.

## Revendications

1. Procédé *in vitro* pour lutter contre la colonisation d'un site par un champignon, qui n'est pas dans un biofilm, ledit procédé comprenant la mise en contact du site et/ou du champignon, qui n'est pas dans un biofilm, avec un oligomère d'alginate de 2 à 50 résidus monomériques dont au moins 80 % sont des résidus G conjointement avec au moins un agent antifongique, dans lequel
(i) ledit champignon est *Candida albicans* et ledit agent antifongique est la nystatine, l'amphotéricine B ou le fluconazole ;
(ii) ledit champignon est *Candida tropicalis, Candida parapsilosis, Candida krusei* ou *Candida lusitaniae* et ledit agent antifongique est la nystatine ou le fluconazole ;
(iii) ledit champignon est *Candida lusitaniae* et ledit agent antifongique est la terbinafine ;
(iv) ledit champignon est *Aspergillus niger* ou *Aspergillus fumigatus* et ledit agent antifongique est la nystatine, l'amphotéricine B, le miconazole, le voriconazole, ou la terbinafine ;
(v) ledit champignon est *Aspergillus flavus* et ledit agent antifongique est la nystatine, l'amphotéricine B, le fluconazole, le miconazole, le voriconazole, ou la terbinafine ; ou
(vi) ledit champignon est *Cryptococcus neoformans* et ledit agent antifongique est la nystatine, l'amphotéricine B, le miconazole ou le fluconazole,
et dans lequel l'efficacité dudit agent antifongique à inhiber la croissance et/ou la viabilité dudit champignon, lorsqu'il n'est pas dans un biofilm, est renforcée.

2. Oligomère d'alginate de 2 à 50 résidus monomériques dont au moins 80 % sont des résidus G pour une utilisation conjointement avec au moins un agent antifongique dans le traitement d'un sujet infecté, suspecté d'être infecté, ou risquant de contracter une infection, avec un champignon, dans lequel le champignon n'est pas dans un biofilm, dans lequel
(i) ledit champignon est *Candida albicans* et ledit agent antifongique est la nystatine, l'amphotéricine B ou le fluconazole ;
(ii) ledit champignon est *Candida tropicalis, Candida parapsilosis, Candida krusei* ou *Candida lusitaniae* et ledit agent antifongique est la nystatine ou le fluconazole ;
(iii) ledit champignon est *Candida lusitaniae* et ledit agent antifongique est la terbinafine ;
(iv) ledit champignon est *Aspergillus niger* ou *Aspergillus fumigatus* et ledit agent antifongique est la nystatine, l'amphotéricine B, le miconazole, le voriconazole, ou la terbinafine ;
(v) ledit champignon est *Aspergillus flavus* et ledit agent antifongique est la nystatine, l'amphotéricine B, le fluconazole, le miconazole, le voriconazole, ou la terbinafine ; ou
(vi) ledit champignon est *Cryptococcus neoformans* et ledit agent antifongique est la nystatine, l'amphotéricine B, le miconazole ou le fluconazole,
et dans lequel l'efficacité dudit agent antifongique à inhiber la croissance et/ou la viabilité dudit champignon, lorsqu'il n'est pas dans un biofilm, est renforcée.

3. Produit contenant un oligomère d'alginate de 2 à 50 résidus monomériques dont au moins 80 % sont des résidus G et un agent antifongique sous la forme d'une préparation combinée pour une utilisation séparée, simultanée ou séquentielle dans le traitement d'un sujet infecté, suspecté d'être infecté, ou risquant de contracter une infection, avec un champignon, dans lequel le champignon n'est pas dans un biofilm, dans lequel
(i) ledit champignon est *Candida albicans* et ledit agent antifongique est la nystatine, l'amphotéricine B ou le fluconazole ;
(ii) ledit champignon est *Candida tropicalis, Candida parapsilosis, Candida krusei* ou *Candida lusitaniae* et ledit agent antifongique est la nystatine ou le fluconazole ;
(iii) ledit champignon est *Candida lusitaniae* et ledit agent antifongique est la terbinafine ;
(iv) ledit champignon est *Aspergillus niger* ou *Aspergillus fumigatus* et ledit agent antifongique est la nystatine, l'amphotéricine B, le miconazole, le voriconazole, ou la terbinafine ;
(v) ledit champignon est *Aspergillus flavus* et ledit agent antifongique est la nystatine, l'amphotéricine B, le fluconazole, le miconazole, le voriconazole, ou la terbinafine ; ou
(vi) ledit champignon est *Cryptococcus neoformans* et ledit agent antifongique est la nystatine, l'amphotéricine B, le miconazole ou le fluconazole,
et dans lequel l'efficacité dudit agent antifongique à inhiber la croissance et/ou la viabilité dudit champignon, lorsqu'il n'est pas dans un biofilm, est renforcée.

4. Oligomère d'alginate selon la revendication 2 ou produit selon la revendication 3, dans lequel l'infection avec un champignon qui n'est pas dans un biofilm est une infection d'une surface corporelle interne ou externe sélectionnée parmi une surface dans la cavité bucco-dentaire, les voies génitales, les voies urinaires, les voies respiratoires, les voies gastro-intestinales, le péritoine, l'oreille moyenne, la prostate, l'intima vasculaire, les yeux, incluant la conjonctive ou les tissus cornéens, les tissus des poumons, les valvules cardiaques, la peau, le cuir chevelu, les ongles, l'intérieur de plaies ou la surface des tissus surrénaliens, hépatiques, rénaux, pancréatiques, hypophysaires, thyroïdiens, immunitaires, ovariens, testiculaires, prostatiques, endométriaux, oculaires, mammaires, adipeux, épithéliaux, endothéliaux, nerveux, musculaires, pulmonaires, épidermiques et osseux, ou dans un fluide corporel sélectionné parmi le sang, le plasma, le sérum, le liquide céphalo-rachidien, les contenus des voies gastro-intestinales, les expectorations, les sécrétions pulmonaires et le sperme, ou dans ou sur des tissus corporels sélectionnés parmi des tissus surrénaliens, hépatiques, rénaux, pancréatiques, hypophysaires, thyroïdiens, immunitaires, ovariens, testiculaires, prostatiques, endométriaux, oculaires, mammaires, adipeux, épithéliaux, endothéliaux, nerveux, musculaires, pulmonaires, épidermiques et osseux.

5. Oligomère d'alginate ou produit selon l'une quelconque des revendications 2 à 4, dans lequel le sujet est un sujet présentant une infection préétablie, un sujet immunodéprimé, un sujet soumis à des soins intensifs ou essentiels, un sujet souffrant d'un traumatisme, un sujet présentant une brûlure, un sujet présentant une plaie aiguë et/ou chronique, un sujet nouveau-né, un sujet âgé, un sujet cancéreux, un sujet malnutri, un sujet alcoolique, un sujet souffrant d'une affection auto-immune, un sujet présentant une réduction ou une suppression des sécrétions épithéliales ou endothéliales et/ou une clairance des sécrétions, un sujet soumis à une antibiothérapie ou relevant de celle-ci, un sujet soumis à une thérapie par stéroïdes ou relevant de celle-ci ou un sujet équipé d'un dispositif médical.

6. Oligomère d'alginate ou produit selon la revendication 5, dans lequel le sujet est sélectionné parmi
(i) un sujet ayant une affection sélectionnée parmi le VIH, une septicémie, un choc septique, le SIDA, un cancer du système immunitaire, une polyarthrite rhumatoïde, un diabète de type 1, la maladie de Crohn, une BPCO, MPOC, COAP, une bronchite, une fibrose kystique, un emphysème, un cancer du poumon, un asthme, une pneumonie ou une sinusite,
(ii) un sujet se préparant à, soumis à ou relevant d'une chimiothérapie et/ou d'une radiothérapie, un sujet soumis à une greffe d'organe,
(iv) un sujet résidant dans un établissement de santé,
(v) un fumeur, ou
(vi) un sujet présentant une affection ou une maladie respiratoire.

7. Procédé selon la revendication 1, dans lequel le champignon qui n'est pas dans un biofilm se trouve sur une surface sélectionnée parmi des surfaces de traitement d'aliments ou de boissons, de machine ou d'équipement de préparation, stockage ou distribution, des surfaces d'un appareil de climatisation, des surfaces de machines industrielles, des surfaces de réservoirs de stockage, des surfaces d'équipement médical ou chirurgical, des surfaces d'équipement aquatique/marin ou des surfaces de bâtiments et autres structures.

8. Procédé selon la revendication 7, dans lequel la surface est sélectionnée parmi un équipement ou des surfaces de traitement d'aliments, stockage, distribution ou préparation, des réservoirs, des dispositifs de transport, des sols, des canalisations, des réfrigérateurs, des congélateurs, des surfaces d'équipement, des murs, valves, courroies, tuyaux, des conduites de climatisation, des appareils de refroidissement, des circuits de distribution d'aliments ou de boissons, des échangeurs de chaleur, des coques de bateau, des conduites d'eau dentaires, des conduites de forage, des lentilles de contact, des boîtes de rangement de lentilles de contact, des cathéters, des dispositifs prothétiques ou des dispositifs médicaux implantables.

9. Procédé selon la revendication 1, dans lequel le champignon qui n'est pas dans un biofilm se trouve dans un matériau sélectionné parmi des déchets cliniques/scientifiques, de la terre, du compost, des produits animaux et végétaux, des aliments pour animaux ou êtres humains, des produits d'hygiène personnelle, des cosmétiques, des systèmes d'approvisionnement en eau potable, des conduites d'eaux usées, des aliments agricoles et des conduites d'eau, des formulations insecticides, des formulations pesticides, des formulations herbicides, des lubrifiants industriels, des matériaux de construction, des milieux de culture cellulaire et tissulaire, des cultures cellulaires et tissulaires, des milieux de culture de végétaux et des cultures de végétaux.

10. Procédé, oligomère d'alginate ou produit selon l'une quelconque des revendications 1 à 9, dans lequel l'oligomère d'alginate a un degré de polymérisation moyen en nombre de 2 à 35, 2 à 30, 2 à 25 ou 2 à 20.

11. Procédé, oligomère d'alginate ou produit selon l'une quelconque des revendications 1 à 9, dans lequel l'oligomère d'alginate est un 2- à 35-mère, 2- à 30-mère, 3- à 35-mère, 3- à 28-mère, 4- à 25-mères, 5- à 20-mère, 6- à 22-mère, 8- à 20-mère, ou 10- à 15-mère.

12. Procédé, oligomère d'alginate ou produit selon l'une quelconque des revendications 1 à 11, dans lequel l'oligomère d'alginate a au moins 90 % ou au moins 95 % de résidus G.

13. Procédé, oligomère d'alginate ou produit selon l'une quelconque des revendications 1 à 12, dans lequel au moins 80 % des résidus G sont agencés en blocs de G.
